# EUROPEAN PATENT APPLICATION

(11) **EP 4 529 922 A1**
(43) Date of publication of application: **02.04.2025**
(21) Application number: 23201068.6
(22) Date of filing: 29.09.2023
(51) Int. Cl.: A61K 9/20, A61K 31/4439, A61K 31/553, A61P 31/18

(54) **PHARMACEUTICAL FORMULATIONS OF BICTEGRAVIR AND LENACAPAVIR**

(71) Applicant: Gilead Sciences, Inc., Foster City, CA 94404 (US)
(72) Inventor: FOGARTY, Julie, Redwood City (US)
(74) Representative: Carpmaels & Ransford LLP

(57) **Abstract**

The disclosure provides a solid oral dosage form comprising bictegravir or a pharmaceutically acceptable salt thereof, and lenacapavir or a pharmaceutically acceptable salt thereof.

## Description

### TECHNICAL FIELD

Pharmaceutical formulations suitable for treating viral infections such as HIV are disclosed, in particular tablets comprising bictegravir and lenacapavir.

### BACKGROUND

Human immunodeficiency virus, type 1 (HIV-1) infection is a life-threatening and serious disease of major public health significance, with approximately 35 million people infected worldwide (Joint United Nations Programme on HIV/AIDS (UNAIDS). Global report: UNAIDS report on the global AIDS epidemic, **2013**). Standard of care for the treatmeTnt of HIV-1 infection uses combination antiretroviral therapy (ART) to suppress viral replication to below detectable limits, increase CD4 cell counts, and halt disease progression.

The success of potent and well-tolerated ART means that most patients with a HIV-1 (PWH) with a history of virologic failure and viral resistance can achieve and maintain virologic suppression, although often on a complicated multi-tablet regimen dosed more than once daily, with disparate dosing schedules. A multi-tablet regimen is more difficult for PWH to adhere to consistently over the long-term, and poor adherence is a common reason for virologic failure. Low pill burden is associated with higher levels of adherence (Nachega, Clin Infect Dis 2014, 58:1297-307; Raboud, AIDS behave 2011, 15(7):1397-409). Additionally, PWH receiving single-tablet regimens are less likely to discontinue therapy (*i*.*e*. higher persistence) compared with those receiving multi-tablet regimens (Hines, Patient preference and adherence 2019, 13:1927-39). In 2 meta-analyses, PWH on a single-tablet regimen were more likely to achieve and maintain virologic suppression (Clay, Medicine, 2015, 94 (42):1-14; Clay, AIDS research and therapy 2018, 15:17). Convenience (e.g. pill burden, dosing frequency, availability of FDC or single-tablet regimen formulations) is one of the regimen-specific considerations in selecting an initial ARV regimen. Therefore, there remains a significant need to simplify treatment regimens for all PWH, including those who are virologically suppressed on a complicated regimen due to their prior history of virologic failure and viral resistance.

### SUMMARY

The present disclosure provides tablets containing bictegravir (BIC) and lenacapavir (LEN). These tablets are suitable for use in medicine, and in particular in treating viral infections such as HIV. These tablets are intended to be pharmaceutically acceptable.

In one aspect, the disclosure provides a tablet comprising bictegravir or a pharmaceutically acceptable salt thereof and lenacapavir or a pharmaceutically acceptable salt thereof, wherein the amount of bictegravir or pharmaceutically acceptable salt thereof corresponds to about 50-100 mg bictegravir free acid, and the amount of lenacapavir or pharmaceutically acceptable salt thereof corresponds to about 25-100 mg lenacapavir free acid.

In another aspect, the disclosure provides a tablet comprising bictegravir or a pharmaceutically acceptable salt thereof and lenacapavir or a pharmaceutically acceptable salt thereof, wherein the amount of bictegravir or pharmaceutically acceptable salt thereof corresponds to about 75 mg bictegravir free acid, and the amount of lenacapavir or pharmaceutically acceptable salt thereof corresponds to about 25 mg lenacapavir free acid.

In yet another aspect, the disclosure provides a tablet comprising bictegravir or a pharmaceutically acceptable salt thereof and lenacapavir or a pharmaceutically acceptable salt thereof, wherein the amount of bictegravir or pharmaceutically acceptable salt thereof corresponds to about 75 mg bictegravir free acid, and the amount of lenacapavir or pharmaceutically acceptable salt thereof corresponds to about 50 mg lenacapavir free acid.

In still another aspect, the disclosure also provides methods of producing tablets and methods for treating patients.

### BRIEF DESCRIPTION OF DRAWINGS

Figure 1 is a flow diagram illustrating the preparation of a tablet formulation of bictegravir.
Figure 2 is a flow diagram illustrating the preparation of a tablet formulation of lenacapavir.
Figure 3 is a flow diagram illustrating the preparation of a monolayer tablet comprising bictegravir and lenacapavir.
Figure 4 is a flow diagram illustrating the preparation of a bilayer tablet comprising bictegravir and lenacapavir.
Figures 5A and 5B show the results of studies carried out on bilayer and monolayer tablet formulations comprising bictegravir and lenacapavir to assess the dissolution of bictegravir as compared to the dissolution of bictegravir from a combination of single agent tablets containing the same doses of bictegravir and lenacapavir, respectively. Figure 5A shows the results for tablets containing 75 mg bictegravir and 25 mg lenacapavir, respectively, and Figure 5B shows the results for tablets containing 75 mg bictegravir and 50 mg lenacapavir.
Figures 6A and 6B show the results of studies carried out on bilayer and monolayer tablet formulations comprising bictegravir and lenacapavir to assess the dissolution of lenacapavir as compared to the dissolution of lenacapavir from a combination of single agent tablets containing the same doses of bictegravir and lenacapavir, respectively. Figure 6A shows the results for tablets containing 75 mg bictegravir and 25 mg lenacapavir, respectively, and Figure 6B shows the results for tablets containing 75 mg bictegravir and 50 mg lenacapavir.
Figures 7A, 7B, 8A, and 8B show the results of clinical human studies assessing the PK parameters of monolayer tablets comprising bictegravir and lenacapavir. Figures 7A and 7B show the plasma concentration of bictegravir and Figures 8A and 8B show the plasma concentration of lenacapavir as a function of time.
Figures 9A and 9B show the results of dissolution studies carried out on bilayer tablets formulations comprising different fillers in the bictegravir layer, as compared to the dissolution from a combination of single agent tablets comprising bictegravir and lenacapavir, respectively. Figure 9A shows the dissolution of bictegravir from the tested tablet formulations, and Figure 9B shows the dissolution of lenacapavir from the tested tablet formulations.

### DETAILED DESCRIPTION

The tablets disclosed herein comprise two active pharmaceutical ingredients (APIs): bictegravir (or a pharmaceutically acceptable salt thereof) and lenacapavir (or a pharmaceutically acceptable salt thereof).

### Bictegravir

Bictegravir (BIC) is a potent HIV integrase inhibitor with *in vitro* activity against wild type HIV-1. It has the following formula (see WO2014/100323):

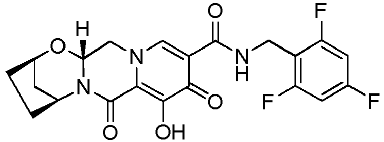

Its IUPAC name is (2R,5S,13aR)-8-hydroxy-7,9-dioxo-N-(2,4,6-trifluorobenzyl)-2,3,4,5,7,9,13,13a-octahydro-2,5-methanopyrido[1',2':4,5]pyrazino[2,1-b][1,3]oxazepine-10-carboxamide. Its CAS name is 2,5-Methanopyrido[1',2':4,5]pyrazino[2,1-b][1,3]oxazepine-10-carboxamide, 2,3,4,5,7,9,13,13a-octahydro-8-hydroxy-7,9-dioxo-N-[(2,4,6-trifluorophenyl)methyl]-, (2R,5S,13aR).

The tablets disclosed herein include bictegravir, usually in the form of a pharmaceutically acceptable salt. Bictegravir or its salts can be present within a tablet in solvated or unsolvated form, and references to "bictegravir" include both of these forms. In particular, references to bictegravir or a pharmaceutically acceptable salt thereof include pharmaceutically acceptable salts of bictegravir and pharmaceutically acceptable hydrates of bictegravir. In some embodiments, bictegravir is included as its sodium salt (bictegravir sodium), having the formula below:

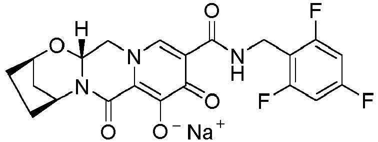

As used herein, and in the absence of a specific reference to a particular pharmaceutically acceptable salt and/or solvate of the compound of bictegravir, any dosages, whether expressed in *e.g.* milligrams or as a % by weight, should be taken as referring to the amount of bictegravir free acid, *i.e.* the amount of:

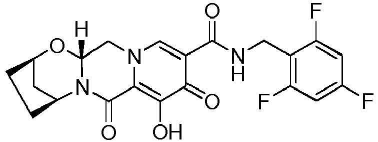

Tablets are provided containing an amount of bictegravir or a pharmaceutically acceptable salt thereof corresponding to about 50-100 mg of bictegravir free acid *(e.g.* as about 52-105 mg of bictegravir sodium).

The amount of bictegravir or a pharmaceutically acceptable salt thereof in the tablet may correspond to about 65-85 mg of bictegravir free acid *(e.g.* as about 68-89 mg of bictegravir sodium).

The amount of bictegravir or a pharmaceutically acceptable salt thereof in the tablet may correspond to about 75-100 mg of bictegravir free acid *(e.g.* as about 78-105 mg of bictegravir sodium).

The amount of bictegravir or a pharmaceutically acceptable salt thereof in the tablet may correspond to about 50-75 mg of bictegravir free acid *(e.g.* as about 52-78 mg of bictegravir sodium).

The amount of bictegravir or a pharmaceutically acceptable salt thereof in the tablet may correspond to about 60-75 mg of bictegravir free acid *(e.g.* as about 63-78 mg of bictegravir sodium).

The amount of bictegravir or a pharmaceutically acceptable salt thereof in the tablet may correspond to about 100 mg of bictegravir free acid *(e.g.* as about 105 mg of bictegravir sodium).

The amount of bictegravir or a pharmaceutically acceptable salt thereof in the tablet may correspond to about 60 mg of bictegravir free acid *(e.g.* as about 63 mg of bictegravir sodium).

The amount of bictegravir or a pharmaceutically acceptable salt thereof in the tablet may correspond to about 50 mg of bictegravir free acid *(e.g.* as about 52 mg of bictegravir sodium).

In one embodiment, the amount of bictegravir or a pharmaceutically acceptable salt thereof in the tablet of the disclosure corresponds to about 75 mg of bictegravir free acid (*e.g.* as about 78 mg of bictegravir sodium).

### Lenacapavir

Lenacapavir (LEN) is a first-in-class capsid inhibitor with multistage, selective activity against the HIV capsid protein. It has the following formula (see WO2018/035359):

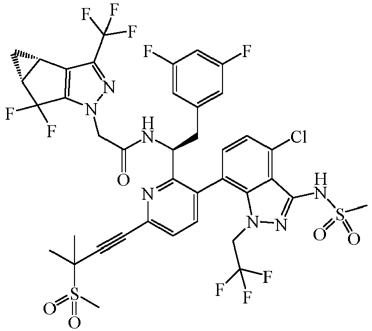

Its IUPAC name is N-((S)-1-(3-(4-chloro-3-(methylsulfonamido)-1-(2,2,2-trifluoroethyl)-1H-indazol-7-yl)-6-(3-methyl-3-(methylsulfonyl)but-1-yn-1-yl)pyrindin-2-yl)-2-(3,5-difluorophenyl)ethyl)-2-(5,5-difluoro-3-(trifluoromethyl)-3b,4,4a,5-tetrahydro-1H-cyclopropa[3,4]cyclopenta[1,2-c]pyrazol-1-yl)acetamide.

The tablets disclosed herein include lenacapavir, usually in the form of a pharmaceutically acceptable salt. Lenacapavir or its salts can be present within an oral dosage form in solvated or unsolvated form, and references to "lenacapavir" include both of these forms. In particular, references to lenacapavir or a pharmaceutically acceptable salt thereof include pharmaceutically acceptable salts of lenacapavir and pharmaceutically acceptable hydrates of lenacapavir. In some embodiments, lenacapavir is included as its sodium salt (lenacapavir sodium), having the formula below:

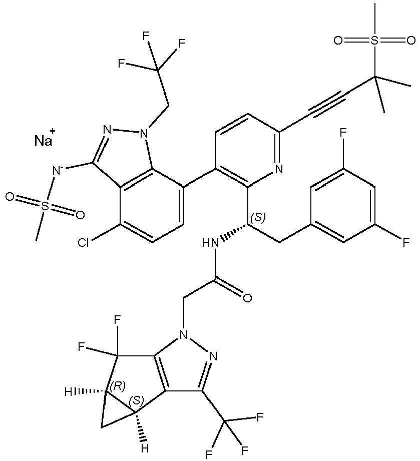

As used herein, and in the absence of a specific reference to a particular pharmaceutically acceptable salt and/or solvate of the compound of lenacapavir (*e.g.* lenacapavir sodium), any dosages, whether expressed in e.g. milligrams or as a % by weight, should be taken as referring to the amount of lenacapavir free acid, *i.e.* the amount of:

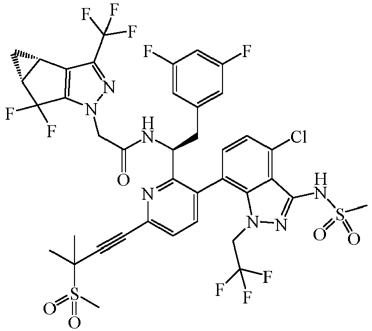

Tablets are provided containing an amount of lenacapavir or a pharmaceutically acceptable salt thereof corresponding to about 25-100 mg of lenacapavir free acid *(e.g.* as about 26-102 mg of lenacapavir sodium).

The amount of lenacapavir or a pharmaceutically acceptable salt thereof in the tablet may correspond to about 25-75 mg of lenacapavir free acid *(e.g.* as about 26-77 mg of lenacapavir sodium).

The amount of lenacapavir or a pharmaceutically acceptable salt thereof in the tablet may correspond to about 25-50 mg of lenacapavir free acid *(e.g.* as about 26-51 mg of lenacapavir sodium).

The amount of lenacapavir or a pharmaceutically acceptable salt thereof in the tablet may correspond to about 50-75 mg of lenacapavir free acid *(e.g.* as about 51-77 mg of lenacapavir sodium).

The amount of lenacapavir or a pharmaceutically acceptable salt thereof in the tablet may correspond to about 40-60 mg of lenacapavir free acid *(e.g.* as about 41-61 mg of lenacapavir sodium).

The amount of lenacapavir or a pharmaceutically acceptable salt thereof in the tablet may correspond to about 75 mg of lenacapavir free acid *(e.g.* as about 77 mg of lenacapavir sodium).

In one embodiment, the amount of lenacapavir or a pharmaceutically acceptable salt thereof corresponds to about 50 mg of lenacapavir free acid *(e.g.* as about 51 mg of lenacapavir sodium). In one embodiment, the amount of lenacapavir or a pharmaceutically acceptable salt thereof corresponds to about 25 mg of lenacapavir free acid *(e.g.* as about 26 mg of lenacapavir sodium).

### Dosage forms

The inventors have formulated bictegravir and lenacapavir into a single tablet that provides the combined therapeutic effect of each individual active agent. In addition to the clinical benefits described above that may result from the use of bictegravir and lenacapavir, the tablets of the present disclosure afford further advantages by virtue of being a single fixed dose combination tablet. In particular, the tablet provides both bictegravir and lenacapavir in amounts so as to be comparable to or better than the combination of single agent tablets containing equivalent amounts of bictegravir and lenacapavir in terms of pharmacokinetic properties. Also, the tablets of the disclosure reduce the patient pill burden and treatment regimen complexity, which may lead to improved patient compliance and reduced likelihood of virologic failure.

The provision of a tablet with particular pharmacokinetic parameters that are comparable to or better than the combination of the single agent tablets containing equivalent amounts of bictegravir and lenacapavir is a particular advantage afforded by the present disclosure. Achieving such pharmacokinetic properties may require the use of specific dosages of bictegravir and lenacapavir.

The tablets disclosed herein are intended for pharmaceutical use in human subjects. Accordingly, they must be of an appropriate size and weight for oral human administration. In some embodiments, they have a total weight of less than about 1.5 g. In some embodiments, they are less than about 1.0 g.

### Dosages

A tablet comprising bictegravir or a pharmaceutically acceptable salt thereof and lenacapavir or a pharmaceutically acceptable salt thereof is provided, wherein the amount of bictegravir or pharmaceutically acceptable salt thereof corresponds to about 50-100 mg bictegravir free acid, and the amount of lenacapavir or pharmaceutically acceptable salt thereof corresponds to about 25-100 mg lenacapavir free acid.

The amount of bictegravir or pharmaceutically acceptable salt thereof in the tablet may correspond to about 65-85 mg bictegravir free acid. The amount of bictegravir or pharmaceutically acceptable salt thereof in the tablet may correspond to about 75-100 mg bictegravir free acid. The amount of bictegravir or pharmaceutically acceptable salt thereof in the tablet may correspond to about 50-75 mg bictegravir free acid.

The amount of lenacapavir or pharmaceutically acceptable salt thereof in the tablet may correspond to about 25-75 mg lenacapavir free acid. The amount of lenacapavir or pharmaceutically acceptable salt thereof in the tablet may correspond to about 25-50 mg lenacapavir free acid. The amount of lenacapavir or pharmaceutically acceptable salt thereof in the tablet may correspond to about 50-75 mg lenacapavir free acid. The amount of lenacapavir or a pharmaceutically acceptable salt thereof in the tablet may correspond to about 40-60 mg of about lenacapavir free acid.

The amount of bictegravir or pharmaceutically acceptable salt thereof may correspond to about 75-100 mg bictegravir free acid and the amount of lenacapavir or pharmaceutically acceptable salt thereof may correspond to about 25-75 mg lenacapavir free acid. The amount of bictegravir or pharmaceutically acceptable salt thereof may correspond to about 75-100 mg bictegravir free acid and the amount of lenacapavir or pharmaceutically acceptable salt thereof may correspond to about 25-50 mg lenacapavir free acid. The amount of bictegravir or pharmaceutically acceptable salt thereof may correspond to about 75-100 mg bictegravir free acid and the amount of lenacapavir or pharmaceutically acceptable salt thereof may correspond to about 50-75 mg lenacapavir free acid. The amount of bictegravir or pharmaceutically acceptable salt thereof may correspond to about 75-100 mg bictegravir free acid and the amount of lenacapavir or pharmaceutically acceptable salt thereof may correspond to about 40-60 mg lenacapavir free acid.

The amount of bictegravir or pharmaceutically acceptable salt thereof may correspond to about 50-75 mg bictegravir free acid and the amount of lenacapavir or pharmaceutically acceptable salt thereof may correspond to about 25-75 mg lenacapavir free acid. The amount of bictegravir or pharmaceutically acceptable salt thereof may correspond to about 50-75 mg bictegravir free acid and the amount of lenacapavir or pharmaceutically acceptable salt thereof may correspond to about 25-50 mg lenacapavir free acid. The amount of bictegravir or pharmaceutically acceptable salt thereof may correspond to about 50-75 mg bictegravir free acid and the amount of lenacapavir or pharmaceutically acceptable salt thereof may correspond to about 50-75 mg lenacapavir free acid. The amount of bictegravir or pharmaceutically acceptable salt thereof may correspond to about 50-75 mg bictegravir free acid and the amount of lenacapavir or pharmaceutically acceptable salt thereof may correspond to about 40-60 mg lenacapavir free acid.

The amount of bictegravir or pharmaceutically acceptable salt thereof may correspond to about 65-85 mg bictegravir free acid and the amount of lenacapavir or pharmaceutically acceptable salt thereof may correspond to about 25-75 mg lenacapavir free acid. The amount of bictegravir or pharmaceutically acceptable salt thereof may correspond to about 65-85 mg bictegravir free acid and the amount of lenacapavir or pharmaceutically acceptable salt thereof may correspond to about 50-75 mg lenacapavir free acid.

In some embodiments, the amount of bictegravir or pharmaceutically acceptable salt thereof corresponds to about 65-85 mg bictegravir free acid and the amount of lenacapavir or pharmaceutically acceptable salt thereof corresponds to about 25-50 mg lenacapavir free acid. In some embodiments, the amount of bictegravir or pharmaceutically acceptable salt thereof corresponds to about 65-85 mg bictegravir free acid and the amount of lenacapavir or pharmaceutically acceptable salt thereof corresponds to about 25 mg or about 50 mg lenacapavir free acid.

The amount of bictegravir or pharmaceutically acceptable salt thereof in the tablet may correspond to about 50, 60, 75, or 100 mg bictegravir free acid. In some embodiments, the amount of bictegravir or pharmaceutically acceptable salt thereof corresponds to about 50 mg bictegravir free acid. In some embodiments, the amount of bictegravir or pharmaceutically acceptable salt thereof corresponds to about 75 mg bictegravir free acid. In some embodiments, the amount of bictegravir or pharmaceutically acceptable salt thereof corresponds to about 100 mg bictegravir free acid.

In one embodiment, the amount of bictegravir or pharmaceutically acceptable salt thereof corresponds to about 75 mg bictegravir free acid.

The amount of lenacapavir or pharmaceutically acceptable salt thereof in the tablet may correspond to about 25, 50, 75, or 100 mg lenacapavir free acid. In some embodiments, the amount of lenacapavir or pharmaceutically acceptable salt thereof corresponds to about 100 mg lenacapavir free acid. In some embodiments, the amount of lenacapavir or pharmaceutically acceptable salt thereof corresponds to about 75 mg lenacapavir free acid.

In one embodiment, the amount of lenacapavir or pharmaceutically acceptable salt thereof corresponds to about 25 or about 50 mg lenacapavir free acid. In one embodiment, the tablet contains an amount of bictegravir or pharmaceutically acceptable salt thereof that corresponds to about 75 mg bictegravir free acid and an amount of lenacapavir or pharmaceutically acceptable salt thereof that corresponds to about 25 mg lenacapavir free acid. In one embodiment, the tablet contains an amount of bictegravir or pharmaceutically acceptable salt thereof that corresponds to about 75 mg bictegravir free acid and an amount of lenacapavir or pharmaceutically acceptable salt thereof that corresponds to about 50 mg lenacapavir free acid.

A tablet comprising bictegravir or a pharmaceutically acceptable salt thereof and lenacapavir or a pharmaceutically acceptable salt thereof is provided, wherein the amount of bictegravir or pharmaceutically acceptable salt thereof corresponds to about 75 mg bictegravir free acid, and the amount of lenacapavir or pharmaceutically acceptable salt thereof corresponds to about 100 mg lenacapavir free acid.

A tablet comprising bictegravir or a pharmaceutically acceptable salt thereof and lenacapavir or a pharmaceutically acceptable salt thereof is provided, wherein the amount of bictegravir or pharmaceutically acceptable salt thereof corresponds to about 75 mg bictegravir free acid, and the amount of lenacapavir or pharmaceutically acceptable salt thereof corresponds to about 75 mg lenacapavir free acid.

In one embodiment, a tablet comprises bictegravir or a pharmaceutically acceptable salt thereof and lenacapavir or a pharmaceutically acceptable salt thereof, wherein the amount of bictegravir or pharmaceutically acceptable salt thereof corresponds to about 75 mg bictegravir free acid, and the amount of lenacapavir or pharmaceutically acceptable salt thereof corresponds to about 25 mg lenacapavir free acid. In some embodiments, this tablet is a bilayer tablet.

In one embodiment, a tablet comprises bictegravir or a pharmaceutically acceptable salt thereof and lenacapavir or a pharmaceutically acceptable salt thereof, wherein the amount of bictegravir or pharmaceutically acceptable salt thereof corresponds to about 75 mg bictegravir free acid, and the amount of lenacapavir or pharmaceutically acceptable salt thereof corresponds to about 50 mg lenacapavir free acid. In some embodiments, this tablet is a bilayer tablet.

### Monolayer tablets

The tablets of the present disclosure may be in the form of a monolayer tablet. In one embodiment, a tablet comprising bictegravir or a pharmaceutically acceptable salt thereof and lenacapavir or a pharmaceutically acceptable salt thereof is provided, wherein the amount of bictegravir or pharmaceutically acceptable salt thereof corresponds to about 50-100 mg bictegravir free acid, and the amount of lenacapavir or pharmaceutically acceptable salt thereof corresponds to about 25-100 mg lenacapavir free acid, and wherein the tablet is a monolayer tablet.

In some embodiments, the tablet is a monolayer tablet and the amount of bictegravir or pharmaceutically acceptable salt thereof corresponds to about 65-85 mg bictegravir free acid. In some embodiments, the tablet is a monolayer tablet and the amount of bictegravir or pharmaceutically acceptable salt thereof corresponds to about 75-100 mg bictegravir free acid. In some embodiments, the tablet is a monolayer tablet and the amount of bictegravir or pharmaceutically acceptable salt thereof corresponds to about 50-75 mg bictegravir free acid.

In some embodiments, the tablet is a monolayer tablet and the amount of lenacapavir or pharmaceutically acceptable salt thereof corresponds to about 25-75 mg lenacapavir free acid. In some embodiments, the tablet is a monolayer tablet and the amount of lenacapavir or pharmaceutically acceptable salt thereof corresponds to about 25-50 mg lenacapavir free acid. In some embodiments, the tablet is a monolayer tablet and the amount of lenacapavir or pharmaceutically acceptable salt thereof corresponds to about 50-75 mg lenacapavir free acid.

In a monolayer tablet, the amount of bictegravir or pharmaceutically acceptable salt thereof may correspond to about 65-85 mg bictegravir free acid and the amount of lenacapavir or pharmaceutically acceptable salt thereof may correspond to about 25-75 mg lenacapavir free acid. In some embodiments, in a monolayer tablet, the amount of bictegravir or pharmaceutically acceptable salt thereof corresponds to about 65-85 mg bictegravir free acid and the amount of lenacapavir or pharmaceutically acceptable salt thereof corresponds to about 40-60 mg lenacapavir free acid.

The amount of bictegravir or pharmaceutically acceptable salt thereof in a monolayer tablet may correspond to about 50, 60, 75, or 100 mg bictegravir free acid. The amount of bictegravir or pharmaceutically acceptable salt thereof in a monolayer tablet may correspond to about 50 mg bictegravir free acid. The amount of bictegravir or pharmaceutically acceptable salt thereof in a monolayer tablet may correspond to about 60 mg bictegravir free acid. The amount of bictegravir or pharmaceutically acceptable salt thereof in a monolayer tablet may correspond to about 100 mg bictegravir free acid. In some embodiments, the amount of bictegravir or pharmaceutically acceptable salt thereof in a monolayer tablet corresponds to about 75 mg bictegravir free acid.

The amount of lenacapavir or pharmaceutically acceptable salt thereof in a monolayer tablet may correspond to about 25, 50, 75, or 100 mg lenacapavir free acid. The amount of lenacapavir or pharmaceutically acceptable salt thereof in a monolayer tablet may correspond to about 100 mg lenacapavir free acid. The amount of lenacapavir or pharmaceutically acceptable salt thereof in a monolayer tablet may correspond to about 75 mg lenacapavir free acid. The amount of lenacapavir or pharmaceutically acceptable salt thereof in a monolayer tablet may correspond to about 50 mg lenacapavir free acid. In some embodiments, the amount of lenacapavir or pharmaceutically acceptable salt thereof in a monolayer tablet corresponds to about 25 mg lenacapavir free acid.

In one embodiment, a monolayer tablet comprising bictegravir or a pharmaceutically acceptable salt thereof and lenacapavir or a pharmaceutically acceptable salt thereof is provided, wherein the amount of bictegravir or pharmaceutically acceptable salt thereof corresponds to about 75 mg bictegravir free acid, and the amount of lenacapavir or pharmaceutically acceptable salt thereof corresponds to about 100 mg lenacapavir free acid.

In one embodiment, a monolayer tablet comprising bictegravir or a pharmaceutically acceptable salt thereof and lenacapavir or a pharmaceutically acceptable salt thereof is provided, wherein the amount of bictegravir or pharmaceutically acceptable salt thereof corresponds to about 75 mg bictegravir free acid, and the amount of lenacapavir or pharmaceutically acceptable salt thereof corresponds to about 75 mg lenacapavir free acid.

In one embodiment, a monolayer tablet comprising bictegravir or a pharmaceutically acceptable salt thereof and lenacapavir or a pharmaceutically acceptable salt thereof is provided, wherein the amount of bictegravir or pharmaceutically acceptable salt thereof corresponds to about 75 mg bictegravir free acid, and the amount of lenacapavir or pharmaceutically acceptable salt thereof corresponds to about 50 mg lenacapavir free acid

In one embodiment, a monolayer tablet comprising bictegravir or a pharmaceutically acceptable salt thereof and lenacapavir or a pharmaceutically acceptable salt thereof is provided, wherein the amount of bictegravir or pharmaceutically acceptable salt thereof corresponds to about 75 mg bictegravir free acid, and the amount of lenacapavir or pharmaceutically acceptable salt thereof corresponds to about 25 mg lenacapavir free acid.

The present disclosure provides a monolayer tablet of bictegravir and lenacapavir which provides dissolution and bioavailability of both active ingredients which is at least comparable to the combination of the single agent tablets containing equivalent amounts of bictegravir and lenacapavir.

### Multilayer tablets

The tablets of the present disclosure may be in the form of a multilayer tablet. In one embodiment, the tablet is a bilayer tablet. The inventors have found that the dissolution of lenacapavir is impacted by the presence of bictegravir and *vice versa* when formulated into a monolayer tablet at certain dosages. The inventors have discovered that such interactions may be overcome by separating the APIs by use of a bilayer tablet. The pharmacokinetic properties (including, but not limited to, AUC_{inf}, AUCₗₐₛₜ and T_{1/2}) of certain bilayer tablets may improve relative to monolayer tablets containing the same amounts of bictegravir and lenacapavir and relative to single agent tablets containing the same amounts of bictegravir and lenacapavir. A tablet comprising bictegravir or a pharmaceutically acceptable salt thereof and lenacapavir or a pharmaceutically acceptable salt thereof is provided, wherein the amount of bictegravir or pharmaceutically acceptable salt thereof corresponds to about 50-100 mg bictegravir free acid, and the amount of lenacapavir or pharmaceutically acceptable salt thereof corresponds to about 25-100 mg lenacapavir free acid, and wherein the tablet is a multilayer tablet. In some embodiments, the tablet is a bilayer tablet.

In some embodiments, the bilayer tablet comprises (a) a first layer comprising bictegravir or a pharmaceutically acceptable salt thereof, and (b) a second layer comprising lenacapavir or a pharmaceutically acceptable salt thereof. In some embodiments, the first layer is substantially free of lenacapavir or a pharmaceutically acceptable salt thereof. In some embodiments, the first layer contains less than about 1% by weight lenacapavir or a pharmaceutically acceptable salt thereof. In some embodiments, the second layer is substantially free of bictegravir or a pharmaceutically acceptable salt thereof. In some embodiments, the second layer contains less than about 1% by weight bictegravir or a pharmaceutically acceptable salt thereof. In one embodiment, the first layer is free of lenacapavir or a pharmaceutically acceptable salt thereof. In one embodiment, the second layer is free of bictegravir or a pharmaceutically acceptable salt thereof. In one embodiment, the first layer is free of lenacapavir or a pharmaceutically acceptable salt thereof, and the second layer is free of bictegravir or a pharmaceutically acceptable salt thereof.

In some embodiments, the bilayer tablet comprises (a) a first layer comprising lenacapavir or a pharmaceutically acceptable salt thereof, and (b) a second layer comprising bictegravir or a pharmaceutically acceptable salt thereof. In some embodiments, the first layer is substantially free of bictegravir or a pharmaceutically acceptable salt thereof. In some embodiments, the first layer contains less than about 1% by weight bictegravir or a pharmaceutically acceptable salt thereof. In some embodiments, the second layer is substantially free of lenacapavir or a pharmaceutically acceptable salt thereof. In some embodiments, the second layer contains less than about 1% by weight lenacapavir or a pharmaceutically acceptable salt thereof. In one embodiment, the first layer is free of bictegravir or a pharmaceutically acceptable salt thereof. In one embodiment, the second layer is free of lenacapavir or a pharmaceutically acceptable salt thereof. In one embodiment, the first layer is free of bictegravir or a pharmaceutically acceptable salt thereof, and the second layer is free of lenacapavir or a pharmaceutically acceptable salt thereof.

In some embodiments, the amount of bictegravir or pharmaceutically acceptable salt thereof in the bilayer tablet may correspond to about 50, 60, 75, or 100 mg bictegravir free acid. In some embodiments, the amount of bictegravir or pharmaceutically acceptable salt thereof in the bilayer tablet may correspond to about 50 mg bictegravir free acid. In some embodiments, the amount of bictegravir or pharmaceutically acceptable salt thereof in the bilayer tablet may correspond to about 60 mg bictegravir free acid. In some embodiments, the amount of bictegravir or pharmaceutically acceptable salt thereof in the bilayer tablet may correspond to about 75 mg bictegravir free acid. In some embodiments, the amount of bictegravir or pharmaceutically acceptable salt thereof in the bilayer tablet may corresponds to about 100 mg bictegravir free acid.

In some embodiments, the tablet is a bilayer tablet and the amount of lenacapavir or pharmaceutically acceptable salt thereof corresponds to about 25-75 mg lenacapavir free acid. In some embodiments, tablet is a bilayer tablet and the amount of lenacapavir or pharmaceutically acceptable salt thereof corresponds to about 25-50 mg lenacapavir free acid. In some embodiments, the tablet is a bilayer tablet and the amount of lenacapavir or pharmaceutically acceptable salt thereof corresponds to about 50-75 mg lenacapavir free acid. In some embodiments, the tablet is a bilayer tablet and the amount of lenacapavir or pharmaceutically acceptable salt thereof corresponds to about 40-60 mg lenacapavir free acid.

In some embodiments, the amount of lenacapavir or pharmaceutically acceptable salt thereof in the bilayer tablet may correspond to about 25, 50, 75, or 100 mg lenacapavir free acid. In some embodiments, the amount of lenacapavir or pharmaceutically acceptable salt thereof in the bilayer tablet may correspond to about 100 mg lenacapavir free acid. In some embodiments, the amount of lenacapavir or pharmaceutically acceptable salt thereof in the bilayer tablet may correspond to about 75 mg lenacapavir free acid. In some embodiments, the amount of lenacapavir or pharmaceutically acceptable salt thereof in the bilayer tablet may correspond to about 50 mg lenacapavir free acid. In some embodiments, the amount of lenacapavir or pharmaceutically acceptable salt thereof in the bilayer tablet may correspond to about 25 mg lenacapavir free acid.

In some embodiments, the tablet is a bilayer tablet, the amount of bictegravir or pharmaceutically acceptable salt thereof corresponds to about 65-85 mg bictegravir free acid and the amount of lenacapavir or pharmaceutically acceptable salt thereof corresponds to about 25-75 mg lenacapavir free acid. In some embodiments, the tablet is a bilayer tablet, the amount of bictegravir or pharmaceutically acceptable salt thereof corresponds to about 65-85 mg bictegravir free acid and the amount of lenacapavir or pharmaceutically acceptable salt thereof corresponds to about 25-50 mg lenacapavir free acid.

In some embodiments, in a bilayer tablet, the amount of bictegravir or pharmaceutically acceptable salt thereof corresponds to about 65-85 mg bictegravir free acid and the amount of lenacapavir or pharmaceutically acceptable salt thereof corresponds to about 25 mg lenacapavir free acid. In some embodiments, the amount of lenacapavir or pharmaceutically acceptable salt thereof corresponds to about 50 mg lenacapavir free acid.

In one embodiment, a bilayer tablet is provided comprising (a) a first layer comprising bictegravir or a pharmaceutically acceptable salt thereof, wherein the amount of bictegravir or pharmaceutically acceptable salt thereof corresponds to about 50-100 mg bictegravir free acid, and (b) a second layer comprising lenacapavir or a pharmaceutically acceptable salt thereof, wherein the amount of lenacapavir or pharmaceutically acceptable salt thereof corresponds to about 25-100 mg lenacapavir free acid. In some embodiments, the tablet is a bilayer tablet and the amount of bictegravir or pharmaceutically acceptable salt thereof corresponds to about 65-85 mg bictegravir free acid. In some embodiments, the tablet is a bilayer tablet and the amount of bictegravir or pharmaceutically acceptable salt thereof corresponds to about 75-100 mg bictegravir free acid. In some embodiments, the tablet is a bilayer tablet and the amount of bictegravir or pharmaceutically acceptable salt thereof corresponds to about 50-75 mg bictegravir free acid. In some embodiments, tablet is a bilayer tablet and the amount of bictegravir or pharmaceutically acceptable salt thereof corresponds to about 75 mg bictegravir free acid.

In some embodiments, a bilayer tablet is provided comprising (a) a first layer comprising bictegravir or a pharmaceutically acceptable salt thereof, wherein the amount of bictegravir or pharmaceutically acceptable salt thereof corresponds to about 75 mg bictegravir free acid, and (b) a second layer comprising lenacapavir or a pharmaceutically acceptable salt thereof, wherein the amount of lenacapavir or pharmaceutically acceptable salt thereof corresponds to about 50 mg lenacapavir free acid.

In some embodiments, a bilayer tablet is provided comprising (a) a first layer comprising bictegravir or a pharmaceutically acceptable salt thereof, wherein the amount of bictegravir or pharmaceutically acceptable salt thereof corresponds to about 75 mg bictegravir free acid, and (b) a second layer comprising lenacapavir or a pharmaceutically acceptable salt thereof, wherein the amount of lenacapavir or pharmaceutically acceptable salt thereof corresponds to about 25 mg lenacapavir free acid.

In some embodiments, the bilayer tablet comprises (a) a first layer comprising lenacapavir or a pharmaceutically acceptable salt thereof, wherein the amount of lenacapavir or pharmaceutically acceptable salt thereof corresponds to about 50 mg lenacapavir free acid; and (b) a second layer comprising bictegravir or a pharmaceutically acceptable salt thereof, wherein the amount of bictegravir or pharmaceutically acceptable salt thereof corresponds to about 75 mg bictegravir free acid.

In some embodiments, the bilayer tablet comprises (a) a first layer comprising lenacapavir or a pharmaceutically acceptable salt thereof, wherein the amount of lenacapavir or pharmaceutically acceptable salt thereof corresponds to about 25 mg lenacapavir free acid; and (b) a second layer comprising bictegravir or a pharmaceutically acceptable salt thereof, wherein the amount of bictegravir or pharmaceutically acceptable salt thereof corresponds to about 75 mg bictegravir free acid.

### Dissolution

The tablets disclosed herein are typically immediate release tablets.

In one embodiment, the disclosure provides a tablet which releases at least about 50% of bictegravir or pharmaceutically acceptable salt thereof in about 30 minutes, measured using USP apparatus II, in 250 mL of fasted state simulated intestinal fluid, pH 6.5, at 37°C and paddle speed of 75 rpm. In some embodiments, the tablet disclosed herein releases at least about 60% of bictegravir or pharmaceutically acceptable salt thereof in about 30 minutes, measured using USP apparatus II, in 250 mL of fasted state simulated intestinal fluid, pH 6.5, at 37°C and paddle speed of 75 rpm. In some embodiments, a tablet that releases at least about 70% of bictegravir or pharmaceutically acceptable salt thereof in about 30 minutes is provided, measured using USP apparatus II, in 250 mL of fasted state simulated intestinal fluid, pH 6.5, at 37°C and paddle speed of 75 rpm.

In one embodiment, the disclosure provides a tablet which releases at least about 50% of lenacapavir or pharmaceutically acceptable salt thereof in about 15 minutes, measured using USP apparatus II, in 900 mL of 2% w/w Cremophor EL in 30 mM potassium phosphate, pH 6.0, at 37°C and paddle speed of 75 rpm. In some embodiments, the tablet releases at least about 70% of lenacapavir or pharmaceutically acceptable salt thereof in about 15 minutes, measured using USP apparatus II, in 900 mL of 2% w/w Cremophor EL in 30 mM potassium phosphate, pH 6.0, at 37°C and paddle speed of 75 rpm. In some embodiments, a tablet that releases at least about 80% of lenacapavir or pharmaceutically acceptable salt thereof in about 15 minutes is provided, measured using USP apparatus II, in 900 mL of 2% w/w Cremophor EL in 30 mM potassium phosphate, pH 6.0, at 37°C and paddle speed of 75 rpm.

In some embodiments, the method of measuring the dissolution of lenacapavir from the tablets is performed on a batch of tablets containing an amount of lenacapavir corresponding to about 300 mg lenacapavir free acid. Accordingly, in one embodiment, the disclosure provides a tablet which releases at least about 50% of lenacapavir or pharmaceutically acceptable salt thereof in about 15 minutes, measured using USP apparatus II, in 900 mL of 2% w/w Cremophor EL in 30 mM potassium phosphate, pH 6.0, at 37°C and paddle speed of 75 rpm, and wherein the dissolution method is performed on a batch of tablets containing an amount of lenacapavir corresponding to about 300 mg lenacapavir free acid. In some embodiments, the tablet releases at least about 70% of lenacapavir or pharmaceutically acceptable salt thereof in about 15 minutes, measured using USP apparatus II, in 900 mL of 2% w/w Cremophor EL in 30 mM potassium phosphate, pH 6.0, at 37°C and paddle speed of 75 rpm, and wherein the dissolution method is performed on a batch of tablets containing an amount of lenacapavir corresponding to about 300 mg lenacapavir free acid. In some embodiments, a tablet that releases at least about 80% of lenacapavir or pharmaceutically acceptable salt thereof in about 15 minutes is provided, measured using USP apparatus II, in 900 mL of 2% w/w Cremophor EL in 30 mM potassium phosphate, pH 6.0, at 37°C and paddle speed of 75 rpm, and wherein the dissolution method is performed on a batch of tablets containing an amount of lenacapavir corresponding to 300 mg lenacapavir free acid.

### Excipients

Tablets will typically include one or more excipients. Excipients should be compatible with the other ingredients of the formulation and physiologically innocuous to the recipient thereof. Examples of suitable excipients are well known to the person skilled in the art of tablet formulation and may be found *e.g.* in *Handbook of Pharmaceutical Excipients* (eds. Rowe, Sheskey & Quinn), 6th edition **2009.** As used herein the term "excipients" is intended to refer to *inter alia* basifying agents, solubilisers, glidants, fillers, binders, lubricant, diluents, preservatives, surface active agents, dispersing agents and the like. The term also includes agents such as sweetening agents, flavoring agents, coloring agents and preserving agents. Such components will generally be present in admixture within the tablet.

In some embodiments, tablets can contain excipients including glidants, fillers, binders, polymers and the like. In some embodiments, examples of polymers include hydroxypropylmethyl cellulose (HPMC), polyvinylpyrrolidone (PVP), polyvinylpyrrolidone vinyl acetate (PVP-VA), and hydroxypropyl methylcellulose acetate succinate (HPMC-AS). In some embodiments, the polymer is copovidone. In some embodiments, tablets provided herein include about 5-15 mg copovidone, such as about 10-25 mg copovidone or 5-8 mg copovidone. In some embodiments, the tablets provided herein include 1-6 % w/w copovidone.

Examples of solubilisers include, but are not limited to, ionic surfactants (including both ionic and non-ionic surfactants) such as sodium lauryl sulphate, cetyltrimethylammonium bromide, polysorbates (such as polysorbate 20 or 80), poloxamers (such as poloxamer 188, 207, or 407), and macrogols. The tablets of the disclosure may include a poloxamer, in particular poloxamer 407. In some embodiments, the amount of poloxamer is about 0.5-7 mg, such as about 1-3 mg or about 2-5 mg. In some embodiments, the amount of poloxamer is about 0.1-1.6 %w/w, such as about 0.3-0.8 %w/w.

Examples of lubricants, glidants and flow aids include, but are not limited to, magnesium stearate, calcium stearate, stearic acid, hydrogenated vegetable oil, glyceryl palmitostearate, glyceryl behenate, sodium stearyl fumarate, colloidal silicon dioxide, and talc. In some embodiments, the lubricant is magnesium stearate. In some embodiments, the tablets include about 1-50 mg magnesium stearate, such as about 5-10 mg, in some embodiments, about 5-8 mg. In some embodiments, the tablets include about 1-2 %w/w magnesium stearate.

Examples of disintegrants include, but are not limited to, starches, celluloses, cross-linked PVP, sodium starch glycolate, croscarmellose sodium, *etc.* In some embodiments, the disintegrant is croscarmellose sodium. In some embodiments, the tablets include about 25-50 mg croscarmellose sodium, such as about 30-50 mg. In some embodiments, the tablets include about 3-10 %w/w croscarmellose sodium.

Examples of binders include, but are not limited to, cross-linked PVP, HPMC, microcrystalline cellulose, sucrose, starches, *etc.*

Examples of fillers (also known as bulking agents or diluents) include, but are not limited to, starches, maltodextrins, polyols (such as lactose), and celluloses. Tablets provided herein may include microcrystalline cellulose and/or mannitol. In some embodiments, the tablet includes microcrystalline cellulose. Tablets provided herein may include 70-250 mg microcrystalline cellulose, such as about 70-100 mg or about 200-250 mg. Tablets provided herein may include about 20-50 %w/w microcrystalline cellulose, such as about 15-25 %w/w or about 40-50 %w/w. Tablets provided herein may include 50-200 mg mannitol, such as 150-200 mg, *e.g.* 50-125 mg. Tablets provided herein may include about 10-40 %w/w mannitol, such as about 10-25 %w/w or about 35-40 %w/w.

In some embodiments, the tablet is a bilayer tablet comprising (a) a first layer comprising the bictegravir or a pharmaceutically acceptable salt thereof and (b) a second layer comprising the lenacapavir or a pharmaceutically acceptable salt thereof, wherein the first layer comprises a filler selected from mannitol or microcrystalline cellulose or a combination thereof. In such tablets, the weight ratio of mannitol to microcrystalline cellulose in the first layer may be about 2:1 to about 1:2 such as about 2:1, about 1:1, or about 1:2. The inventors have found that the use of microcrystalline cellulose as the only filler in the bictegravir layer of the bilayer tablet resulted in improved processability and manufacturability, as compared to a bilayer tablet using both mannitol and microcrystalline cellulose as the filler in the bictegravir layer. Accordingly, in some embodiments of the tablets, the filler in the first layer is microcrystalline cellulose and the first layer is substantially free from mannitol. In some embodiments, the first layer contains less than 1 %w/w mannitol. In some embodiments, the first layer contains no mannitol. In such tablets, the first layer may comprise about 30-75 %w/w microcrystalline cellulose. In some embodiments, the first layer comprises about 50-75 %w/w microcrystalline cellulose. In some embodiments, the first layer comprises about 60-70 %w/w microcrystalline cellulose.

Tablets provided herein may be uncoated or coated. Although uncoated tablets may be used, it is more usual to provide a coated tablet, in which case a conventional non-enteric coating may be used. In some embodiments, the tablets are coated with a non-functional coating, *i.e.* a coating that does not affect the release properties of the tablet. In some embodiments, the tablet is coated with a titanium dioxide free film coating.

Film coatings are known in the art and can be composed of hydrophilic polymer materials, but are not limited to, polysaccharide materials, such as hydroxypropylmethyl cellulose (HPMC), methylcellulose, hydroxyethyl cellulose (HEC), hydroxypropyl cellulose (HPC), poly(vinylalcohol-co-ethylene glycol) and other water soluble polymers. Though the water soluble material included in the film coating of the present disclosure may include a single polymer material, it may also be formed using a mixture of more than one polymer. The coating may be white or colored, *e.g.* yellow or brown. Suitable coatings include, but are not limited to, polymeric film coatings such as those comprising polyvinyl alcohol *e.g.* 'Opadry^{®} II' (which includes part-hydrolysed polyvinyl acetate (PVA), titanium dioxide, macrogol 3350 and talc, with optional coloring such as iron oxide or indigo carmine or iron oxide yellow). In one embodiment, the polymeric coating is Opadry^{®} TF, TiO₂ free formulated film. The amount of coating will generally be between about 2-6% of the core's weight, and in certain specific embodiments, about 4%. Unless specifically stated otherwise, where the dosage form is coated, it is to be understood that a reference to % weight of the tablet means that of the uncoated tablet, *i.e.* excluding the coating.

The tablet of the disclosure may comprise bictegravir or a pharmaceutically acceptable salt thereof and lenacapavir or a pharmaceutically acceptable salt thereof, wherein the amount of bictegravir or pharmaceutically acceptable salt thereof corresponds to about 50-100 mg bictegravir free acid, and the amount of lenacapavir or pharmaceutically acceptable salt thereof corresponds to about 25-100 mg lenacapavir free acid, wherein the tablet further comprises copovidone, poloxamer, mannitol, microcrystalline cellulose, croscarmellose sodium, and magnesium stearate. In some embodiments, the amount of bictegravir or pharmaceutically acceptable salt thereof corresponds to about 75 mg bictegravir free acid and the amount of lenacapavir or pharmaceutically acceptable salt thereof corresponds to about 25 mg or 50 mg lenacapavir free acid. In some embodiments, the tablet comprises bictegravir sodium. In some embodiments, the tablet comprises lenacapavir sodium. In some embodiments, the tablet comprises bictegravir sodium and lenacapavir sodium. In some embodiments, the tablet is coated with a film coating.

The tablet may consist of bictegravir or a pharmaceutically acceptable salt thereof, lenacapavir or a pharmaceutically acceptable salt thereof, copovidone, poloxamer, mannitol, microcrystalline cellulose, croscarmellose sodium, and magnesium stearate, wherein the amount of bictegravir or pharmaceutically acceptable salt thereof corresponds to about 50-100 mg bictegravir free acid, and the amount of lenacapavir or pharmaceutically acceptable salt thereof corresponds to about 25-100 mg lenacapavir free acid. In some embodiments, the amount of bictegravir or pharmaceutically acceptable salt thereof corresponds to about 75 mg bictegravir free acid and the amount of lenacapavir or pharmaceutically acceptable salt thereof corresponds to about 25 mg or 50 mg lenacapavir free acid. In some embodiments, the bictegravir or pharmaceutically acceptable salt thereof is bictegravir sodium. In some embodiments, the lenacapavir or pharmaceutically acceptable salt thereof is lenacapavir sodium. In some embodiments, the tablet is coated with a film coating.

The tablet of the disclosure may be a monolayer tablet comprising bictegravir or a pharmaceutically acceptable salt thereof and lenacapavir or a pharmaceutically acceptable salt thereof, wherein the amount of bictegravir or pharmaceutically acceptable salt thereof corresponds to about 50-100 mg bictegravir free acid, and the amount of lenacapavir or pharmaceutically acceptable salt thereof corresponds to about 25-100 mg lenacapavir free acid, wherein the tablet further comprises copovidone, poloxamer, mannitol, microcrystalline cellulose, croscarmellose sodium, and magnesium stearate. In some embodiments, in this monolayer tablet, the amount of bictegravir or pharmaceutically acceptable salt thereof corresponds to about 75 mg bictegravir free acid and the amount of lenacapavir or pharmaceutically acceptable salt thereof corresponds to about 50 mg lenacapavir free acid. The monolayer tablet may consist of bictegravir or a pharmaceutically acceptable salt thereof, lenacapavir or a pharmaceutically acceptable salt thereof, copovidone, poloxamer, mannitol, microcrystalline cellulose, croscarmellose sodium, and magnesium stearate, wherein the amount of bictegravir or pharmaceutically acceptable salt thereof corresponds to about 50-100 mg bictegravir free acid, and the amount of lenacapavir or pharmaceutically acceptable salt thereof corresponds to about 25-100 mg lenacapavir free acid. In some embodiments, in these monolayer tablets, the bictegravir or pharmaceutically acceptable salt thereof is bictegravir sodium. In some embodiments, the lenacapavir or pharmaceutically acceptable salt thereof is lenacapavir sodium. In some embodiments, this monolayer tablet is coated with a film coating.

The tablet of the disclosure may be a bilayer tablet comprising (a) a first layer comprising bictegravir or a pharmaceutically acceptable salt thereof and (b) a second layer comprising lenacapavir or a pharmaceutically acceptable salt thereof, wherein the amount of bictegravir or pharmaceutically acceptable salt thereof corresponds to about 50-100 mg bictegravir free acid, and the amount of lenacapavir or pharmaceutically acceptable salt thereof corresponds to about 25-100 mg lenacapavir free acid, wherein the tablet further comprises copovidone, poloxamer, mannitol, microcrystalline cellulose, croscarmellose sodium, and magnesium stearate. In some embodiments, in this bilayer tablet, the amount of bictegravir or pharmaceutically acceptable salt thereof corresponds to about 75 mg bictegravir free acid and the amount of lenacapavir or pharmaceutically acceptable salt thereof corresponds to about 25 mg lenacapavir free acid. In some embodiments, in this bilayer tablet, the amount of bictegravir or pharmaceutically acceptable salt thereof corresponds to about 75 mg bictegravir free acid and the amount of lenacapavir or pharmaceutically acceptable salt thereof corresponds to about 50 mg lenacapavir free acid. The first layer of the bilayer tablet may comprise bictegravir or a pharmaceutically acceptable salt thereof, microcrystalline cellulose, croscarmellose sodium, and magnesium stearate. In some embodiments, the first layer of the bilayer tablet is substantially free from mannitol. In some embodiments, the first layer contains less than 1 %w/w mannitol. In some embodiments, the first layer contains no mannitol. The second layer of the bilayer tablet may comprise lenacapavir or a pharmaceutically acceptable salt thereof, copovidone, poloxamer, mannitol, microcrystalline cellulose, croscarmellose sodium, and magnesium stearate. In some embodiments, in this bilayer tablet, the bictegravir or pharmaceutically acceptable salt thereof is bictegravir sodium. In some embodiments, the lenacapavir or pharmaceutically acceptable salt thereof is lenacapavir sodium. In some embodiments, this bilayer tablet is coated with a film coating.

In some embodiments, the disclosed bilayer tablet is a bilayer tablet wherein the amount of bictegravir or pharmaceutically acceptable salt thereof corresponds to about 75 mg bictegravir free acid and the amount of lenacapavir or pharmaceutically acceptable salt thereof corresponds to about 25 mg lenacapavir free acid, wherein the first layer of the bilayer tablet comprises bictegravir or a pharmaceutically acceptable salt thereof in an amount corresponding to about 75 mg bictegravir free acid, microcrystalline cellulose, croscarmellose sodium, and magnesium stearate, wherein the first layer of the bilayer tablet is substantially free from mannitol, and wherein the second layer of the bilayer tablet comprises lenacapavir or a pharmaceutically acceptable salt thereof in an amount corresponding to about 25 mg lenacapavir free acid, copovidone, poloxamer, mannitol, microcrystalline cellulose, croscarmellose sodium, and magnesium stearate. In some embodiments, the first layer contains less than 1 %w/w mannitol. In some embodiments, the first layer contains no mannitol.

In some embodiments, the disclosed bilayer tablet is a bilayer tablet wherein the amount of bictegravir or pharmaceutically acceptable salt thereof corresponds to about 75 mg bictegravir free acid and the amount of lenacapavir or pharmaceutically acceptable salt thereof corresponds to about 50 mg lenacapavir free acid, wherein the first layer of the bilayer tablet comprises bictegravir or a pharmaceutically acceptable salt thereof in an amount corresponding to about 75 mg bictegravir free acid, microcrystalline cellulose, croscarmellose sodium, and magnesium stearate, wherein the first layer of the bilayer tablet is substantially free from mannitol, and wherein the second layer of the bilayer tablet comprises lenacapavir or a pharmaceutically acceptable salt thereof in an amount corresponding to about 50 mg lenacapavir free acid, copovidone, poloxamer, mannitol, microcrystalline cellulose, croscarmellose sodium, and magnesium stearate. In some embodiments, the first layer contains less than 1 %w/w mannitol. In some embodiments, the first layer contains no mannitol.

The tablet of the disclosure may comprise about 1-6 %w/w copovidone, about 0-2 %w/w poloxamer, about 10-50 %w/w mannitol, about 10-60 %w/w microcrystalline cellulose, about 6-10 %w/w croscarmellose sodium and about 1-2 %w/w magnesium stearate. In some embodiments, the tablets containing these excipients comprise about 10-25 %w/w bictegravir or a pharmaceutically acceptable salt thereof and about 5-30 %w/w lenacapavir or a pharmaceutically acceptable salt thereof. The weight percentages denote a proportion of the whole tablet.

A monolayer tablet is provided comprising about 1-6 %w/w copovidone, about 0.1-2 %w/w poloxamer, about 20-50 %w/w mannitol, about 10-30 %w/w microcrystalline cellulose, about 5-15 %w/w croscarmellose sodium and about 0.1-10 %w/w magnesium stearate. In certain embodiments, the tablet comprises about 10-25 %w/w bictegravir or a pharmaceutically acceptable salt thereof and about 5-30 %w/w lenacapavir or a pharmaceutically acceptable salt thereof. The weight percentages denote a proportion of the whole tablet.

A monolayer tablet is provided comprising about 1-6 %w/w copovidone, about 0.3-1.6 %w/w poloxamer, about 25-45 %w/w mannitol, about 10-25 %w/w microcrystalline cellulose, about 6-10 %w/w croscarmellose sodium and about 1-2 %w/w magnesium stearate. In certain embodiments, the tablet comprises about 10-25 %w/w bictegravir or a pharmaceutically acceptable salt thereof and about 5-30 %w/w lenacapavir or a pharmaceutically acceptable salt thereof. The weight percentages denote a proportion of the whole tablet.

A monolayer tablet is provided comprising an amount of bictegravir or pharmaceutically acceptable salt thereof which corresponds to about 75 mg bictegravir free acid and an amount of lenacapavir or pharmaceutically acceptable salt thereof which corresponds to about 25 mg lenacapavir free acid, and wherein the monolayer table further comprises about 1-2 %w/w copovidone, about 0.2-0.4 %w/w poloxamer, about 40-45 %w/w mannitol, about 20-25 %w/w microcrystalline cellulose, about 7-9 %w/w croscarmellose sodium and about 1-2 %w/w magnesium stearate. In this embodiment, the monolayer tablet comprises about 15-20 %w/w bictegravir or a pharmaceutically acceptable salt thereof and about 5-8 %w/w lenacapavir or a pharmaceutically acceptable salt thereof. The weight percentages denote a proportion of the whole tablet.

A monolayer tablet is provided comprising an amount of bictegravir or pharmaceutically acceptable salt thereof which corresponds to about 75 mg bictegravir free acid and an amount of lenacapavir or pharmaceutically acceptable salt thereof which corresponds to about 25 mg lenacapavir free acid, and wherein the monolayer table further comprises about 1.4 %w/w copovidone, about 0.4 %w/w poloxamer, about 43 %w/w mannitol, about 22%w/w microcrystalline cellulose, about 8 %w/w croscarmellose sodium and about 1.5 %w/w magnesium stearate. In this embodiment, the monolayer tablet comprises about 18 %w/w bictegravir or a pharmaceutically acceptable salt thereof and about 6 %w/w lenacapavir or a pharmaceutically acceptable salt thereof. The weight percentages denote a proportion of the whole tablet.

A monolayer tablet is provided comprising an amount of bictegravir or pharmaceutically acceptable salt thereof which corresponds to about 75 mg bictegravir free acid and an amount of lenacapavir or pharmaceutically acceptable salt thereof which corresponds to about 50 mg lenacapavir free acid, and wherein the monolayer tablet further comprises about 2-3 %w/w copovidone, about 0.6-0.8 %w/w poloxamer, about 35-40 %w/w mannitol, about 18-22 %w/w microcrystalline cellulose, about 7-9 %w/w croscarmellose sodium and about 1-2 %w/w magnesium stearate. In this embodiment, the monolayer tablet comprises about 15-20 %w/w bictegravir or a pharmaceutically acceptable salt thereof and about 10-14 %w/w lenacapavir or a pharmaceutically acceptable salt thereof. The weight percentages denote a proportion of the whole tablet.

A monolayer tablet is provided comprising an amount of bictegravir or pharmaceutically acceptable salt thereof which corresponds to about 75 mg bictegravir free acid and an amount of lenacapavir or pharmaceutically acceptable salt thereof which corresponds to about 50 mg lenacapavir free acid, and wherein the monolayer tablet further comprises about 2.8 %w/w copovidone, about 0.8 %w/w poloxamer, about 38 %w/w mannitol, about 19 %w/w microcrystalline cellulose, about 8 %w/w croscarmellose sodium and about 1.5 %w/w magnesium stearate. In this embodiment, the monolayer tablet comprises about 18 %w/w bictegravir or a pharmaceutically acceptable salt thereof and about 12 %w/w lenacapavir or a pharmaceutically acceptable salt thereof. The weight percentages denote a proportion of the whole tablet.

A bilayer tablet is provided comprising about 0.1-5 %w/w copovidone, about 0.1-2 %w/w poloxamer, about 5-30 %w/w mannitol, about 30-60 %w/w microcrystalline cellulose, about 5-20 %w/w croscarmellose sodium and about 0.1-5 %w/w magnesium stearate. In these embodiments, the bilayer tablet comprises about 10-25 %w/w bictegravir or a pharmaceutically acceptable salt thereof and about 5-20 %w/w lenacapavir or a pharmaceutically acceptable salt thereof. The weight percentages denote a proportion of the whole tablet.

A bilayer tablet is also provided comprising about 1-3 %w/w copovidone, about 0.3-0.8 %w/w poloxamer, about 10-25 %w/w mannitol, about 40-60 %w/w microcrystalline cellulose, about 6-10 %w/w croscarmellose sodium and about 1-2 %w/w magnesium stearate. In these embodiments, the bilayer tablet comprises about 10-25 %w/w bictegravir or a pharmaceutically acceptable salt thereof and about 5-20 %w/w lenacapavir or a pharmaceutically acceptable salt thereof. In these embodiments, the weight percentages denote a proportion of the whole tablet.

A bilayer tablet is provided comprising an amount of bictegravir or pharmaceutically acceptable salt thereof which corresponds to about 75 mg bictegravir free acid and an amount of lenacapavir or pharmaceutically acceptable salt thereof which corresponds to about 25 mg lenacapavir free acid, and wherein the bilayer tablet further comprises about 1-3 %w/w copovidone, about 0.3-0.8 %w/w poloxamer, about 10-25 %w/w mannitol, about 45-55 %w/w microcrystalline cellulose, about 6-10 %w/w croscarmellose sodium and about 1-2 %w/w magnesium stearate. In these embodiments, the bilayer tablet comprises about 15-20 %w/w bictegravir or a pharmaceutically acceptable salt thereof and about 5-10 %w/w lenacapavir or a pharmaceutically acceptable salt thereof. In some embodiments, the bilayer tablet comprises (a) a first layer comprising about 15-20 %w/w bictegravir or a pharmaceutically acceptable salt thereof, about 40-50 %w/w microcrystalline cellulose, about 4-8 %w/w croscarmellose sodium, and about 0.1-2 %w/w magnesium stearate and (b) a second layer comprising about 5-10 %w/w lenacapavir or a pharmaceutically acceptable salt thereof, about 0.1-5 %w/w copovidone, about 0.1-2 %w/w poloxamer, about 5-20 %w/w mannitol, about 1-10 %w/w microcrystalline cellulose, about 1-3 %w/w croscarmellose sodium, and about 0.1-1 %w/w magnesium stearate. In these embodiments, the weight percentages denote a proportion of the whole tablet. In some embodiments, the bilayer tablet is coated with a film coating. In some embodiments, the coating is a non-functional coating.

A bilayer tablet is provided comprising an amount of bictegravir or pharmaceutically acceptable salt thereof which corresponds to about 75 mg bictegravir free acid and an amount of lenacapavir or pharmaceutically acceptable salt thereof which corresponds to about 25 mg lenacapavir free acid, and wherein the bilayer tablet further comprises about 1.4 %w/w copovidone, about 0.4 %w/w poloxamer, about 12.5 %w/w mannitol, about 51.7 %w/w microcrystalline cellulose, about 8 %w/w croscarmellose sodium and about 1.5 %w/w magnesium stearate. In these embodiments, the bilayer tablet comprises about 18 %w/w bictegravir or a pharmaceutically acceptable salt thereof and about 6 %w/w lenacapavir or a pharmaceutically acceptable salt thereof. In some embodiments, the bilayer tablet comprises (a) a first layer comprising about 18 %w/w bictegravir or a pharmaceutically acceptable salt thereof, about 45 %w/w microcrystalline cellulose, about 6 %w/w croscarmellose sodium, and about 1 %w/w magnesium stearate and (b) a second layer comprising about 6 %w/w lenacapavir or a pharmaceutically acceptable salt thereof, about 1.4 %w/w copovidone, about 0.4 %w/w poloxamer, about 12.5 %w/w mannitol, about 6 %w/w microcrystalline cellulose, about 2% croscarmellose sodium, and about 0.4 %w/w magnesium stearate. In these embodiments, the weight percentages denote a proportion of the whole tablet.

A bilayer tablet is provided comprising an amount of bictegravir or pharmaceutically acceptable salt thereof which corresponds to about 75 mg bictegravir free acid and an amount of lenacapavir or pharmaceutically acceptable salt thereof which corresponds to about 50 mg lenacapavir free acid, and wherein the bilayer tablet further comprises about 1-3 %w/w copovidone, about 0.3-0.8 %w/w poloxamer, about 10-25 %w/w mannitol, about 40-50 %w/w microcrystalline cellulose, about 6-10 %w/w croscarmellose sodium and about 1-2 %w/w magnesium stearate. In these embodiments, the bilayer tablet comprises about 10-20 %w/w bictegravir or a pharmaceutically acceptable salt thereof and about 5-10 %w/w lenacapavir or a pharmaceutically acceptable salt thereof. In some embodiments, the bilayer tablet comprises (a) a first layer comprising about 10-20 %w/w bictegravir or a pharmaceutically acceptable salt thereof, about 30-40 %w/w microcrystalline cellulose, about 2-6 %w/w croscarmellose sodium, and about 0.5-1.5 %w/w magnesium stearate and (b) a second layer comprising about 8-13 %w/w lenacapavir or a pharmaceutically acceptable salt thereof, about 2-3 %w/w copovidone, about 0.1-1 %w/w poloxamer, about 15-25 %w/w mannitol, about 7-13 %w/w microcrystalline cellulose, about 2-4 %w/w croscarmellose sodium, and about 0.1-1 %w/w magnesium stearate. In these embodiments, the weight percentages denote a proportion of the whole tablet.

A bilayer tablet is provided comprising an amount of bictegravir or pharmaceutically acceptable salt thereof which corresponds to about 75 mg bictegravir free acid and an amount of lenacapavir or pharmaceutically acceptable salt thereof which corresponds to about 50 mg lenacapavir free acid, and wherein the bilayer tablet further comprises about 2 %w/w copovidone, about 0.6 %w/w poloxamer, about 19 %w/w mannitol, about 45 %w/w microcrystalline cellulose, about 8 %w/w croscarmellose sodium and about 1.5 %w/w magnesium stearate. In these embodiments, the bilayer tablet comprises about 14 %w/w bictegravir or a pharmaceutically acceptable salt thereof and about 9 %w/w lenacapavir or a pharmaceutically acceptable salt thereof. In some embodiments, the bilayer tablet comprises (a) a first layer comprising about 14 %w/w bictegravir or a pharmaceutically acceptable salt thereof, about 35 %w/w microcrystalline cellulose, about 4 %w/w croscarmellose sodium, and about 0.8 %w/w magnesium stearate and (b) a second layer comprising about 9 %w/w lenacapavir or a pharmaceutically acceptable salt thereof, about 2 %w/w copovidone, about 0.6 %w/w poloxamer, about 19 %w/w mannitol, about 10 %w/w microcrystalline cellulose, about 4 %w/w croscarmellose sodium, and about 0.7 %w/w magnesium stearate. In these embodiments, the weight percentages denote a proportion of the whole tablet.

In some embodiments, the bilayer tablet is coated with a film coating. In some embodiments, the coating is a non-functional coating.

In some embodiments, the tablet comprises about 5-50 mg copovidone, about 0.5-10 mg poloxamer, about 50-200 mg mannitol, about 50-250 mg microcrystalline cellulose, about 25-50 mg croscarmellose sodium and about 1-10 mg magnesium stearate.

In some embodiments, the tablet comprises an amount of bictegravir or pharmaceutically acceptable salt thereof which corresponds to about 75 mg bictegravir free acid, an amount of lenacapavir or pharmaceutically acceptable salt thereof which corresponds to about 25-100 mg lenacapavir free acid, 5-50 mg copovidone, about 0.5-10 mg poloxamer, about 50-200 mg mannitol, about 50-250 mg microcrystalline cellulose, about 25-50 mg croscarmellose sodium and about 1-10 mg magnesium stearate.

In some embodiments, this tablet comprises about 5-30 mg copovidone, about 0.5-7 mg poloxamer, about 50-200 mg mannitol, about 55-250 mg microcrystalline cellulose, about 25-50 mg croscarmellose sodium and about 1-10 mg magnesium stearate.

In some embodiments, the tablet comprises an amount of bictegravir or pharmaceutically acceptable salt thereof which corresponds to about 75 mg bictegravir free acid, an amount of lenacapavir or pharmaceutically acceptable salt thereof which corresponds to about 25-100 mg lenacapavir free acid, 5-30 mg copovidone, about 0.5-7 mg poloxamer, about 50-200 mg mannitol, about 55-250 mg microcrystalline cellulose, about 25-50 mg croscarmellose sodium and about 1-10 mg magnesium stearate.

In some embodiments, the tablet comprises an amount of bictegravir or pharmaceutically acceptable salt thereof which corresponds to about 75 mg bictegravir free acid, an amount of lenacapavir or pharmaceutically acceptable salt thereof which corresponds to about 25 mg lenacapavir free acid, 5-15 mg copovidone, about 0.5-5 mg poloxamer, about 50-200 mg mannitol, about 70-250 mg microcrystalline cellulose, about 25-50 mg croscarmellose sodium and about 1-10 mg magnesium stearate.

In some embodiments, the tablet comprises an amount of bictegravir or pharmaceutically acceptable salt thereof which corresponds to about 75 mg bictegravir free acid, an amount of lenacapavir or pharmaceutically acceptable salt thereof which corresponds to about 50 mg lenacapavir free acid, 5-15 mg copovidone, about 0.5-5 mg poloxamer, about 50-200 mg mannitol, about 70-250 mg microcrystalline cellulose, about 25-50 mg croscarmellose sodium and about 1-10 mg magnesium stearate.

In some embodiments, the tablet is a monolayer tablet and comprises 5-30 mg copovidone, about 0.5-10 mg poloxamer, about 100-200 mg mannitol, about 50-100 mg microcrystalline cellulose, about 25-50 mg croscarmellose sodium, and about 5-10 mg magnesium stearate.

In some embodiments, the tablet is a monolayer tablet and comprises an amount of bictegravir or pharmaceutically acceptable salt thereof which corresponds to about 75 mg bictegravir free acid, an amount of lenacapavir or pharmaceutically acceptable salt thereof which corresponds to about 25-100 mg lenacapavir free acid, 5-30 mg copovidone, about 0.5-10 mg poloxamer, about 100-200 mg mannitol, about 50-100 mg microcrystalline cellulose, about 25-50 mg croscarmellose sodium, and about 5-10 mg magnesium stearate.

In some embodiments, the tablet is a monolayer tablet and comprises an amount of bictegravir or pharmaceutically acceptable salt thereof which corresponds to about 75 mg bictegravir free acid, an amount of lenacapavir or pharmaceutically acceptable salt thereof which corresponds to about 50 mg lenacapavir free acid, about 5-15 mg copovidone, about 0.5-5 mg poloxamer, about 150-200 mg mannitol, about 70-100 mg microcrystalline cellulose, about 25-50 mg croscarmellose sodium, and about 5-10 mg magnesium stearate.

In some embodiments, the tablet is a monolayer tablet and comprises an amount of bictegravir or pharmaceutically acceptable salt thereof which corresponds to about 75 mg bictegravir free acid, an amount of lenacapavir or pharmaceutically acceptable salt thereof which corresponds to about 25 mg lenacapavir free acid, about 5-15 mg copovidone, about 0.5-5 mg poloxamer, about 150-200 mg mannitol, about 70-100 mg microcrystalline cellulose, about 25-50 mg croscarmellose sodium, and about 5-10 mg magnesium stearate.

In some embodiments, the tablet is a monolayer tablet, wherein the amount of bictegravir or pharmaceutically acceptable salt thereof corresponds to about 75 mg bictegravir free acid, and the amount of lenacapavir or pharmaceutically acceptable salt thereof corresponds to about 50 mg lenacapavir free acid, and wherein the tablet comprises about 10-15 mg copovidone, about 2-5 mg poloxamer, about 150-175 mg mannitol, about 75-90 mg microcrystalline cellulose, about 30-40 mg croscarmellose sodium, and about 5-8 mg magnesium stearate. In some embodiments, the tablet weighs about 430-440 mg. In some embodiments, the tablet is coated with a film coating. In some embodiments, the coating is a non-functional coating.

In some embodiments, the tablet is a monolayer tablet and comprises an amount of bictegravir or pharmaceutically acceptable salt thereof which corresponds to about 75 mg bictegravir free acid, an amount of lenacapavir or pharmaceutically acceptable salt thereof which corresponds to about 50 mg lenacapavir free acid, about 12 mg copovidone, about 3 mg poloxamer, about 165 mg mannitol, about 83 mg microcrystalline cellulose, about 35 mg croscarmellose sodium, and about 7 mg magnesium stearate. In some embodiments, the tablet is coated with a film coating. In some embodiments, the coating is a non-functional coating.

In some embodiments, the tablet is a monolayer tablet and comprises about 79 mg d, about 51 mg lenacapavir sodium, about 12 mg copovidone, about 3 mg poloxamer, about 165 mg mannitol, about 83 mg microcrystalline cellulose, about 35 mg croscarmellose sodium, and about 7 mg magnesium stearate. In some embodiments, the tablet is coated with a film coating. In some embodiments, the coating is a non-functional coating.

In some embodiments, the tablet is a monolayer tablet and comprises an amount of bictegravir or pharmaceutically acceptable salt thereof which corresponds to about 75 mg bictegravir free acid, an amount of lenacapavir or pharmaceutically acceptable salt thereof which corresponds to about 50 mg lenacapavir free acid, about 12.2 mg copovidone, about 3.3 mg poloxamer, about 165.0 mg mannitol, about 82.5 mg microcrystalline cellulose, about 34.7 mg croscarmellose sodium, and about 6.5 mg magnesium stearate. In some embodiments, the tablet is coated with a film coating. In some embodiments, the coating is a non-functional coating.

In some embodiments, the tablet is a monolayer tablet and comprises about 78.6 mg bictegravir sodium, about 51.1 mg lenacapavir sodium, about 12.2 mg copovidone, about 3.3 mg poloxamer, about 165.0 mg mannitol, about 82.5 mg microcrystalline cellulose, about 34.7 mg croscarmellose sodium, and about 6.5 mg magnesium stearate. In some embodiments, the tablet is coated with a film coating. In some embodiments, the coating is a non-functional coating.

In some embodiments, the tablet is a monolayer tablet, wherein the amount of bictegravir or pharmaceutically acceptable salt thereof corresponds to about 75 mg bictegravir free acid, and the amount of lenacapavir or pharmaceutically acceptable salt thereof corresponds to about 25 mg lenacapavir free acid, and wherein the tablet comprises about 5-8 mg copovidone, about 1-3 mg poloxamer, about 175-200 mg mannitol, about 90-100 mg microcrystalline cellulose, about 30-40 mg croscarmellose sodium, and about 5-8 mg magnesium stearate. In some embodiments, the tablet weighs about 430-440 mg. In some embodiments, the tablet is coated with a film coating. In some embodiments, the coating is a non-functional coating.

In some embodiments, the tablet is a monolayer tablet and comprises an amount of bictegravir or pharmaceutically acceptable salt thereof which corresponds to about 75 mg bictegravir free acid, an amount of lenacapavir or pharmaceutically acceptable salt thereof which corresponds to about 25 mg lenacapavir free acid, about 6 mg copovidone, about 2 mg poloxamer, about 187 mg mannitol, about 94 mg microcrystalline cellulose, about 35 mg croscarmellose sodium, and about 7 mg magnesium stearate. In some embodiments, the tablet weighs about 434 mg. In some embodiments, the tablet is coated with a film coating. In some embodiments, the coating is a non-functional coating.

In some embodiments, the tablet is a monolayer tablet and comprises about 79 mg bictegravir sodium, about 26 mg lenacapavir sodium, about 6 mg copovidone, about 2 mg poloxamer, about 187 mg mannitol, about 94 mg microcrystalline cellulose, about 35 mg croscarmellose sodium, and about 7 mg magnesium stearate. In some embodiments, the tablet is coated with a film coating. In some embodiments, the coating is a non-functional coating.

In some embodiments, the tablet is a monolayer tablet and comprises an amount of bictegravir or pharmaceutically acceptable salt thereof which corresponds to about 75 mg bictegravir free acid, an amount of lenacapavir or pharmaceutically acceptable salt thereof which corresponds to about 25 mg lenacapavir free acid, about 6.1 mg copovidone, about 1.6 mg poloxamer, about 187.3 mg mannitol, about 93.6 mg microcrystalline cellulose, about 34.7 mg croscarmellose sodium, and about 6.5 mg magnesium stearate. In some embodiments, the tablet is coated with a film coating. In some embodiments, the coating is a non-functional coating. In some embodiments, the tablet weighs about 434 mg excluding the film coating.

In some embodiments, the tablet is a monolayer tablet and comprises about 78.6 mg bictegravir sodium, about 25.6 mg lenacapavir sodium, about 6.1 mg copovidone, about 1.6 mg poloxamer, about 187.3 mg mannitol, about 93.6 mg microcrystalline cellulose, about 34.7 mg croscarmellose sodium, and about 6.5 mg magnesium stearate. In some embodiments, the tablet is coated with a film coating. In some embodiments, the coating is a non-functional coating

In some embodiments, the tablet is a monolayer tablet, wherein the amount of bictegravir or pharmaceutically acceptable salt thereof corresponds to about 75 mg bictegravir free acid, and the amount of lenacapavir or pharmaceutically acceptable salt thereof corresponds to about 100 mg lenacapavir free acid, and wherein the tablet comprises about 20-25 mg copovidone, about 5-8 mg poloxamer, about 115-125 mg mannitol, about 55-65 mg microcrystalline cellulose, about 30-40 mg croscarmellose sodium, and about 5-8 mg magnesium stearate. In some embodiments, the tablet is coated with a film coating. In some embodiments, the coating is a non-functional coating. In some embodiments, the tablet weighs about 430-440 mg excluding the film coating.

In some embodiments, the tablet is a monolayer tablet and comprises an amount of bictegravir or pharmaceutically acceptable salt thereof which corresponds to about 75 mg bictegravir free acid, an amount of lenacapavir or pharmaceutically acceptable salt thereof which corresponds to about 100 mg lenacapavir free acid, about 25 mg copovidone, about 7 mg poloxamer, about 121 mg mannitol, about 60 mg microcrystalline cellulose, about 35 mg croscarmellose sodium, and about 7 mg magnesium stearate. In some embodiments, the tablet is coated with a film coating. In some embodiments, the coating is a non-functional coating. In some embodiments, the tablet weighs about 434 mg excluding the film coating.

In some embodiments, the tablet is a monolayer tablet and comprises about 79 mg bictegravir sodium, about 102 mg lenacapavir sodium, about 25 mg copovidone, about 7 mg poloxamer, about 121 mg mannitol, about 60 mg microcrystalline cellulose, about 35 mg croscarmellose sodium, and about 7 mg magnesium stearate. In some embodiments, the tablet is coated with a film coating. In some embodiments, the coating is a non-functional coating.

In some embodiments, the tablet is a monolayer tablet and comprises an amount of bictegravir or pharmaceutically acceptable salt thereof which corresponds to about 75 mg bictegravir free acid, an amount of lenacapavir or pharmaceutically acceptable salt thereof which corresponds to about 100 mg lenacapavir free acid, about 24.5 mg copovidone, about 6.6 mg poloxamer, about 120.6 mg mannitol, about 60.3 mg microcrystalline cellulose, about 34.7 mg croscarmellose sodium, and about 6.5 mg magnesium stearate. In some embodiments, the tablet is coated with a film coating. In some embodiments, the coating is a non-functional coating. In some embodiments, the tablet weighs about 434 mg excluding the film coating.

In some embodiments, the tablet is a monolayer tablet and comprises about 78.6 mg bictegravir sodium, about 102.2 mg lenacapavir sodium, about 24.5 mg copovidone, about 6.6 mg poloxamer, about 120.6 mg mannitol, about 60.3 mg microcrystalline cellulose, about 34.7 mg croscarmellose sodium, and about 6.5 mg magnesium stearate. In some embodiments, the tablet is coated with a film coating. In some embodiments, the coating is a non-functional coating.

In some embodiments, the tablet is a bilayer tablet, and the tablet comprises about 5-15 mg copovidone, about 0.5-5 mg poloxamer, 50-125 mg mannitol, about 200-250 mg microcrystalline cellulose, about 25-50 mg croscarmellose sodium and about 5-10 mg magnesium stearate.

In some embodiments, the tablet is a bilayer tablet and comprises an amount of bictegravir or pharmaceutically acceptable salt thereof which corresponds to about 75 mg bictegravir free acid, an amount of lenacapavir or pharmaceutically acceptable salt thereof which corresponds to about 25-50 mg lenacapavir free acid, about 5-15 mg copovidone, about 0.5-5 mg poloxamer, 50-125 mg mannitol, about 200-250 mg microcrystalline cellulose, about 25-50 mg croscarmellose sodium and about 5-10 mg magnesium stearate.

In some embodiments, the tablet is a bilayer tablet and comprises an amount of bictegravir or pharmaceutically acceptable salt thereof which corresponds to about 75 mg bictegravir free acid, an amount of lenacapavir or pharmaceutically acceptable salt thereof which corresponds to about 50 mg lenacapavir free acid, about 5-15 mg copovidone, about 0.5-5 mg poloxamer, 50-125 mg mannitol, about 200-250 mg microcrystalline cellulose, about 25-50 mg croscarmellose sodium and about 5-10 mg magnesium stearate.

In some embodiments, the tablet is a bilayer tablet and comprises an amount of bictegravir or pharmaceutically acceptable salt thereof which corresponds to about 75 mg bictegravir free acid, an amount of lenacapavir or pharmaceutically acceptable salt thereof which corresponds to about 25 mg lenacapavir free acid, about 5-15 mg copovidone, about 0.5-5 mg poloxamer, about 50-125 mg mannitol, about 200-250 mg microcrystalline cellulose, about 25-50 mg croscarmellose sodium and about 5-10 mg magnesium stearate.

In some embodiments, the first layer of the bilayer tablet comprises an amount of bictegravir or pharmaceutically acceptable salt thereof corresponding to about 75 mg bictegravir free acid, and wherein the first layer further comprises about 185-200 mg microcrystalline cellulose, about 20-30 mg croscarmellose sodium and about 3-5 mg magnesium stearate. In some embodiments, the first layer of the bilayer tablet does not contain any filler besides microcrystalline cellulose. In some embodiments, the first layer of the bilayer tablet does not contain any mannitol.

In some embodiments, the first layer of the bilayer tablet comprises amount of bictegravir or pharmaceutically acceptable salt thereof corresponding to about 75 mg bictegravir free acid, and wherein the first layer further comprises about 193 mg microcrystalline cellulose, about 24 mg croscarmellose sodium and about 5 mg magnesium stearate. In some embodiments, this bilayer tablet does not contain any filler besides microcrystalline cellulose. In some embodiments, this bilayer tablet does not contain any mannitol.

In some embodiments, the first layer of the bilayer tablet comprises about 79 mg bictegravir sodium, about 193 mg microcrystalline cellulose, about 24 mg croscarmellose sodium and about 5 mg magnesium stearate. In some embodiments, this bilayer tablet does not contain any filler besides microcrystalline cellulose. In some embodiments, this bilayer tablet does not contain any mannitol.

In some embodiments, the first layer of the bilayer tablet comprises amount of bictegravir or pharmaceutically acceptable salt thereof corresponding to about 75 mg bictegravir free acid, and wherein the first layer further comprises about 193.0 mg microcrystalline cellulose, about 24.0 mg croscarmellose sodium and about 4.5 mg magnesium stearate. In some embodiments, this bilayer tablet does not contain any filler besides microcrystalline cellulose. In some embodiments, this bilayer tablet does not contain any mannitol.

In some embodiments, the first layer of the bilayer tablet comprises about 78.5 mg bictegravir sodium, 193.0 mg microcrystalline cellulose, about 24.0 mg croscarmellose sodium and about 4.5 mg magnesium stearate. In some embodiments, this bilayer tablet does not contain any filler besides microcrystalline cellulose. In some embodiments, this bilayer tablet does not contain any mannitol.

In some embodiments, the second layer of the bilayer tablet comprises an amount of lenacapavir or pharmaceutically acceptable salt thereof corresponding to about 25 mg lenacapavir free acid, and wherein the second layer further comprises about 5-8 mg copovidone, about 1-3 mg poloxamer, about 45-60 mg mannitol, about 20-30 mg microcrystalline cellulose, about 5-15 mg croscarmellose sodium and about 1-3 mg magnesium stearate.

In some embodiments, the second layer of the bilayer tablet comprises an amount of lenacapavir or pharmaceutically acceptable salt thereof corresponding to about 25 mg lenacapavir free acid, and wherein the second layer further comprises about 6 mg copovidone, about 2 mg poloxamer, about 53 mg mannitol, about 27 mg microcrystalline cellulose, about 10 mg croscarmellose sodium and about 2 mg magnesium stearate.

In some embodiments, the second layer of the bilayer tablet comprises about 26 mg lenacapavir sodium, about 6 mg copovidone, about 2 mg poloxamer, about 53 mg mannitol, about 27 mg microcrystalline cellulose, about 10 mg croscarmellose sodium and about 2 mg magnesium stearate.

In some embodiments, the second layer of the bilayer tablet comprises an amount of lenacapavir or pharmaceutically acceptable salt thereof corresponding to about 25 mg lenacapavir free acid, and wherein the second layer further comprises about 6.1 mg copovidone, about 1.7 mg poloxamer, about 53.2 mg mannitol, about 26.6 mg microcrystalline cellulose, about 10.0 mg croscarmellose sodium and about 1.9 mg magnesium stearate.

In some embodiments, the second layer of the bilayer tablet comprises about 25.6 mg lenacapavir sodium, about 6.1 mg copovidone, about 1.7 mg poloxamer, about 53.2 mg mannitol, about 26.6 mg microcrystalline cellulose, about 10.0 mg croscarmellose sodium and about 1.9 mg magnesium stearate.

In some embodiments, the second layer of the bilayer tablet comprises an amount of lenacapavir or pharmaceutically acceptable salt thereof corresponding to about 50 mg lenacapavir free acid, and wherein the second layer further comprises about 10-15 mg copovidone, about 2-5 mg poloxamer, about 100-110 mg mannitol, about 45-60 mg microcrystalline cellulose, about 15-25 mg croscarmellose sodium and about 2-5 mg magnesium stearate.

In some embodiments, the second layer of the bilayer tablet comprises an amount of lenacapavir or pharmaceutically acceptable salt thereof corresponds to about 50 mg lenacapavir free acid, and wherein the second layer further comprises about 12 mg copovidone, about 3 mg poloxamer, about 106 mg mannitol, about 53 mg microcrystalline cellulose, about 20 mg croscarmellose sodium and about 4 mg magnesium stearate.

In some embodiments, the second layer of the bilayer tablet comprises about 51 mg lenacapavir sodium, about 12 mg copovidone, about 3 mg poloxamer, about 106 mg mannitol, about 53 mg microcrystalline cellulose, about 20 mg croscarmellose sodium and about 4 mg magnesium stearate.

In some embodiments, the second layer of the bilayer tablet comprises an amount of lenacapavir or pharmaceutically acceptable salt thereof corresponds to about 50 mg lenacapavir free acid, and wherein the second layer further comprises about 12.2 mg copovidone, about 3.3 mg poloxamer, about 106.4 mg mannitol, about 53.2 mg microcrystalline cellulose, about 20.0 mg croscarmellose sodium and about 3.8 mg magnesium stearate.

In some embodiments, the second layer of the bilayer tablet comprises about 51.1 mg lenacapavir sodium, about 12.2 mg copovidone, about 3.3 mg poloxamer, about 106.4 mg mannitol, about 53.2 mg microcrystalline cellulose, about 20.0 mg croscarmellose sodium and about 3.8 mg magnesium stearate.

In some embodiments, the first layer of the bilayer tablet comprises an amount of lenacapavir or pharmaceutically acceptable salt thereof corresponding to about 25-50 mg lenacapavir free acid. In some embodiments, the first layer of the bilayer tablet comprises about 25.6-51.1 mg lenacapavir sodium. In some embodiments, the first layer further comprises about 5-15 mg copovidone, about 1-5 mg poloxamer, about 45-60 mg mannitol, about 20-55 mg microcrystalline cellulose, about 5-25 mg croscarmellose sodium and about 1-5 mg magnesium stearate. In some embodiments, the first layer of the bilayer tablet comprises an amount of lenacapavir or pharmaceutically acceptable salt thereof corresponding to about 50 mg lenacapavir free acid. In some embodiments, the first layer of the bilayer tablet comprises about 51.2 mg lenacapavir sodium. In some embodiments, the first layer of the bilayer tablet comprises an amount of lenacapavir or pharmaceutically acceptable salt thereof corresponding to about 25 mg lenacapavir free acid. In some embodiments, the first layer of the bilayer tablet comprises about 25.6 mg lenacapavir sodium.

In some embodiments, the second layer of the bilayer tablet comprises an amount of bictegravir or pharmaceutically acceptable salt thereof corresponding to about 75 mg bictegravir free acid. In some embodiments, the second layer of the bilayer tablet comprises about 78.5 mg bictegravir sodium. In some embodiments, the second layer further comprises about 185-200 mg microcrystalline cellulose, about 20-30 mg croscarmellose sodium and about 3-5 mg magnesium stearate. In some embodiments, the second layer of the bilayer tablet does not contain any filler besides microcrystalline cellulose. In some embodiments, the second layer of the bilayer tablet does not contain any mannitol.

In some embodiments, the first layer of the bilayer tablet comprises an amount of lenacapavir or pharmaceutically acceptable salt thereof corresponding to about 25 mg lenacapavir free acid, and the second layer of the bilayer tablet comprises an amount of bictegravir or pharmaceutically acceptable salt thereof corresponding to about 75 mg bictegravir free acid.

In some embodiments, the first layer of the bilayer tablet comprises an amount of lenacapavir or pharmaceutically acceptable salt thereof corresponding to about 50 mg lenacapavir free acid, and the second layer of the bilayer tablet comprises an amount of bictegravir or pharmaceutically acceptable salt thereof corresponding to about 75 mg bictegravir free acid.

In some embodiments, the multilayer tablet further comprises a film coating.

In some embodiments, the tablet is a bilayer tablet comprising (a) a first layer comprising bictegravir or a pharmaceutically acceptable salt thereof, and (b) a second layer comprising lenacapavir or a pharmaceutically acceptable salt thereof,
wherein the amount of bictegravir or pharmaceutically acceptable salt thereof corresponds to about 75 mg bictegravir free acid, and wherein the first layer further comprises about 175-210 mg microcrystalline cellulose, about 15-35 mg croscarmellose sodium and about 1-10 mg magnesium stearate, and
wherein the amount of lenacapavir or pharmaceutically acceptable salt thereof corresponds to about 25 mg lenacapavir free acid, and wherein the second layer further comprises about 1-10 mg copovidone, about 1-6 mg poloxamer, about 30-75 mg mannitol, about 15-40 mg microcrystalline cellulose, about 1-20 mg croscarmellose sodium and about 1-10 mg magnesium stearate.

In some embodiments, the tablet further comprises a film coating. In some embodiments, the first layer is substantially free from mannitol. In some embodiments, the tablet weighs about 420-430 mg excluding the film coating.

In some embodiments, the tablet is a bilayer tablet comprising (a) a first layer comprising bictegravir or a pharmaceutically acceptable salt thereof, and (b) a second layer comprising lenacapavir or a pharmaceutically acceptable salt thereof,
wherein the amount of bictegravir or pharmaceutically acceptable salt thereof corresponds to about 75 mg bictegravir free acid, and wherein the first layer further comprises about 185-200 mg microcrystalline cellulose, about 20-30 mg croscarmellose sodium and about 3-5 mg magnesium stearate, and
wherein the amount of lenacapavir or pharmaceutically acceptable salt thereof corresponds to about 25 mg lenacapavir free acid, and wherein the second layer further comprises about 5-8 mg copovidone, about 1-3 mg poloxamer, about 45-60 mg mannitol, about 20-30 mg microcrystalline cellulose, about 5-15 mg croscarmellose sodium and about 1-3 mg magnesium stearate.

In some embodiments, the tablet further comprises a film coating. In some embodiments, the first layer is substantially free from mannitol. In some embodiments, the tablet weighs about 420-430 mg excluding the film coating.

In some embodiments, the tablet is a bilayer tablet comprising (a) a first layer comprising bictegravir or a pharmaceutically acceptable salt thereof, and (b) a second layer comprising lenacapavir or a pharmaceutically acceptable salt thereof,
wherein the amount of bictegravir or pharmaceutically acceptable salt thereof corresponds to about 75 mg bictegravir free acid, and wherein the first layer further comprises about 193 mg microcrystalline cellulose, about 24 mg croscarmellose sodium and about 5 mg magnesium stearate, and
wherein the amount of lenacapavir or pharmaceutically acceptable salt thereof corresponds to about 25 mg lenacapavir free acid, and wherein the second layer further comprises about 6 mg copovidone, about 2 mg poloxamer, about 53 mg mannitol, about 27 mg microcrystalline cellulose, about 10 mg croscarmellose sodium and about 2 mg magnesium stearate.

In some embodiments, the tablet further comprises a film coating. In some embodiments, the first layer is substantially free from mannitol.

In some embodiments, the tablet is a bilayer tablet comprising (a) a first layer comprising about 79 mg bictegravir sodium, and (b) a second layer comprising about 26 mg lenacapavir sodium,
wherein the first layer further comprises about 193 mg microcrystalline cellulose, about 24 mg croscarmellose sodium and about 5 mg magnesium stearate, and
wherein the second layer further comprises about 6 mg copovidone, about 2 mg poloxamer, about 53 mg mannitol, about 27 mg microcrystalline cellulose, about 10 mg croscarmellose sodium and about 2 mg magnesium stearate.

In some embodiments, the tablet further comprises a film coating. In some embodiments, the first layer is substantially free from mannitol.

In some embodiments, the tablet is a bilayer tablet comprising (a) a first layer comprising bictegravir or a pharmaceutically acceptable salt thereof, and (b) a second layer comprising lenacapavir or a pharmaceutically acceptable salt thereof,
wherein the amount of bictegravir or pharmaceutically acceptable salt thereof corresponds to about 75 mg bictegravir free acid, and wherein the first layer further comprises about 193.0 mg microcrystalline cellulose, about 24.0 mg croscarmellose sodium and about 4.5 mg magnesium stearate, and
wherein the amount of lenacapavir or pharmaceutically acceptable salt thereof corresponds to about 25 mg lenacapavir free acid, and wherein the second layer further comprises about 6.1 mg copovidone, about 1.7 mg poloxamer, about 53.2 mg mannitol, about 26.6 mg microcrystalline cellulose, about 10.0 mg croscarmellose sodium and about 1.9 mg magnesium stearate.

In some embodiments, the tablet further comprises a film coating.

In some embodiments, the tablet is a bilayer tablet comprising (a) a first layer comprising about 78.5 mg bictegravir sodium, and (b) a second layer comprising about 25.6 mg lenacapavir sodium,
wherein the first layer further comprises about 193.0 mg microcrystalline cellulose, about 24.0 mg croscarmellose sodium and about 4.5 mg magnesium stearate,
and wherein the second layer further comprises about 6.1 mg copovidone, about 1.7 mg poloxamer, about 53.2 mg mannitol, about 26.6 mg microcrystalline cellulose, about 10.0 mg croscarmellose sodium and about 1.9 mg magnesium stearate,

In some embodiments, the tablet further comprises a film coating.

In some embodiments, the tablet is a bilayer tablet comprising (a) a first layer comprising bictegravir or a pharmaceutically acceptable salt thereof, and (b) a second layer comprising lenacapavir or a pharmaceutically acceptable salt thereof,
wherein the amount of bictegravir or pharmaceutically acceptable salt thereof corresponds to about 75 mg bictegravir free acid, and wherein the first layer further comprises about 175-210 mg microcrystalline cellulose, about 15-35 mg croscarmellose sodium and about 1-10 mg magnesium stearate, and
wherein the amount of lenacapavir or pharmaceutically acceptable salt thereof corresponds to about 50 mg lenacapavir free acid, and wherein the second layer further comprises about 5-25 mg copovidone, about 1-10 mg poloxamer, about 75-125 mg mannitol, about 40-65 mg microcrystalline cellulose, about 10-30 mg croscarmellose sodium and about 1-10 mg magnesium stearate.

In some embodiments, the tablet further comprises a film coating. In some embodiments, the first layer is substantially free from mannitol. In some embodiments, the tablet weighs about 540-560 mg excluding the film coating.

In some embodiments, the tablet is a bilayer tablet comprising (a) a first layer comprising bictegravir or a pharmaceutically acceptable salt thereof, and (b) a second layer comprising lenacapavir or a pharmaceutically acceptable salt thereof,
wherein the amount of bictegravir or pharmaceutically acceptable salt thereof corresponds to about 75 mg bictegravir free acid, and wherein the first layer further comprises about 185-200 mg microcrystalline cellulose, about 20-30 mg croscarmellose sodium and about 3-5 mg magnesium stearate, and
wherein the amount of lenacapavir or pharmaceutically acceptable salt thereof corresponds to about 50 mg lenacapavir free acid, and wherein the second layer further comprises about 10-15 mg copovidone, about 2-5 mg poloxamer, about 100-110 mg mannitol, about 45-60 mg microcrystalline cellulose, about 15-25 mg croscarmellose sodium and about 2-5 mg magnesium stearate.

In some embodiments, the tablet further comprises a film coating. In some embodiments, the first layer is substantially free from mannitol. In some embodiments, the tablet weighs about 545-555 mg excluding the film coating.

In some embodiments, the tablet is a bilayer tablet comprising (a) a first layer comprising bictegravir or a pharmaceutically acceptable salt thereof, and (b) a second layer comprising lenacapavir or a pharmaceutically acceptable salt thereof,
wherein the amount of bictegravir or pharmaceutically acceptable salt thereof corresponds to about 75 mg bictegravir free acid, and wherein the first layer further comprises about 193 mg microcrystalline cellulose, about 24 mg croscarmellose sodium and about 5 mg magnesium stearate, and
wherein the amount of lenacapavir or pharmaceutically acceptable salt thereof corresponds to about 50 mg lenacapavir free acid, and wherein the second layer further comprises about 12 mg copovidone, about 3 mg poloxamer, about 106 mg mannitol, about 53 mg microcrystalline cellulose, about 20 mg croscarmellose sodium and about 4 mg magnesium stearate.

In some embodiments, the tablet further comprises a film coating. In some embodiments, the first layer is substantially free from mannitol.

In some embodiments, the tablet is a bilayer tablet comprising (a) a first layer comprising about 79 mg bictegravir sodium, and (b) a second layer comprising about 51 mg lenacapavir sodium,
wherein the first layer further comprises about 193 mg microcrystalline cellulose, about 24 mg croscarmellose sodium and about 5 mg magnesium stearate, and
wherein the second layer further comprises about 12 mg copovidone, about 3 mg poloxamer, about 106 mg mannitol, about 53 mg microcrystalline cellulose, about 20 mg croscarmellose sodium and about 4 mg magnesium stearate.

In some embodiments, the tablet further comprises a film coating. In some embodiments, the first layer is substantially free from mannitol.

In some embodiments, the tablet is a bilayer tablet comprising (a) a first layer comprising bictegravir or a pharmaceutically acceptable salt thereof, and (b) a second layer comprising lenacapavir or a pharmaceutically acceptable salt thereof,
wherein the amount of bictegravir or pharmaceutically acceptable salt thereof corresponds to about 75 mg bictegravir free acid, and wherein the first layer further comprises about 193.0 mg microcrystalline cellulose, about 24.0 mg croscarmellose sodium and about 4.5 mg magnesium stearate, and
wherein the amount of lenacapavir or pharmaceutically acceptable salt thereof corresponds to about 50 mg lenacapavir free acid, and wherein the second layer further comprises about 12.2 mg copovidone, about 3.3 mg poloxamer, about 106.4 mg mannitol, about 53.2 mg microcrystalline cellulose, about 20.0 mg croscarmellose sodium and about 3.8 mg magnesium stearate.

In some embodiments, the tablet further comprises a film coating. In some embodiments, the first layer is substantially free from mannitol.

In some embodiments, the tablet is a bilayer tablet comprising (a) a first layer comprising 78.5 mg bictegravir sodium, and (b) a second layer comprising 51.1 mg lenacapavir sodium,
wherein the first layer further comprises about 193.0 mg microcrystalline cellulose, about 24.0 mg croscarmellose sodium and about 4.5 mg magnesium stearate, and
wherein the second layer further comprises about 12.2 mg copovidone, about 3.3 mg poloxamer, about 106.4 mg mannitol, about 53.2 mg microcrystalline cellulose, about 20.0 mg croscarmellose sodium and about 3.8 mg magnesium stearate.

In some embodiments, the tablet further comprises a film coating. In some embodiments, the first layer is substantially free from mannitol.

In some embodiments the film coating comprises polyvinyl alcohol, titanium dioxide, polyethylene glycol, talc, and iron oxide yellow. In some embodiments the film coating comprises Opadry II 85F520107 Yellow. In some embodiments, the film coating comprises polyvinyl alcohol, titanium dioxide, polyethylene glycol, talc, and iron oxide red. In some embodiments the film coating comprises Opadry II 85F86937 Brown.

In some embodiments, the first layer of the bilayer tablet is in contact with the second layer.

### Pharmacokinetics

The inventors have found that it is possible to formulate bictegravir (in particular bictegravir sodium) and lenacapavir (in particular lenacapavir sodium) in a tablet capable of releasing each active ingredient (measured by *e.g.* AUC_{inf}, Cₘₐₓ) in an amount which is at least comparable to that of standard comparators (*i.e.* single agent tablets comprising the equivalent amount of bictegravir and lenacapavir). In particular, in some embodiments the tablets of the disclosure provide plasma concentrations (AUC_{inf}, Cₘₐₓ) of one or more of the two active pharmaceutical ingredients that is comparable to the plasma concentrations produced by the administration of a combination of single agent tablets.

Achieving comparable release of bictegravir and lenacapavir to the single agent formulations from a fixed dose combination formulation was initially a challenge because the dissolution and subsequent bioavailability of the active agents was found to vary depending on the specific dosages included in the formulations. Without being bound by any particular theory, it is believed that the two active agents interact with one another in a manner which impacts their respective solubility and resulting bioavailability. For example, the dissolution of lenacapavir from monolayer tablets comprising 75 mg bictegravir and 25 mg lenacapavir was reduced relative to the combination of the equivalent single agent tablets. Additionally, the dissolution of bictegravir from monolayer tablets comprising 75 mg bictegravir and 100 mg lenacapavir was reduced relative to the combination of the equivalent single agent tablets. There is thus a balance to be struck between the dosage of bictegravir and the dosage of lenacapavir in order to achieve sufficient dissolution of both active agents and provide bioequivalence to the combination of the single agent tablets.

As used herein, F (bioavailability) is the fraction of an administered drug that reaches the systemic circulation.

In certain specific embodiments, the tablet of the disclosure provides a bioavailability of at least about 20% of bictegravir in fasted, pentagastrin pre-treated dogs, in some embodiments, at least about 30%.

In certain specific embodiments, the tablet of the disclosure provides a bioavailability of at least about 2% of lenacapavir in fasted, pentagastrin pre-treated dogs, in some embodiments, at least about 3%.

In some embodiments" the tablet which provides the bioavailability of at least about 20% of bictegravir in fasted, pentagastrin pre-treated dogs is a monolayer tablet comprising bictegravir or a pharmaceutically acceptable salt thereof and lenacapavir or a pharmaceutically acceptable salt thereof, wherein the amount of bictegravir or a pharmaceutically acceptable salt thereof corresponds to about 75 mg bictegravir free acid and the amount of lenacapavir or a pharmaceutically acceptable salt thereof corresponds to about 50 mg lenacapavir free acid. In some embodiments, this monolayer tablet provides a bioavailability of at least about 3% of lenacapavir in fasted, pentagastrin pre-treated dogs.

In some embodiments" the tablet which provides the bioavailability of at least about 30% of bictegravir in fasted, pentagastrin pre-treated dogs is a bilayer tablet comprising (a) a first layer comprising bictegravir or a pharmaceutically acceptable salt thereof and (b) a second layer comprising lenacapavir or a pharmaceutically acceptable salt thereof, wherein the amount of bictegravir or a pharmaceutically acceptable salt thereof corresponds to about 75 mg bictegravir free acid and the amount of lenacapavir or a pharmaceutically acceptable salt thereof corresponds to about 25 mg lenacapavir free acid. In some embodiments, this monolayer tablet provides a bioavailability of at least 2% of lenacapavir in fasted, pentagastrin pre-treated dogs.

F, Cₘₐₓ, AUC_{0-Day8}, AUC_{inf}, and AUCₗₐₛₜ are standard pharmacokinetic parameters that can be estimated manually or by using modelling software well known in the art, such as the Pharsight WinNonlin package using a non-compartmental model. The general basis for calculation of these quantities is well-known *(e.g.* see Rowland & Tozer (2010) Clinical Pharmacokinetics and Pharmacodynamics: Concepts and Applications ISBN 978-0781750097, or Jambhekar & Breen (2012) Basic Pharmacokinetics ISBN 978-0853699804). Typically, the parameters will be assessed as the average (*e.g.* geometric or arithmetic mean) from within a group of at least 12 (and normally between 24 and 60) healthy human adults. Parameters should be measured in accordance with standards and practices which would be acceptable to a pharmaceutical regulatory agency such as FDA, EMA, MHLW, or WHO. The values may be based on measurements taken at appropriate intervals following the time of tablet ingestion, such as every hour, or at increasingly sparse sampling intervals, such as 1, 3, 5, 7, 9, 11, 13, 15, 20, and 24 hours after ingestion. They can be assessed either following a single-dose of drug or at steady state but will typically be assessed following a single-dose. The clinical data were assessed following a single dose administration.

### Manufacturing methods

Methods for producing the compositions and dosage forms (in particular the tablets of the disclosure) described herein are also provided. A tablet can be made by compression or molding, optionally with one or more excipients. Compressed tablets may be prepared by compressing in a suitable machine the active ingredient in a free-flowing form such as a powder or granules, optionally mixed with excipients. In general, tableting methods are well known in the art of pharmacy. Techniques and formulations generally are found in Remington's Pharmaceutical Sciences (Mack Publishing Co., Easton, PA), which is hereby incorporated by reference herein in its entirety.

In some embodiments, the method comprises blending bictegravir and lenacapavir (or their pharmaceutically acceptable salts) with excipients, followed by compression.

In some embodiments of forming a monolayer tablet according to the disclosure, bictegravir and lenacapavir (or their pharmaceutically acceptable salts) are first co-blended with excipients and granulated, for example by dry granulation. This step involves, in certain embodiments, roller compaction and/or milling. In some embodiments, the granulation of the co-blended bictegravir and lenacapavir (or their pharmaceutically acceptable salts) are further combined with extragranular excipients, including but not limited to magnesium stearate, then compressed.

In some embodiments a multilayer tablet *(e.g.* a bilayer tablet) is produced, the method comprising (a) compressing the bictegravir or a pharmaceutically acceptable salt thereof with excipients as a first layer, and (b) compressing lenacapavir or a pharmaceutically acceptable salt thereof with excipients to form a second layer. The first layer and second layer may be compressed separately and subsequently combined. In some embodiments, the first layer is formed by compression and subsequently the second layer is compressed onto the first layer.

In some embodiments a multilayer tablet *(e.g.* a bilayer tablet) is produced, the method comprising (a) compressing the lenacapavir or a pharmaceutically acceptable salt thereof with excipients as a first layer, and (b) compressing bictegravir or a pharmaceutically acceptable salt thereof with excipients to form a second layer. The first layer and second layer may be compressed separately and subsequently combined. In some embodiments, the first layer is formed by compression and subsequently the second layer is compressed onto the first layer.

In some embodiments, lenacapavir or a pharmaceutically acceptable salt thereof is spray dried prior to further formulation to form a lenacapavir dispersion including one or more excipients. In some embodiments, a lenacapavir dispersion is obtained by spray drying a mixture comprising lenacapavir or a pharmaceutically acceptable salt thereof and one or more excipients in a suitable solvent. Solvents suitable for spray-drying can be any organic solvent in which lenacapavir or a pharmaceutically acceptable salt thereof is miscible. The solvent should have relatively low toxicity and be removed from the dispersion to a level that is acceptable according to The International Committee on Harmonization (ICH) guidelines. Removal of solvent to this level may require a post drying step such as for instance tray-drying, subsequent to the spray-drying process. Solvents include alcohols such as methanol, ethanol, n-propanol, iso-propanol, and butanol, in particular methanol; ketones such as acetone, methyl ethyl ketone and methyl iso-butyl ketone; esters such as ethyl acetate and propylacetate; and various other solvents such as acetonitrile, dichloromethane, toluene, and 1,1,1-trichloroethane. Lower volatility solvents such as dimethyl acetamide or dimethylsulfoxide can also be used.

The lenacapavir dispersion may be blended and granulated with bictegravir or a pharmaceutically acceptable salt thereof, optionally with excipients. This step may be done by roller compaction and/or milling. In some embodiments, the step is done by roller compaction. The co-blended bictegravir and lenacapavir granules are further combined with extragranular excipients, then compressed to form a monolayer tablet. In some embodiments, the excipient is magnesium stearate.

In some embodiments, a bilayer tablet is produced. Prior to the production of the tablet, the lenacapavir or a pharmaceutically acceptable salt thereof is generally spray dried with one or one or more excipients to form a lenacapavir dispersion. The lenacapavir dispersion may be blended and granulated with additional excipients. This step may be done by roller compaction and/or milling. In some embodiments, this step is done by milling. The lenacapavir granules are further combined with extragranular excipients (in some embodiments magnesium stearate). Separately, when producing a bilayer tablet of the present disclosure, the bictegravir or a pharmaceutically acceptable salt thereof is blended with excipients and granulated (in some embodiments using roller compaction). In some embodiments, the bictegravir granules are then compressed as a first layer and subsequently, the lenacapavir granules are compressed as a second layer to form a bilayer tablet. In some embodiments, the lenacapavir granules are then compressed as a first layer and subsequently, the bictegravir granules are compressed as a second layer to form a bilayer tablet. The first layer and second layer may be compressed separately and subsequently combined. In some embodiments, the first layer is formed by compression and subsequently the second layer is compressed onto the first layer. In some embodiments, the resulting bilayer tablet is coated with a film coating. In some embodiments, this tablet is coated with a non-functional coating.

In some embodiments, the methods include a step of coating the tablet cores after compression, *e.g.* with a film coating as described above.

### Therapeutic methods

The tablets of the disclosure may be used to treat or prevent HIV (*e.g.* HIV-1). In some embodiments, the tablets of the disclosure may be used to treat or prevent HIV-1 or HIV-2.

The tablets of the disclosure may be used to treat HIV (*e.g.* HIV-1). In some embodiments, the tablets of the disclosure may be used to treat HIV-1 or HIV-2.

In certain embodiments, the tablets of the disclosure may be used to prevent HIV (*e.g.* HIV-1). In some embodiments, the tablets of the disclosure may be used to prevent HIV-1 or HIV-2.

Accordingly, methods for treating a subject having HIV are provided, comprising administering a tablet of the disclosure to the subject. Similarly, a tablet of the disclosure is provided for use in such treatment methods.

The disclosure also provides the use of bictegravir or a pharmaceutically acceptable salt thereof and lenacapavir or a pharmaceutically acceptable salt thereof, in the manufacture of a tablet of the disclosure for treating HIV. In some embodiments, the disclosure provides the use of bictegravir or a pharmaceutically acceptable salt thereof, and lenacapavir or a pharmaceutically acceptable salt thereof, in the manufacture of tablet of the disclosure for preventing HIV.

A method of treating an HIV infection in a human having or at risk of having the infection is provided, wherein the method includes administering to the human the tablets disclosed herein. Similarly, a method of preventing HIV infection in a human having or at risk of becoming infected infection is provided, wherein the method includes administering to the human the tablets disclosed herein. A method of preventing HIV infection from taking hold if the individual is exposed to the virus and/or to keep the virus from establishing a permanent infection and/or to prevent the appearance of symptoms of the disease and/or to prevent the virus from reaching detectable levels in the blood is also provided, for example for pre-exposure prophylaxis (PrEP) or post-exposure prophylaxis (PEP). Accordingly, methods for reducing the risk of acquiring HIV (*e.g*., HIV-1 and/or HIV-2) are provided. In some embodiments, the human patient is virologically suppressed, meaning that the patient's HIV RNA level in serum viral load has been reduced to less than 50 copies/mL or to a level below the limits of detection. In some embodiments, the human patient is virologically suppressed and treatment experienced, meaning that the patient has previously taken one or more forms of HIV medication.

The tablets disclosed herein are provided for use in the treatment of an HIV infection in a human having or at risk of having the infection. Similarly, the tablets disclosed herein are provided for use in the prevention of an HIV infection in a human having or at risk of having the infection. The tablets disclosed herein are also provided for use in a method of preventing HIV infection from taking hold if the individual is exposed to the virus and/or to keep the virus from establishing a permanent infection and/or to prevent the appearance of symptoms of the disease and/or to prevent the virus from reaching detectable levels in the blood, for example for pre-exposure prophylaxis (PrEP) or post-exposure prophylaxis (PEP). Accordingly, the tablets are provided for use in methods for reducing the risk of acquiring HIV (*e.g*., HIV-1 and/or HIV-2). In some embodiments, the human patient is virologically suppressed. In some embodiments, the human patient is virologically suppressed and treatment experienced, meaning that the patient has previously taken one or more forms of HIV medication.

A method of using a tablet disclosed herein in therapy is provided. In particular, a method of treating the proliferation of the HIV virus, treating AIDS, or delaying the onset of AIDS or ARC symptoms in a human (*e.g*., men having sex with men or transsexual women having sex with men) is provided, comprising administering to the human a tablet disclosed herein.

A tablet disclosed herein for use in a method of therapy is provided. In particular, a tablet disclosed herein for use in a method of treating the proliferation of the HIV virus, treating AIDS, or delaying the onset of AIDS or ARC symptoms in a human (*e.g*., for example men having sex with men or transsexual women having sex with men) is provided.

The human patient having an HIV infection may be virologically suppressed, meaning that the patient's HIV RNA level in serum viral load has been reduced to less than 50 copies/mL or to a level below the limits of detection. In one embodiment, the human patient is treatment experienced, meaning that the patient has previously taken one or more forms of HIV medication. The tablets disclosed herein may be provided for use to prevent HIV infection from taking hold if the individual is exposed to the virus and/or to keep the virus from establishing a permanent infection and/or to prevent the appearance of symptoms of the disease and/or to prevent the virus from reaching detectable levels in the blood, for example for pre-exposure prophylaxis (PrEP) or post-exposure prophylaxis (PEP). Accordingly, in certain embodiments, methods for reducing the risk of acquiring HIV (*e.g*., HIV-1 and/or HIV-2) are provided. For example, methods for reducing the risk of acquiring HIV (*e.g*., HIV-1 and/or HIV-2) comprise administration of the tablets disclosed herein. In certain specific embodiments, methods for reducing the risk of acquiring HIV (*e.g*., HIV-1 and/or HIV-2) comprise administration of a tablet disclosed herein in combination with safer sex practices. In certain embodiments, methods for reducing the risk of acquiring HIV *(e.g.,* HIV-1 and/or HIV-2) comprise administration to an individual at risk of acquiring HIV. Examples of individuals at high risk for acquiring HIV include, without limitation, an individual who is at risk of sexual transmission of HIV.

In another embodiment, the use of a tablet disclosed herein for the manufacture of a medicament for the treatment of an HIV infection in a human being having or at risk of having the infection is disclosed. Similarly, in another embodiment, the use of a tablet disclosed herein for the manufacture of a medicament for the prevention of an HIV infection in a human being having or at risk of having the infection is disclosed.

In another embodiment, an article of manufacture comprising a tablet disclosed herein; and packaging material comprising a label which indicates that the tablet can be used to treat infection by HIV is disclosed. Similarly, in another embodiment, an article of manufacture comprising a tablet disclosed herein; and packaging material comprising a label which indicates that the tablet can be used to prevent HIV infection is disclosed.

The methods disclosed herein involve administering tablet disclosed herein to the subject, typically a human, and will generally involve repeated administrations, typically once daily. The treatment may be prophylactic or therapeutic treatment.

The tablets disclosed herein may be administered prior to and/or after an event that would expose the individual to HIV or that would otherwise increase the individual's risk of acquiring HIV, *e.g.,* as pre-exposure prophylaxis (PrEP) and/or as post-exposure prophylaxis (PEP). Examples of events that could increase an individual's risk of acquiring HIV include, without limitation, no condom use during anal intercourse with an HIV positive partner or a partner of unknown HIV status; anal intercourse with more than 3 sex partners; exchange of money, gifts, shelter or drugs for anal sex; sex with male partner and diagnosis of sexually transmitted infection; and no consistent use of condoms with sex partner known to be HIV positive.

In certain embodiments, *e.g*., when administered as PrEP, the tablets disclosed herein are administered 2 to 72 hours, 2 to 48 hours, 2 to 24 hours, or 2 to 12 hours prior to an event that would increase the individual's risk of acquiring HIV (*e.g*., prior to sex or other exposure to the HIV virus). In some embodiments, the tablets disclosed herein are administered within 72 hours, 60 hours, 48 hours, 24 hours, 12 hours, 9 hours, 6 hours, 4 hours, 3 hours, 2 hours or 1 hour prior to an event that would increase the individual's risk of acquiring HIV (*e.g*., prior to sex or other exposure to the HIV virus). In certain embodiments, when the tablets disclosed herein are administered prior to an event that would increase the individual's risk of acquiring HIV, they are administered daily prior to the event. In certain embodiments, when the tablets disclosed herein are administered prior to an event that would increase the individual's risk of acquiring HIV, they are administered one to three times prior to the event.

In certain embodiments, *e.g*., when administered as part of a PrEP regimen or as PEP, the tablets disclosed herein are administered 2 to 48 hours, 2 to 36 hours, 2 to 24 hours, or 2 to 12 hours following an event that would increase the individual's risk of acquiring HIV (*e.g*., following sex or other exposure to the HIV virus). In certain embodiments, *e.g*., when administered as PEP, the tablets disclosed herein are administered for 7 days, 14 days, 21 days, 28 days, 30 days, or 45 days following an event that would increase the individual risk of acquiring HIV (*e.g*., following sex or other exposure to the HIV virus). In certain embodiments, *e.g*., when administered as PEP, the tablets disclosed herein are administered for 30 days following an event that would increase the individual risk of acquiring HIV (*e.g*., following sex or other exposure to the HIV virus). In certain embodiments, the tablets disclosed herein are administered less than 1 hour, 2 hours, 3 hours, 4, hours, 5 hours, 6 hours, 7 hours, 8 hours, 9 hours, 12 hours, 18 hours, 24 hours, 36 hours, or 48 hours following an event that would increase the individual's risk of acquiring HIV (*e.g*., following sex or other exposure to the HIV virus). In certain other embodiments, tablets disclosed herein are administered for 1 day, 2 days, 3, days 4 days, or 5 days following an event that would increase the individual's risk of acquiring HIV (*e.g*., following sex or other exposure to the HIV virus). In certain embodiments, when the tablets disclosed herein are administered following to an event that would increase the individual's risk of acquiring HIV, they are administered daily following to the event. In certain embodiments, when the tablets disclosed herein are administered following an event that would increase the individual's risk of acquiring HIV, they are administered one to three times following the event. In certain embodiments, when the tablets disclosed herein are administered following to an event that would increase the individual's risk of acquiring HIV, they are administered once following the event.

In certain embodiments, (*e.g*., when administered as PrEP, the tablets disclosed herein are administered prior to an event that would increase the individual's risk of acquiring HIV *(e.g.,* prior to sex) and following the event. For example, in certain embodiments, *e.g*., when administered as PrEP, the tablets disclosed herein are administered 2 to 72 hours, 2 to 48 hours, 2 to 24 hours, or 2 to 12 hours prior to an event that would increase the individual's risk of acquiring HIV (*e.g*., prior to sex) and 2 to 48 hours, 2 to 36 hours, 2 to 24 hours, or 2 to 12 hours following the event. For example, in some embodiments, one or more (*e.g*., one, two, or three) tablets disclosed herein are administered one to three days prior to an event that would increase the individual's risk of acquiring HIV (*e.g*., prior to sex) and once per day for a period of one to five days following the event. In some embodiments, one or more (*e.g*., one, two, or three) tablets disclosed herein are administered 2 to 24 hours prior to an event that would increase the individual's risk of acquiring HIV (*e.g*., prior to sex) and one or more times (*e.g*., one, two, or three times) 2 to 48 hours following the event. In some embodiments, the tablets disclosed herein are administered once per week, twice per week, three times per week, four times per week, or five times per week and one or more times (*e.g*., one, two, or three times) 2 to 48 hours following an event that would increase the individual's risk of acquiring HIV (*e.g*., prior to sex). In one embodiment, the tablets disclosed herein are administered twice per week (one composition (*i.e*., tablet) per day) and once (one composition) following an event that increases the individual's risk of acquiring HIV (*e.g*., one tablet within 24 hours of exposure, such as following sex).

In some embodiments, the tablet is administered once daily. In some embodiments, the administration follows a loading dose of lenacapavir or pharmaceutically acceptable salt thereof corresponding to about 1200 mg lenacapavir free acid. This loading dose may be administered over multiple days. In some embodiments, the loading dose is administered over two days. In one embodiment, an amount of lenacapavir or pharmaceutically acceptable salt thereof corresponding to about 600 mg lenacapavir free acid is administered on the first day, and an amount of lenacapavir or pharmaceutically acceptable salt thereof corresponding to about 600 mg lenacapavir free acid is administered on the second day.

In some embodiments, the loading dose is administered over eight days. In one embodiment, an amount of lenacapavir or pharmaceutically acceptable salt thereof corresponding to about 600 mg lenacapavir free acid is administered on the first day, an amount of lenacapavir or pharmaceutically acceptable salt thereof corresponding to about 600 mg lenacapavir free acid is administered on the second day, and an amount of lenacapavir or pharmaceutically acceptable salt thereof corresponding to about 300 mg lenacapavir free acid is administered on the eighth day.

### Monolayer tablet comprising 75 mg bictegravir and 50 mg lenacapavir

In one embodiment, the disclosure provides a monolayer tablet comprising an amount of bictegravir or pharmaceutically acceptable salt thereof which corresponds to about 75 mg bictegravir free acid and an amount of lenacapavir or pharmaceutically acceptable salt thereof which corresponds to about 50 mg lenacapavir free acid.

The monolayer tablet may further comprise copovidone, poloxamer, mannitol, microcrystalline cellulose, croscarmellose sodium, and magnesium stearate.

This monolayer tablet may have the following composition: about 10-25 %w/w bictegravir or a pharmaceutically acceptable salt thereof, about 10-20 %w/w lenacapavir or a pharmaceutically acceptable salt thereof, about 1-5 %w/w copovidone, about 0.1-2 %w/w poloxamer, about 30-50 %w/w mannitol, about 10-30 %w/w microcrystalline cellulose, about 5-15 %w/w croscarmellose sodium and about 0.1-10 %w/w magnesium stearate.

More specifically, this monolayer tablet may have the following composition: about 10-25 %w/w bictegravir or a pharmaceutically acceptable salt thereof, about 10-20 %w/w lenacapavir or a pharmaceutically acceptable salt thereof, about 1-3 %w/w copovidone, about 0.3-0.8 %w/w poloxamer, about 35-45 %w/w mannitol, about 15-25 %w/w microcrystalline cellulose, about 6-10 %w/w croscarmellose sodium and about 1-2 %w/w magnesium stearate.

Even more specifically, this monolayer tablet may have the following composition: about 15-20 %w/w bictegravir or a pharmaceutically acceptable salt thereof, about 10-14 %w/w lenacapavir or a pharmaceutically acceptable salt thereof, about 2-3 %w/w copovidone, about 0.6-0.8 %w/w poloxamer, about 35-40 %w/w mannitol, about 18-22 %w/w microcrystalline cellulose, about 7-9 %w/w croscarmellose sodium and about 1-2 %w/w magnesium stearate.

Still more specifically, this monolayer tablet may have the following composition: about 18 %w/w bictegravir or a pharmaceutically acceptable salt thereof, about 12 %w/w lenacapavir or a pharmaceutically acceptable salt thereof about 2.8 %w/w copovidone, about 0.8 %w/w poloxamer, about 38 %w/w mannitol, about 19 %w/w microcrystalline cellulose, about 8 %w/w croscarmellose sodium and about 1.5 %w/w magnesium stearate.

This monolayer tablet may comprise about 5-15 mg copovidone, about 0.5-5 mg poloxamer, about 150-200 mg mannitol, about 70-100 mg microcrystalline cellulose, about 25-50 mg croscarmellose sodium, and about 5-10 mg magnesium stearate.

More specifically, this monolayer tablet may comprise about 10-15 mg copovidone, about 2-5 mg poloxamer, about 150-175 mg mannitol, about 75-90 mg microcrystalline cellulose, about 30-40 mg croscarmellose sodium, about 5-8 mg magnesium stearate.

Even more specifically, this monolayer tablet may comprise about 12 mg copovidone, about 3 mg poloxamer, about 165 mg mannitol, about 83 mg microcrystalline cellulose, about 35 mg croscarmellose sodium, about 7 mg magnesium stearate.

Any of the above monolayer tablets disclosed in this section may be coated or uncoated. In some embodiments, the tablet is coated. The coating is a film coating. In some embodiments, the coating is a non-functional film coating. In some embodiments, the coating is a titanium dioxide free film coating.

Any of the above monolayer tablets disclosed in this section may weigh about 430-440 mg excluding the film coating.

This monolayer tablet may contain bictegravir free acid or a pharmaceutically acceptable salt of bictegravir. In one embodiment, the monolayer tablet contains a pharmaceutically acceptable salt of bictegravir. In one embodiment, the pharmaceutically acceptable salt of bictegravir is bictegravir sodium, *i.e.* the monolayer tablet comprises about 79 mg bictegravir sodium.

This monolayer tablet may contain lenacapavir free acid or a pharmaceutically acceptable salt of lenacapavir. In one embodiment, the monolayer tablet contains a pharmaceutically acceptable salt of lenacapavir. In some embodiments, the pharmaceutically acceptable salt of lenacapavir is lenacapavir sodium, *i.e.* the monolayer tablet comprises about 51 mg lenacapavir sodium.

In one embodiment, the lenacapavir or pharmaceutically acceptable salt thereof in the above monolayer tablet is present in a spray dried dispersion.

This monolayer tablet may release at least about 50% of the bictegravir or pharmaceutically acceptable salt thereof in about 30 minutes, measured using USP apparatus II, in 250 mL of fasted-state simulated intestinal fluid, pH 6.5, at 37 °C and paddle speed of 75 rpm. Additionally, this monolayer tablet may release at least about 50% of the lenacapavir or pharmaceutically acceptable salt thereof in about 15 minutes, measured using USP apparatus II, in 900 mL of 2% w/w Cremophor EL in 30 mM potassium phosphate, pH 6.0, at 37 °C and paddle speed of 75 rpm. In one embodiment, this monolayer tablet may release at least about 70% of the lenacapavir or pharmaceutically acceptable salt thereof in about 15 minutes. In one embodiment, this monolayer tablet may release at least about 80% of the lenacapavir or pharmaceutically acceptable salt thereof in about 15 minutes.

This monolayer tablet is provided for use in the therapeutic or prophylactic treatment of an HIV infection in a subject. For example, the monolayer tablet may be used to treat an HIV infection. Alternatively, the monolayer tablet may be used to prevent an HIV infection. The monolayer tablet may be used in pre-exposure prophylaxis (PrEP) or post-exposure prophylaxis (PEP) to prevent an HIV infection.

In some embodiments, the subject to be treated is virologically suppressed. The subject may be treatment experienced or treatment inexperienced. In some embodiments, the subject is treatment experienced. The monolayer tablet above is provided for use in the treatment of an HIV infection in a virologically suppressed, treatment experienced patient.

Generally, this monolayer tablet may be administered once daily. In some embodiments, the administration follows a loading dose of lenacapavir or pharmaceutically acceptable salt thereof corresponding to about 1200 mg lenacapavir free acid. This loading dose may be administered over multiple days, in some embodiments, over two days. For example, an amount of lenacapavir or pharmaceutically acceptable salt thereof corresponding to about 600 mg lenacapavir free acid may be administered on the first day, and an amount of lenacapavir or pharmaceutically acceptable salt thereof corresponding to about 600 mg lenacapavir free acid may be administered on the second day.

In some embodiments, the loading dose is administered over eight days. In one embodiment, an amount of lenacapavir or pharmaceutically acceptable salt thereof corresponding to about 600 mg lenacapavir free acid is administered on the first day, an amount of lenacapavir or pharmaceutically acceptable salt thereof corresponding to about 600 mg lenacapavir free acid is administered on the second day, and an amount of lenacapavir or pharmaceutically acceptable salt thereof corresponding to about 300 mg lenacapavir free acid is administered on the eighth day.

### Bilayer tablet comprising 75 mg bictegravir and 25 mg lenacapavir

In one aspect, the disclosure provides a bilayer tablet comprising an amount of bictegravir or pharmaceutically acceptable salt thereof which corresponds to about 75 mg bictegravir free acid and an amount of lenacapavir or pharmaceutically acceptable salt thereof which corresponds to about 25 mg lenacapavir free acid. In some embodiments, a bilayer tablet comprises (a) a first layer comprising an amount of bictegravir or pharmaceutically acceptable salt thereof which corresponds to about 75 mg bictegravir free acid and (b) a second layer comprising an amount of lenacapavir or pharmaceutically acceptable salt thereof which corresponds to about 25 mg lenacapavir free acid. In some embodiments, the first layer is substantially free of lenacapavir or a pharmaceutically acceptable salt thereof, and the second layer is substantially free of bictegravir or a pharmaceutically acceptable salt thereof.

This bilayer tablet may further comprise copovidone, poloxamer, mannitol, microcrystalline cellulose, croscarmellose sodium, and magnesium stearate.

The first layer of the bilayer tablet may comprise bictegravir or a pharmaceutically acceptable salt thereof, microcrystalline cellulose, croscarmellose sodium, and magnesium stearate. The first layer may contain mannitol, for example in a weight ratio of mannitol to microcrystalline cellulose of about 2: 1 to about 1:2, such as about 2: 1, about 1:1, or about 1:2. In some embodiments, the first layer of the bilayer tablet is substantially free from mannitol (*i*.*e*. less than 1 %w/w mannitol, in some embodiments, no mannitol). In some embodiments, microcrystalline cellulose is the only filler in the first layer. The second layer of the bilayer tablet may comprise lenacapavir or a pharmaceutically acceptable salt thereof, copovidone, poloxamer, mannitol, microcrystalline cellulose, croscarmellose sodium, and magnesium stearate.

In some embodiments, a bilayer tablet comprises a first layer comprising an amount of bictegravir or pharmaceutically acceptable salt thereof which corresponds to about 75 mg bictegravir free acid, microcrystalline cellulose, croscarmellose sodium, and magnesium stearate, and a second layer comprising an amount of lenacapavir or pharmaceutically acceptable salt thereof which corresponds to about 25 mg lenacapavir free acid, copovidone, poloxamer, mannitol, microcrystalline cellulose, croscarmellose sodium, and magnesium stearate. In some embodiments, the first layer of the bilayer tablet is substantially free from mannitol (*i*.*e*. less than about %w/w mannitol, in some embodiments, no mannitol).

This bilayer tablet may have the following composition: about 10-25 %w/w bictegravir or a pharmaceutically acceptable salt thereof, about 5-20 %w/w lenacapavir or a pharmaceutically acceptable salt thereof, about 0.1-5 %w/w copovidone, about 0.1-2 %w/w poloxamer, about 5-30 %w/w mannitol, about 30-60 %w/w microcrystalline cellulose, about 5-20 %w/w croscarmellose sodium and about 0.1-5 %w/w magnesium stearate.

More specifically, this bilayer tablet may have the following composition: about 10-25 %w/w bictegravir or a pharmaceutically acceptable salt thereof, about 5-20 %w/w lenacapavir or a pharmaceutically acceptable salt thereof, about 1-3 %w/w copovidone, about 0.3-0.8 %w/w poloxamer, about 10-25 %w/w mannitol, about 40-60 %w/w microcrystalline cellulose, about 6-10 %w/w croscarmellose sodium and about 1-2 %w/w magnesium stearate.

Even more specifically, this bilayer tablet may have the following composition: about 15-20 %w/w bictegravir or a pharmaceutically acceptable salt thereof, about 5-10 %w/w lenacapavir or a pharmaceutically acceptable salt thereof, about 1-3 %w/w copovidone, about 0.3-0.8 %w/w poloxamer, about 10-25 %w/w mannitol, about 45-55 %w/w microcrystalline cellulose, about 6-10 %w/w croscarmellose sodium and about 1-2 %w/w magnesium stearate, for example a first layer comprising about 10-25 %w/w bictegravir or a pharmaceutically acceptable salt thereof, about 40-50 %w/w microcrystalline cellulose, about 1-10 %w/w croscarmellose sodium, and about 0.1-3 %w/w magnesium stearate, and a second layer comprising about 5-20 %w/w lenacapavir or a pharmaceutically acceptable salt thereof, about 0.1-5 %w/w copovidone, about 0.1-2 %w/w poloxamer, about 5-20 %w/w mannitol, about 1-10 %w/w microcrystalline cellulose, about 1-10 %w/w croscarmellose sodium, and about 0.1-1 %w/w magnesium stearate. In these embodiments, the weight percentages denote a proportion of the whole tablet.

Still more specifically, this bilayer tablet may have the following composition: about 18 %w/w bictegravir or a pharmaceutically acceptable salt thereof, about 6 %w/w lenacapavir or a pharmaceutically acceptable salt thereof, about 1.4 %w/w copovidone, about 0.4 %w/w poloxamer, about 12.5 %w/w mannitol, about 51.7 %w/w microcrystalline cellulose, about 8 %w/w croscarmellose sodium and about 1.5 %w/w magnesium stearate. In some embodiments, a first layer comprises about 18 %w/w bictegravir or a pharmaceutically acceptable salt thereof, about 45 %w/w microcrystalline cellulose, about 6 %w/w croscarmellose sodium, and about 1 %w/w magnesium stearate, and a second layer comprises about 6 %w/w lenacapavir or a pharmaceutically acceptable salt thereof, about 1.4 %w/w copovidone, about 0.4 %w/w poloxamer, about 12.5 %w/w mannitol, about 6 %w/w microcrystalline cellulose, and about 0.4 %w/w magnesium stearate. In these embodiments, the weight percentages denote a proportion of the whole tablet.

This bilayer tablet may comprise about 5-15 mg copovidone, about 0.5-5 mg poloxamer, about 50-125 mg mannitol, about 200-250 mg microcrystalline cellulose, about 30-50 mg croscarmellose sodium, and about 5-10 mg magnesium stearate.

More specifically, this bilayer tablet may comprise about 5-15 mg copovidone, about 0.5-5 mg poloxamer, about 50-125 mg mannitol, about 200-250 mg microcrystalline cellulose, about 30-50 mg croscarmellose sodium and about 5-10 mg magnesium stearate.

Even more specifically, this bilayer tablet may comprise about 5-15 mg copovidone, about 0.5-5 mg poloxamer, about 50-125 mg mannitol, about 200-250 mg microcrystalline cellulose, about 30-50 mg croscarmellose sodium and about 5-10 mg magnesium stearate.

For example, the first layer of the above bilayer tablet may further comprise about 185-200 mg microcrystalline cellulose, about 20-30 mg croscarmellose sodium and about 3-5 mg magnesium stearate.

More specifically, the first layer of the bilayer tablet may further comprise about 193 mg microcrystalline cellulose, about 24 mg croscarmellose sodium and about 5 mg magnesium stearate. Even more specifically, the first layer may further comprise about 193.0 mg microcrystalline cellulose, about 24.0 mg croscarmellose sodium and about 4.5 mg magnesium stearate. The first layer may contain also contain mannitol. In some embodiments, the first layer of this bilayer tablet does not contain any filler besides microcrystalline cellulose.

The second layer of the above bilayer tablet may further comprise about 5-8 mg copovidone, about 1-3 mg poloxamer, about 45-60 mg mannitol, about 20-30 mg microcrystalline cellulose, about 5-15 mg croscarmellose sodium and about 1-3 mg magnesium stearate.

More specifically, the second layer of the above bilayer tablet may further comprise about 6 mg copovidone, about 2 mg poloxamer, about 53 mg mannitol, about 27 mg microcrystalline cellulose, about 10 mg croscarmellose sodium and about 2 mg magnesium stearate.

Even more specifically, the second layer of the above bilayer tablet may further comprise about 6.1 mg copovidone, about 1.7 mg poloxamer, about 53.2 mg mannitol, about 26.6 mg microcrystalline cellulose, about 10.0 mg croscarmellose sodium and about 1.9 mg magnesium stearate.

In some embodiments, the bilayer tablet may have the following composition: a first layer comprising an amount of bictegravir or pharmaceutically acceptable salt thereof corresponding to about 75 mg bictegravir free acid, about 175-210 mg microcrystalline cellulose, about 15-35 mg croscarmellose sodium, and about 1-10 mg magnesium stearate, and a second layer comprising an amount of lenacapavir or pharmaceutically acceptable salt thereof corresponding to about 25 mg lenacapavir free acid, about 1-10 mg copovidone, about 1-6 mg poloxamer, about 30-75 mg mannitol, about 15-40 mg microcrystalline cellulose, about 1-20 mg croscarmellose sodium and about 1-10 mg magnesium stearate.

More specifically, the bilayer tablet above may have the following composition: a first layer comprising an amount of bictegravir or pharmaceutically acceptable salt thereof corresponding to about 75 mg bictegravir free acid, about 185-200 mg microcrystalline cellulose, about 20-30 mg croscarmellose sodium and about 3-5 mg magnesium stearate, and a second layer comprising an amount of lenacapavir or pharmaceutically acceptable salt thereof corresponding to about 25 mg lenacapavir free acid, about 5-8 mg copovidone, about 1-3 mg poloxamer, about 45-60 mg mannitol, about 20-30 mg microcrystalline cellulose, about 5-15 mg croscarmellose sodium and about 1-3 mg magnesium stearate.

Even more specifically, the bilayer tablet above may have the following composition: a first layer comprising an amount of bictegravir or pharmaceutically acceptable salt thereof corresponding to about 75 mg bictegravir free acid, about 193 mg microcrystalline cellulose, about 24 mg croscarmellose sodium and about 5 mg magnesium stearate, and an amount of lenacapavir or pharmaceutically acceptable salt thereof corresponding to about 25 mg lenacapavir free acid, about 6 mg copovidone, about 2 mg poloxamer, about 53 mg mannitol, about 27 mg microcrystalline cellulose, about 10 mg croscarmellose sodium and about 2 mg magnesium stearate.

Still more specifically, the bilayer tablet above has the following composition: a first layer comprising an amount of bictegravir or pharmaceutically acceptable salt thereof corresponding to about 75 mg bictegravir free acid, about 193.0 mg microcrystalline cellulose, about 24.0 mg croscarmellose sodium and about 4.5 mg magnesium stearate, and a second layer comprising an amount of lenacapavir or pharmaceutically acceptable salt thereof corresponding to about 25 mg lenacapavir free acid, about 6.1 mg copovidone, about 1.7 mg poloxamer, about 53.2 mg mannitol, about 26.6 mg microcrystalline cellulose, about 10.0 mg croscarmellose sodium and about 1.9 mg magnesium stearate.

Any of the above bilayer tablets disclosed in this section may be coated or uncoated. In some embodiments, the bilayer is coated. In some embodiment, the coating is a film coating. In some embodiment, the coating is a non-functional film coating.

Any of the above bilayer tablets disclosed in this section may weigh about 420-430 mg excluding the film coating.

This bilayer tablet may contain bictegravir free acid or a pharmaceutically acceptable salt of bictegravir. In some embodiments, the bilayer tablet contains a pharmaceutically acceptable salt of bictegravir. In some embodiments, the pharmaceutically acceptable salt of bictegravir is bictegravir sodium, *i.e.* the bilayer tablet comprises about 79 mg bictegravir sodium.

This bilayer tablet may contain lenacapavir free acid or a pharmaceutically acceptable salt of lenacapavir. In some embodiments, the bilayer tablet contains a pharmaceutically acceptable salt of lenacapavir. In some embodiments, the pharmaceutically acceptable salt of lenacapavir is lenacapavir sodium, *i.e.* the bilayer tablet comprises about 26 mg lenacapavir sodium.

In some embodiments, the lenacapavir or pharmaceutically acceptable salt thereof in the above bilayer tablet is present in a spray dried dispersion.

The above bilayer tablet may release at least about 50% of the bictegravir or pharmaceutically acceptable salt thereof in about 30 minutes, measured using USP apparatus II, in 250 mL of fasted-state simulated intestinal fluid, pH 6.5, at 37 °C and paddle speed of 75 rpm. In some embodiments, above bilayer tablet may release at least about 60% of the bictegravir or pharmaceutically acceptable salt thereof in about 30 minutes. Additionally, the above bilayer tablet may release at least about 50% of the lenacapavir or pharmaceutically acceptable salt thereof in about 15 minutes, measured using USP apparatus II, in 900 mL of 2% w/w Cremophor EL in 30 mM potassium phosphate, pH 6.0, at 37 °C and paddle speed of 75 rpm. In some embodiments, the above bilayer tablet may release at least about 70% of the lenacapavir or pharmaceutically acceptable salt thereof in about 15 minutes. In some embodiments, the above bilayer tablet may release at least about 80% of the lenacapavir or pharmaceutically acceptable salt thereof in about 15 minutes.

### Bilayer tablet comprising 75 mg bictegravir and 50 mg lenacapavir

In one aspect, the disclosure provides a bilayer tablet comprising an amount of bictegravir or pharmaceutically acceptable salt thereof which corresponds to about 75 mg bictegravir free acid and an amount of lenacapavir or pharmaceutically acceptable salt thereof which corresponds to about 50 mg lenacapavir free acid. In some embodiments, a bilayer tablet comprises (a) a first layer comprising an amount of bictegravir or pharmaceutically acceptable salt thereof which corresponds to about 75 mg bictegravir free acid and (b) a second layer comprising an amount of lenacapavir or pharmaceutically acceptable salt thereof which corresponds to about 50 mg lenacapavir free acid. In some embodiments, the first layer is substantially free of lenacapavir or a pharmaceutically acceptable salt thereof, and the second layer is substantially free of bictegravir or a pharmaceutically acceptable salt thereof.

This bilayer tablet may further comprise copovidone, poloxamer, mannitol, microcrystalline cellulose, croscarmellose sodium, and magnesium stearate.

The first layer of the bilayer tablet may comprise bictegravir or a pharmaceutically acceptable salt thereof, microcrystalline cellulose, croscarmellose sodium, and magnesium stearate. The first layer may contain mannitol, for example in a weight ratio of mannitol to microcrystalline cellulose of about 2: 1 to about 1:2, such as about 2: 1, about 1:1, or about 1:2. In some embodiments, the first layer of the bilayer tablet is substantially free from mannitol (i.e. less than about 1 %w/w mannitol, in some embodiments, no mannitol). In some embodiments, microcrystalline cellulose is the only filler in the first layer. The second layer of the bilayer tablet may comprise lenacapavir or a pharmaceutically acceptable salt thereof, copovidone, poloxamer, mannitol, microcrystalline cellulose, croscarmellose sodium, and magnesium stearate.

In some embodiments, a bilayer tablet comprises a first layer comprising an amount of bictegravir or pharmaceutically acceptable salt thereof which corresponds to about 75 mg bictegravir free acid, microcrystalline cellulose, croscarmellose sodium, and magnesium stearate, and a second layer comprising an amount of lenacapavir or pharmaceutically acceptable salt thereof which corresponds to about 50 mg lenacapavir free acid, copovidone, poloxamer, mannitol, microcrystalline cellulose, croscarmellose sodium, and magnesium stearate.

The bilayer tablet is provided be for use in the therapeutic or prophylactic treatment of an HIV infection in a subject. For example, the above bilayer tablet may be used to treat an HIV infection. Alternatively, the above bilayer tablet may be used to prevent an HIV infection. The monolayer tablet may be used in pre-exposure prophylaxis (PrEP) or post-exposure prophylaxis (PEP) to prevent an HIV infection.

In some embodiments, the subject to be treated is virologically suppressed. The subject may be treatment experienced or treatment inexperienced. In some embodiments, the subject is treatment experienced. In some embodiments, the bilayer tablet above is provided for use in the treatment of an HIV infection in a virologically suppressed patient. In some embodiments, the bilayer tablet above is provided for use in the treatment of an HIV infection in a virologically suppressed, treatment experienced patient.

Generally, the bilayer tablet may be administered once daily. In some embodiments, the administration follows a loading dose of lenacapavir or pharmaceutically acceptable salt thereof corresponding to about 1200 mg lenacapavir free acid. This loading dose may be administered over multiple days. In some embodiments, the loading dose is administered over two days. For example, an amount of lenacapavir or pharmaceutically acceptable salt thereof corresponding to about 600 mg lenacapavir free acid may be administered on the first day, and an amount of lenacapavir or pharmaceutically acceptable salt thereof corresponding to about 600 mg lenacapavir free acid may be administered on the second day.

In some embodiments, the loading dose is administered over eight days. In one embodiment, an amount of lenacapavir or pharmaceutically acceptable salt thereof corresponding to about 600 mg lenacapavir free acid is administered on the first day, an amount of lenacapavir or pharmaceutically acceptable salt thereof corresponding to about 600 mg lenacapavir free acid is administered on the second day, and an amount of lenacapavir or pharmaceutically acceptable salt thereof corresponding to about 300 mg lenacapavir free acid is administered on the eighth day.

### Definitions

The term "substantially free" in relation to the presence of a given component within *e.g.* a composition means that less than 5% by weight of the composition *(e.g.* less than 1% by weight of the composition) is that given component. The word "substantially" does not exclude "completely" *e.g.* a composition which is "substantially free" from Y may be completely free from Y. Where necessary, the word "substantially" may be omitted from the definition of the disclosure.

The term "comprise" and variations thereof, such as "comprises" and "comprising", are to be construed in an open, inclusive sense, that is as "including, but not limited to".

The term "between" with reference to two values includes those two values e.g. the range "between" 10 mg and 20 mg encompasses *e.g.* 10, 11, 12, 13, 14, 15, 16, 17, 18, 19 and 20 mg.

As used herein the term "about" is understood by the person of skill in the art to reflect the variability in the numerical value it modifies. Values expressed herein are understood to include a range of variability. To reflect this variability, any numerical value used herein incorporates this variability. As such, numerical values used herein encompass that value stated, as well as the value as modified with "about." In certain embodiments, the term "about" in relation to a numerical value *x* is optional and means, for example, *x*±10%, *x*±5%, or *x*±1%.

"% w/w" means the weight of a component as a percentage of the total weight or dosage form in which the component is present. For example, a composition comprising "5% w/w X" refers to a composition in which the weight of component X is 5% of the total weight of the composition.

Reference throughout this specification to "one embodiment", "an embodiment" or "some embodiments" means that a particular feature, structure or characteristic described in connection with the embodiment is included in at least one embodiment provided herein. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The term "pharmaceutically acceptable" with respect to a substance refers to that substance which is generally regarded as safe and suitable for use without undue toxicity, irritation, allergic response, and the like, commensurate with a reasonable benefit/risk ratio.

"Pharmaceutically acceptable salt" refers to a salt of a compound that is pharmaceutically acceptable and that possesses (or can be converted to a form that possesses) the desired pharmacological activity of the parent compound. Such salts include acid addition salts formed with inorganic acids such as hydrochloric acid, hydrobromic acid, sulfuric acid, nitric acid, phosphoric acid, and the like; or formed with organic acids such as acetic acid, benzenesulfonic acid, benzoic acid, camphorsulfonic acid, citric acid, ethanesulfonic acid, fumaric acid, glucoheptonic acid, gluconic acid, lactic acid, maleic acid, malonic acid, mandelic acid, methanesulfonic acid, 2-napththalenesulfonic acid, oleic acid, palmitic acid, propionic acid, stearic acid, succinic acid, tartaric acid, p-toluenesulfonic acid, trimethylacetic acid, and the like, and salts formed when an acidic proton present in the parent compound is replaced by either a metal ion, *e.g.,* an alkali metal ion, an alkaline earth ion, or an aluminum ion; or coordinates with an organic base such as diethanolamine, triethanolamine, N-methylglucamine and the like. Also included in this definition are ammonium and substituted or quatemized ammonium salts. Representative non-limiting lists of pharmaceutically acceptable salts can be found in S.M. Berge et al., J. Pharma Sci., 66(1), 1-19 (1977), and Remington: The Science and Practice of Pharmacy, R. Hendrickson, ed., 21st edition, Lippincott, Williams & Wilkins, Philadelphia, PA, (2005), at p. 732, Table 38-5, both of which are hereby incorporated by reference herein.

As used herein, the term "salts" includes co-crystals. The term "co-crystal" refers to a crystalline compound comprising two or more molecular components, e.g. wherein proton transfer between the molecular components is partial or incomplete. Accordingly, the term "pharmaceutically acceptable salt" as used herein encompasses salts and co-crystals as defined herein.

The term "solvate" means a molecular complex comprising a compound and one or more pharmaceutically acceptable solvent molecules. Examples of solvent molecules include water and C₁₋₆ alcohols, *e.g*. ethanol. When the solvate is water, the term "hydrate" may be used.

"Treating" and "treatment" of a disease include the following:
(1) inhibiting the disease, *i.e.* arresting or reducing the development of the disease or its clinical symptoms, and
(2) relieving the disease, *i.e.* causing regression of the disease or its clinical symptoms.

The term "effective amount" refers to an amount that may be effective to elicit the desired biological or medical response, including the amount of a compound that, when administered to a subject for treating a disease, is sufficient to effect such treatment for the disease. The effective amount will vary depending on the compound, the disease and its severity and the age, weight, *etc.* of the subject to be treated. The effective amount can include a range of amounts.

"Preventing" and "prevention" of a disease means preventing or reducing the risk of developing the disease, *i.e.* causing the clinical symptoms of the disease not to develop in a subject that may be exposed to or predisposed to the disease but does not yet experience or display symptoms of the disease.

The term "virologically suppressed" used with respect to a human patient having an HIV infection means that antiretroviral therapy (ART) has reduced the patient's HIV RNA level in serum viral load to less than 50 copies/mL or to a level below the limits of detection. Viral suppression does not mean that the person has been cured; if ART is discontinued, the person's HIV viral load will likely return to a detectable level.

The term "treatment experienced" used with respect to a human patient having an HIV infection means that the patient has previously taken one or more forms of HIV medication.

### EXAMPLES

The disclosure will now be illustrated by the following non-limiting examples.

### Example 1 - Comparative BIC 75 mg tablets

Comparative tablets comprising 75 mg bictegravir were manufactured having the following composition:

| **Component** | **Tablet C1** |
|---|---|
| | **BIC 75 mg (mg/tablet)** |
| Bictegravir Sodium | 78.69^{a} (26.23 %w/w) |
| Lactose Monohydrate | 75.81 (25.27 %w/w) |
| Microcrystalline Cellulose | 120.00 (40.00 % w/w) |
| Crospovidone | 21.00 (7.0 %w/w) |
| Sodium Stearyl Fumarate | 4.50 (1.50 %w/w) |
| Tablet Core Tablet | 300.0 (100 %w/w/) |

| **Film Coat** | |
|---|---|
| Opadry II Yellow 85F92259^{b} | 12.00 (4.00 %w/w)^{c} |
| Purified Water^{d} | - |

| | |
|---|---|
| ^{a}Corresponds to 75 mg of bictegravir free acid. ^{b}Opadry II Yellow 85F92259 contains 40.0% w/w Polyvinyl Alcohol (USP/Ph. Eur), 23.3% w/w Titanium Dioxide (USP/Ph. Eur), 20.2% w/w Polyethylene Glycol/Macrogol 3350 (USP/Ph. Eur), 14.8% w/w Talc (USP/Ph. Eur), 1.7% w/w Iron Oxide Yellow (NF). ^{c}Represents a theoretical weight gain of 4% (range 3 to 5%). ^{d}Purified water is used to film-coat the BIC tablets and is removed during the coating process. | |

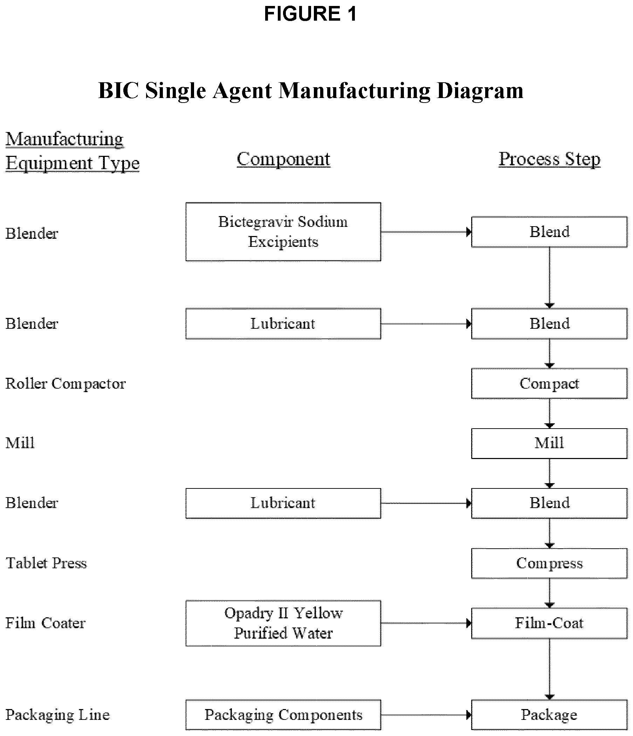

The bictegravir (BIC) tablets were manufactured through a series of unit operations as summarized in the manufacturing process flow diagram of Figure 1. First bictegravir was blended with microcrystalline cellulose, lactose monohydrate, and crospovidone. The mixture was blended with a portion of the sodium stearyl fumarate, roller compacted, and milled. The resulting granules were then blended with the remainder of the sodium stearyl fumarate. The final blend was compressed into tablet cores. The tablet cores were film-coated yellow and packaged.

### Example 2 - Comparative LEN 25 mg and 50 mg tablets

Comparative tablets comprising 25 mg and 50 mg lenacapavir were manufactured having the following composition:

| **Component** | **Composition (%w/w)** | **Tablet C2** | **Tablet C3** |
|---|---|---|---|
| | | **LEN 25 mg (mg/tablet)** | **LEN 50 mg (mg/tablet)** |
| Lenacapavir Sodium | 20.46 | 25.58^{a} | 51.15^{b} |
| Copovidone | 4.88 | 6.10 | 12.20 |
| Poloxamer 407 | 1.33 | 1.66 | 3.33 |
| Mannitol | 42.55 | 53.19 | 106.38 |
| Microcrystalline Cellulose | 21.28 | 26.60 | 53.20 |
| Croscarmellose Sodium | 8.00 | 10.00 | 20.00 |
| Magnesium Stearate | 1.50 | 1.88 | 3.75 |
| Tablet Core Tablet | 100 | 125.0 | 250.0 |

| **Film Coat** | | | |
|---|---|---|---|
| Opadry II Green 85F110187^{c} | 4.00 | 5.00^{d} | 10.00^{d} |
| Purified Water^{e} | - | - | - |

| | | | |
|---|---|---|---|
| ^{a}Corresponds to 25 mg of lenacapavir free acid. ^{b}Corresponds to 50 mg of lenacapavir free acid. ^{c}Opadry II Green 85F110187 contains 40.00% w/w polyvinyl alcohol (USP/PhEur), 20.86% w/w titanium dioxide (USP/PhEur), 20.20% w/w macrogol/PEG 3350 (NF/PhEur), 14.80% w/w talc (USP/PhEur), 3.32% w/w iron oxide yellow (NF), and 0.82% w/w black iron oxide (NF). ^{d}Represents a theoretical weight gain of 4% (range 3 to 5%). ^{e}Purified water is used to film-coat the LEN tablets and is removed during the coating process. | | | |

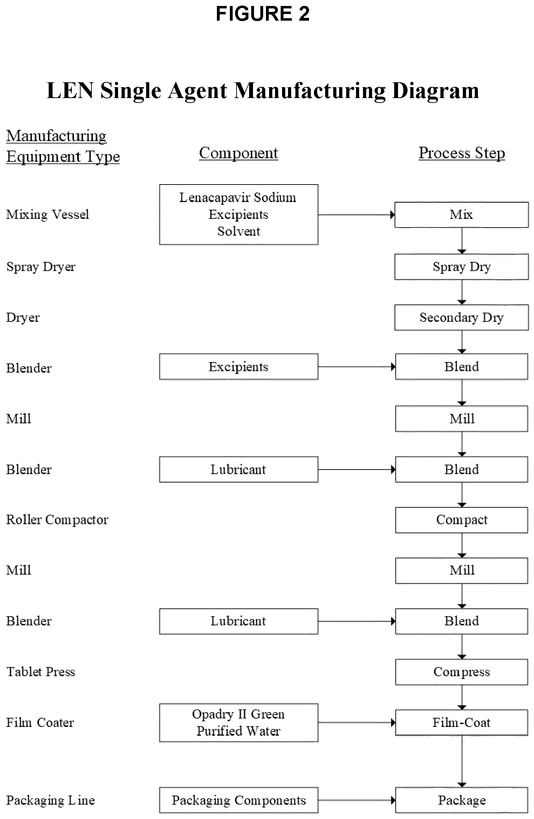

The lenacapavir (LEN) tablets were manufactured through a series of unit operations as summarized in the manufacturing process flow diagram of Figure 2. First lenacapavir sodium and excipients in solution were spray dried and then secondary dried to form lenacapavir spray-dried dispersion. Lenacapavir spray-dried dispersion was blended with intragranular excipients, then milled/dispersed. The mixture was blended with a portion of the lubricant, roller compacted, and milled. The resulting granules were then blended with the remainder of the lubricant. The final blend was compressed into tablet cores. The tablet cores were film-coated green and packaged.

### Example 3 - Monolayer BIC/LEN tablets (75/25 mg, 75/50 mg, 75/75 mg, and 75/100 mg)

Monolayer tablets comprising bictegravir and lenacapavir were manufactured for comparison with the combination of tablets containing the single agents. The composition of the bictegravir/lenacapavir 75/25 mg, 75/50 mg, 75/75 mg, and 75/100 mg monolayer fixed-dose combination tablet formulations evaluated were:

| **Component** | **Tablet M1** | **Tablet M2** | **Tablet M3** | **Tablet M4** |
|---|---|---|---|---|
| | **BIC/LEN 75/25 mg (mg/tablet)** | **BIC/LEN 75/50 mg (mg/tablet)** | **BIC/LEN 75/75 mg (mg/tablet)** | **BIC/LEN 75/100 mg (mg/tablet)** |
| **Tablet Core** | | | | |
| Bictegravir Sodium | 78.56^{a} (18.10 %w/w) | 78.56^{a} (18.10 %w/w) | 78.56^{a} (18.10 %w/w) | 78.56^{a} (18.10 %w/w) |
| Lenacapavir Sodium | 25.56^{b} (5.89 %w/w) | 51.12^{c} (11.78 %w/w) | 76.68^{d} (17.67 %w/w) | 102.24^{e} (23.56 %w/w) |
| Copovidone^{c,d} | 6.12 (1.41 %w/w) | 12.24 (2.82 %w/w) | 18.36 (4.23 %w/w) | 24.48 (5.64 %w/w) |
| Poloxamer 407^{c,d} | 1.65 (0.38 %w/w) | 3.30 (0.76 %w/w) | 4.95 (1.14 %w/w) | 6.60 (1.52 (%w/w) |
| Mannitol | 187.27 (43.15 %w/w) | 165.05 (38.03 %w/w) | 142.83 (32.91%w/w) | 120.61 (27.79 %w/w) |
| Microcrystalline Cellulose | 93.61 (21.57 % w/w) | 82.50 (19.01 %w/w) | 71.39 (16.45 %w/w) | 60.28 (13.89 %w/w) |
| Croscarmellose Sodium | 34.72 (8.0 %w/w) | 34.72 (8.0 %w/w) | 34.72 (8.00 %w/w) | 34.72 (8.0 %w/w) |
| Magnesium Stearate | 6.51 (1.50 %w/w) | 6.51 (1.50 %w/w) | 6.51 (1.50 %w/w) | 6.51 (1.50 %w/w) |
| Tablet Core Tablet | 434.0 (100 %w/w/) | 434.0 (100 %w/w) | 434.0 (100 %w/w) | 434.0 (100 %w/w) |

| **Film-Coating** | | | | |
|---|---|---|---|---|
| Opadry II | 17.36^{f} (4.00 %w/w)^{h} | 17.36^{g} (4.00 %w/w)^{h} | 17.36^{g} (4.00 %w/w)^{h} | 17.36^{g} (4.00 %w/w)^{h} |
| Purified Waterⁱ | - | - | - | - |

| | | | | |
|---|---|---|---|---|
| ^{a}Corresponds to 75 mg of bictegravir free acid. ^{b}Corresponds to 25 mg of lenacapavir free acid. ^{a}Corresponds to 50 mg of lenacapavir free acid. ^{d}Corresponds to 75 mg of lenacapavir free acid.^{e}Corresponds to 100 mg of lenacapavir free acid. ^{f}Opadry II Brown 85F86937 is used. Opadry II Brown 85F86937 contains 40.0% w/w Polyvinyl Alcohol (USP/Ph. Eur), 20.2% w/w Polyethylene Glycol/Macrogol 3350 (USP/Ph. Eur), 19.0% w/w Titanium Dioxide (USP/Ph. Eur), 14.8% w/w Talc (USP/Ph. Eur), 6.0% w/w Iron Oxide Red (NF). ^{g}Opadry II Yellow 85F520107 is used. Opadry II Yellow 85F520107 contains 40.0% w/w Polyvinyl Alcohol (USP/Ph. Eur), 22.6% w/w Titanium Dioxide (USP/Ph. Eur), 20.2% w/w Polyethylene Glycol/Macrogol 3350 (USP/Ph. Eur), 14.8% w/w Talc (USP/Ph. Eur), 2.4% w/w Iron Oxide Yellow (NF). ^{h}Represents a theoretical weight gain of 4% (range 2 to 6%). ⁱPurified water is used to film-coat the BIC/LEN tablets and is removed during the coating process. | | | | |

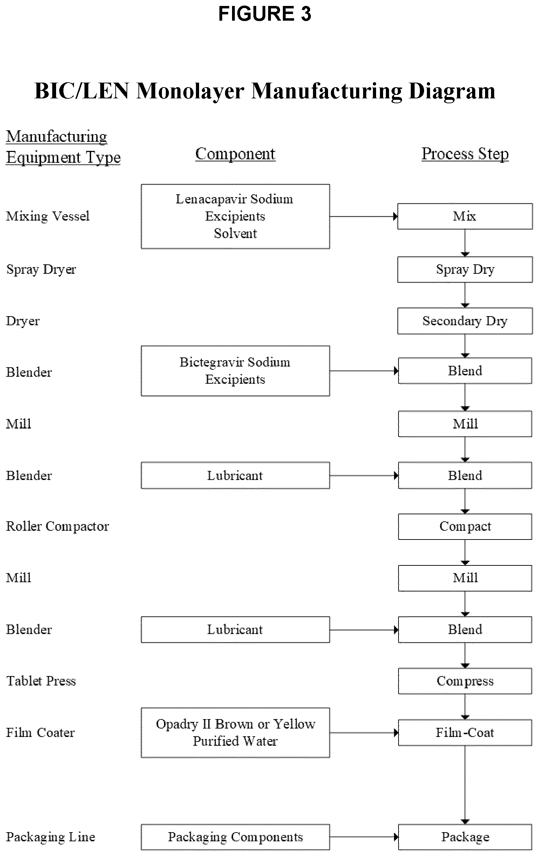

The bictegravir/lenacapavir (BIC/LEN) monolayer tablets were manufactured through a series of unit operations as summarized in the manufacturing process flow diagram of Figure 3. First lenacapavir sodium and excipients in solution were spray dried and then secondary dried to form lenacapavir spray-dried dispersion. Bictegravir sodium and lenacapavir spray-dried dispersion was blended with intragranular excipients, then milled/dispersed. The mixture was blended with a portion of the lubricant, roller compacted, and milled. The resulting granules were then blended with the remainder of the lubricant. The final blend was compressed into tablet cores. The tablet cores were film-coated brown or yellow and packaged.

### Example 4 - Bilayer BIC/LEN tablets (75/25 mg and 75/50 mg)

Bilayer tablets comprising a first layer comprising bictegravir and a second layer comprising lenacapavir were manufactured for comparison with the combination of tablets containing the single agents. The composition of the bictegravir/lenacapavir 75/25 mg and 75/50 mg bilayer fixed-dose combination tablet formulations evaluated were:

| **Component** | **Tablet B1** | **Tablet B2** |
|---|---|---|
| | **BIC/LEN 75/25 mg (mg/tablet)** | **BIC/LEN 75/50 mg (mg/tablet)** |
| ***BIC layer*** | | |
| Bictegravir Sodium | 78.54^{b} (18.48 %w/w) | 78.54^{b} (14.28 %w/w) |
| Microcrystalline Cellulose | 192.96 (45.40 % w/w) | 192.96 (35.08 %w/w) |
| Croscarmellose Sodium | 24.00 (5.65 %w/w) | 24.00 (4.36 %w/w) |
| Magnesium Stearate | 4.50 (1.06 %w/w) | 4.50 (0.82 %w/w) |
| **Subtotal** | 300.0 (70.59 %w/w) | 300.0 (54.54 %w/w) |

| ***LEN layer*** | | |
|---|---|---|
| Lenacapavir Sodium | 25.57^{e} (6.02 %w/w) | 51.14^{f} (9.30 %w/w) |
| Copovidone | 6.10 (1.44 %w/w) | 12.20 (2.22 %w/w) |
| Poloxamer 407 | 1.67 (0.39 %w/w) | 3.33 (0.61 %w/w) |
| Mannitol | 53.19 (12.51 %w/w) | 106.38 (19.34 %w/w) |
| Microcrystalline Cellulose | 26.60 (6.26 % w/w) | 53.20 (9.67 %w/w) |
| Croscarmellose Sodium | 10.00 (2.35 %w/w) | 20.00 (3.64 %w/w) |
| Magnesium Stearate | 1.88 (0.44 %w/w) | 3.75 (0.68 %w/w) |
| **Subtotal** | 125.0 (29.41 %w/w) | 250.0 (45.45 %w/w) |
| Tablet Core Total | 425.0 (100 %w/w) | 550.0 (100 %w/w) |

| ***Film-Coating*** | | |
|---|---|---|
| Opadry II Yellow 85F520107^{d} | 17.00 (4.00 %w/w)^{e} | 22.00 (4.00 %w/w)^{e} |
| Purified Water^{f} | - | - |

| | | |
|---|---|---|
| ^{a}Corresponds to 75 mg of bictegravir free acid. ^{b}Corresponds to 25 mg of lenacapavir free acid. ^{c}Corresponds to 50 mg of lenacapavir free acid. ^{d}Opadry II Yellow 85F520107 contains 40.0% w/w Polyvinyl Alcohol (USP/Ph. Eur), 22.6% w/w Titanium Dioxide (USP/Ph. Eur), 20.2% w/w Polyethylene Glycol/Macrogol 3350 (USP/Ph. Eur), 14.8% w/w Talc (USP/Ph. Eur), 2.4% w/w Iron Oxide Yellow (NF). ^{e}Represents a theoretical weight gain of 4% (range 2 to 6%). ^{f}Purified water is used to film-coat the BIC/LEN tablets and is removed during the coating process. | | |

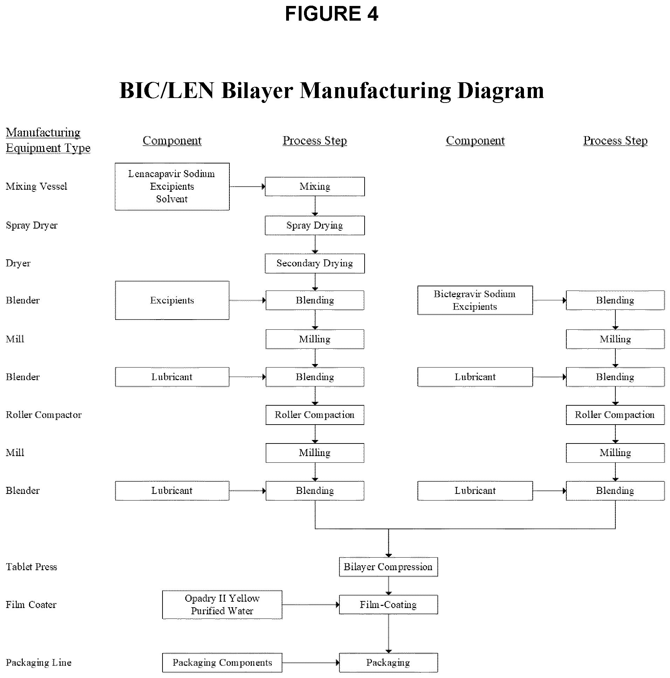

The bictegravir/lenacapavir (BIC/LEN) bilayer tablets were manufactured through a series of unit operations as summarized in the manufacturing process flow diagram of Figure 4.

First lenacapavir sodium and excipients in solution were spray dried and then secondary dried to form lenacapavir spray-dried dispersion. Lenacapavir spray-dried dispersion was blended with intragranular excipients, then milled/dispersed. The mixture was blended with a portion of the lubricant, roller compacted, and milled. The resulting granules were then blended with the remainder of the lubricant to yield the LEN final blend for compression.

Bictegravir sodium was blended with intragranular excipients, then milled/dispersed. The mixture was blended with a portion of the lubricant, roller compacted, and milled. The resulting granules were then blended with the remainder of the lubricant to yield the BIC final blend for compression.

BIC and LEN final blends were compressed into bilayer tablets to yield the BIC/LEN tablet cores, which were film-coated yellow and packaged.

### Example 5 - BIC Non-Sink Dissolution (FaSSIF)

The dissolution of bictegravir in biorelevant media was determined as a function of time. A single tablet was placed in 250 mL of 3.75 mM Fasted-State Simulated Intestinal Fluid (FaSSIF) pH 6.5 (3 mM taurocholate, 0.75 mM lecithin, 0.10 M NaCl) pre-heated to 37 °C and stirring at 75 rpm. Samples (10 mL) were taken at pre-determined timepoints of 0, 5, 10, 15, 20, 30, 45, 60, and 120 minutes. The withdrawn media was immediately replaced with 10 mL of fresh media. Each sample was filtered through a 0.22 µm PES filter (Millex - GP Fast Flow & Low Binding Millipore Express), discarding the first ~8 mL of filtrate. The remaining filtrate was transferred to an amber vial for direct analysis using an appropriate UPLC-UV method.

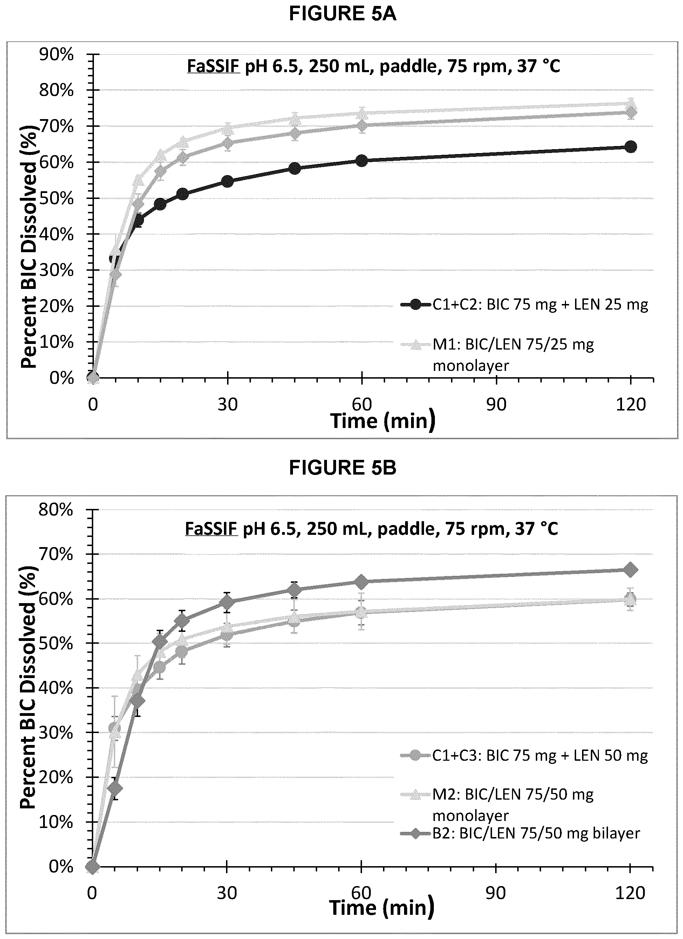

The dissolution of bictegravir from 75/25 mg BIC/LEN monolayer (M1) and bilayer (B1) tablets was compared to the dissolution of bictegravir from the combination of the comparative single agent tablets of 75 mg BIC (C1) and 25 mg LEN (C2). The results are shown in Figure 5A and demonstrate that the dissolution of bictegravir from the bilayer and monolayer tablets was faster than from the comparative single agent tablets.

The dissolution of bictegravir from 75/50 mg BIC/LEN monolayer (M2) and bilayer (B2) tablets was compared to the dissolution of bictegravir from the combination of the comparative single agent tablets of 75 mg BIC (C1) and 50 mg LEN (C3). The results are shown in Figure 5B.

### Example 6 - LEN Non-Sink Dissolution (Cremophor)

The dissolution of lenacapavir in relevant media was determined as a function of time. Multiple tablets (number of tablets determined such that 300 mg of lenacapavir was added to the system) were placed in 900 mL of 2% w/w Cremophor EL in 30 mM potassium phosphate pH 6.0 pre-heated to 37 °C and stirring at 75 rpm. Samples (10 mL) were taken at pre-determined timepoints of 0, 10, 15, 20, 25, 30, 45, 60, and 120 minutes. Each sample was filtered through a 0.22 µm PES filter (Millex - GP Fast Flow & Low Binding Millipore Express), discarding the first ~8 mL of filtrate. The remaining filtrate was transferred to an amber vial for direct analysis using an appropriate UPLC-UV method.

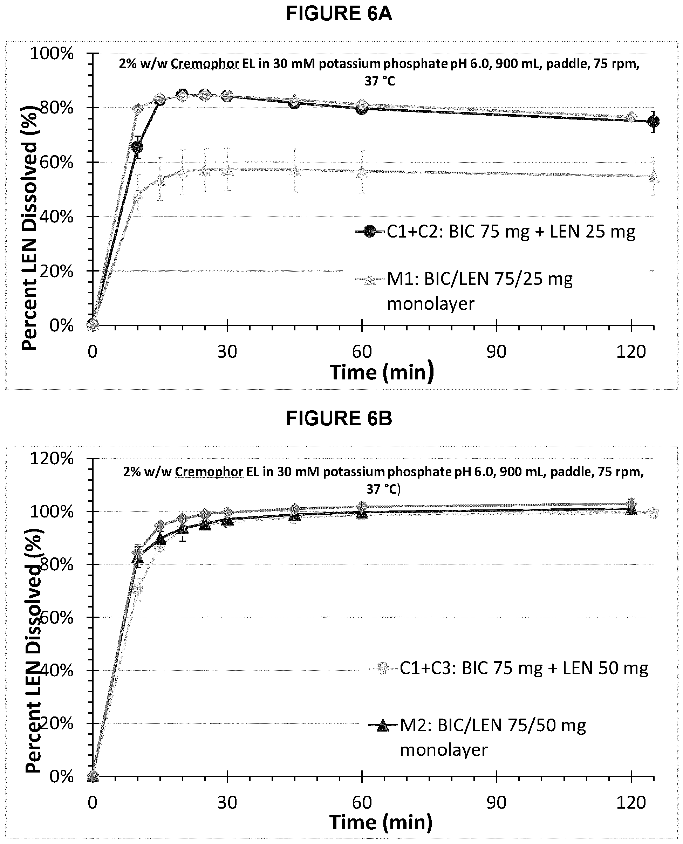

The dissolution of lenacapavir from 75/25 mg BIC/LEN monolayer (M1) and bilayer (B1) tablets was compared to the dissolution of lenacapavir from the combination of comparative single agent tablets of 75 mg BIC (C1) and 25 mg LEN (C2). The results are shown in Figure 6A and demonstrate that the dissolution of lenacapavir from the monolayer tablet was significantly reduced relative to that of the comparative single agent tablets, whilst the dissolution of lenacapavir from the bilayer tablet was similar to the comparative single agent tablets.

The dissolution of lenacapavir from 75/50 mg BIC/LEN monolayer (M2) and bilayer (B2) tablets was compared to the dissolution of lenacapavir from the combination of comparative single agent tablets of 75 mg BIC (C1) and 50 mg LEN (C3). The results are shown in Figure 6B and demonstrate that the dissolution of lenacapavir from the bilayer and monolayer tablets was slightly faster than from the comparative single agent tablets.

These results demonstrate that LEN release from 75/25 mg BIC/LEN monolayer tablets is significantly reduced relative to the combination of 75 mg BIC and 25 mg LEN single agent tablets. However, increasing the amount of lenacapavir from 25 mg to 50 mg in LEN/BIC monolayer tablets that contain 75 mg bictegravir provides comparable LEN dissolution to that observed for comparative 75 mg BIC and 50 mg LEN single agent tablets.

### Example 7 - Pre-Clinical PK in Dogs

The PK properties of bictegravir and lenacapavir in monolayer and bilayer tablets of the disclosure was compared to that arising from the combination of tablets comprising the single agents using the method below.

Each dosing group consisted of 6 male, non-naive purebred beagle dogs. At dosing, the animals weighed between 6 to 13 kg. The animals were fasted overnight prior to dose administration and up to 4 hr after dosing. Each subject was pre-treated with pentagastrin (6 µg/kg) and dosed 30 minutes later with a tablet(s) containing 75 mg of bictegravir and 25 or 50 mg of lenacapavir. Each subject was given 5 mL of water to aid in swallowing followed by an additional 25 mL of water for a total of 30 mL water administered with each dose.

Serial venous blood samples (approximately 1 mL each) were taken from each animal at 0, 0.25, 0.50, 1.0, 2.0, 4.0, 6.0, 8.0, 12.0, 24.0, 48.0, 72.0, 96.0, and 120.0 hours after dosing. The blood samples were collected into Vacutainer^{™} tubes containing K2EDTA as the anticoagulant and were immediately placed on wet ice pending centrifugation for plasma. An LC/MS/MS method was used to measure the concentration of the test compound in plasma. An aliquot of 20 µL of each plasma sample was added to a clean 96 well plate, and 120 µL of 50 nM GS 833737 and 100 ng/mL Carbamazeprine in acetonitrile (ACN) was added. After protein precipitation, an aliquot of 100 µL of the supernatant was transferred to a clean 96-well plate and diluted with 100 µL of water. An aliquot of 0.5 - 1.5 µL of the above solution was injected into a Sciex API-6500+ LC/MS/MS system. A Waters Acquity UPLC system with sample organizer was used for sample injection, elution, and separation. An API-6500+ triple quadrupole mass spectrometer was utilized in multiple reaction monitoring mode (Sciex, Framingham, MA).

For analysis of bictegravir concentration, the system utilized a Waters HSS T3 UPLC column (50 × 2.1 mm, 2.5 µm). Liquid chromatography was performed using two mobile phases: mobile phase A contained 100/0.1 v/v% water/formic acid and mobile phase B contained 100/0.1 v/v% acetonitrile/formic acid.

For analysis of lenacapavir concentration, the system utilized a Waters BEH C8 UPLC column (50 × 2.1 mm, 1.7 µm). Liquid chromatography was performed using two mobile phases: mobile phase A contained 50/25/25/0.1 v/v% water/acetonitrile/methanol/formic acid and mobile phase B contained 50/50/0.1 v/v% acetonitrile/methanol/formic acid.

Non-compartmental pharmacokinetic analysis was performed on the plasma concentration-time data. F(%) refers to oral bioavailability; AUCₗₐₛₜ refers to area under the curve from time zero to the last quantifiable concentration and is a measure of total plasma exposure of the indicated compound; Cₘₐₓ refers to the peak plasma concentration of the compound after administration.

The PK properties of bictegravir in dogs from the evaluated tablets are shown in the table below:

| **BIC pre-clinical PK in fasted, pentagastrin pre-treated dogs** | | | | | | | | |
|---|---|---|---|---|---|---|---|---|
| **PK paramet er** | **C1 + C2** (BI C 75 mg + LE N 25 mg) | **C1 + C3** (BI C 75 mg + LE N 50 mg) | **M1** (BIC/LE N 75/25 mg) | **M2** (BIC/LE N 75/50 mg) | **M3** (BIC/LE N 75/75 mg) | **M4** (BIC/LE N 75/100 mg) | **B1** (BIC/LE N 75/25 mg) | **B2** (BIC/LE N 75/50 mg) |
| **AUCₗₐₛₜ (µM*hr)** | 128 ± 59 | 149 ± 87 | 160 ± 26 | 122 ± 79 | 116 ± 50 | 89 ± 45 | 172 ± 75 | 168 ± 27 |
| **AUC_{inf} (µM*hr)** | 128 ± 59 | 149 ± 87 | 160 ± 26 | 122 ± 79 | 116 ± 50 | 89 ± 45 | 172 ± 75 | 168 ± 27 |
| **Cₘₐₓ (µM)** | 24 ± 5 | 24 ± 8 | 26 ±3 | 21 ±6 | 19 ±3 | 14 ± 5 | 28 ± 5 | 27 ± 5 |
| **Tₘₐₓ (hr)** | 1.3 ± 0.5 | 1.5 ± 0.6 | 1.5 ± 0.6 | 1.3 ± 0.5 | 1.8 ± 0.4 | 2.3 ± 0.8 | 1.7 ± 0.6 | 1.7 ± 0.5 |
| **T_{1/2} (hr)** | 17 ± 10 | 21 ± 7 | 16 ± 7 | 15 ± 7 | 14 ± 7 | 10 ± 8 | 26 ± 10 | 23 ± 5 |
| **F (%)** | 23 ± 10 | 26 ± 15 | 29 ± 7 | 21 ± 13 | 20 ± 10 | 15 ± 7 | 35 ± 18 | 33 ± 8 |

The PK properties of lenacapavir in dogs from the evaluated tablets are shown in the table below:

| **LEN pre-clinical PK in fasted, pentagastrin pre-treated dogs** | | | | | | | | |
|---|---|---|---|---|---|---|---|---|
| **PK paramet er** | **C1 + C2** (BIC 75 mg + LEN 25 mg) | **C1 + C3** (BIC 75 mg + LEN 50 mg) | **M1** (BIC/LE N 75/25 mg) | **M2** (BIC/LE N 75/50 mg) | **M3** (BIC/LE N 75/75 mg) | **M4** (BIC/LE N 75/100 mg) | **B1** (BIC/LE N 75/25 mg) | **B2** (BIC/LE N 75/50 mg) |
| **AUCₗₐₛₜ (µM*hr)** | 1.0 ± 0.2 | 2.0 ± 0.8 | 0.6 ± 0.3 | 3.2 ± 0.9 | 5.0 ± 2.3 | 4.3 ± 1.1 | 2.5 ± 1.8 | 2.7 ± 1.2 |
| **AUC_{inf} (µM*hr)** | 1.1 ± 0.3 | 2.0 ± 0.8 | 0.7 ± 0.3 | 3.3 ± 0.8 | 5.1 ± 2.3 | 4.4 ± 1.1 | 2.8 ± 2.3 | 2.9 ± 1.5 |
| **Cₘₐₓ (µM)** | 0.06 ± 0.02 | 0.13 ± 0.05 | 0.04 ± 0.02 | 0.18 ± 0.07 | 0.29 ± 0.14 | 0.27 ± 0.11 | 0.10 ± 0.06 | 0.12 ± 0.06 |
| **Tₘₐₓ (hr)** | 5 ± 2 | 4 ± 1 | 5 ± 1 | 4 ± 0 | 4 ± 0 | 4 ± 1 | 5 ± 1 | 4 ± 0 |
| **T_{1/2} (hr)** | 18 ± 6 | 23 ± 10 | 19 ± 14 | 24 ± 9 | 23 ± 5 | 21 ± 8 | 22 ± 4 | 29 ± 19 |
| **F (%)** | 2 ± 1 | 2 ± 1 | 1 ± 1 | 3 ± 1 | 3 ± 2 | 2 ± 0 | 7 ± 7 | 3 ± 1 |

### Example 8 - Clinical PK in human subjects

The PK properties in human subjects of bictegravir and lenacapavir in monolayer tablets of the disclosure was compared to that arising from the combination of tablets comprising the individual agents using the method below.

The study was done by a Phase-1, open-label, multicenter, single-dose, multiple-cohort, parallel study in healthy participants to evaluate the relative bioavailability of BIC/LEN fixed-dose combination tablets. Following completion of screening and assessments, eligible participants were administered study drug(s) on Day 1 as described below:
**Cohort 1:** Single doses of 1 ×C1 (BIC 75 mg tablet) and 1 ×C2 (LEN 25 mg tablet) administered simultaneous, orally under fasted conditions.
**Cohort 2:** Single dose of 1 ×M1 (BIC/LEN 75/25 mg monolayer tablet) administered orally under fasted conditions.
**Cohort 5:** Single dose of 1 ×M2 (BIC/LEN 75/50 mg monolayer tablet) administered orally under fasted conditions.
**Cohort 6**: Single dose of 1 ×M4 (BIC/LEN 75/100 mg monolayer tablet) administered orally under fasted conditions.

All study treatments were administered orally at the study site in the morning at approximately the same time each day with 240 mL of water. The study drug(s) were administered in the morning following an overnight fast (no food or drink, except water, for at least 10 hours). Participants were restricted from food intake until after collection of the 4-hour postdose blood draw. Additionally, participants were restricted from water consumption 1 hour before and until 2 hours after dosing, except for the 240 mL of water given with the study treatment(s). A meal (standardized lunch) was provided to the participants after the 4-hour postdose blood draw.

Eligible participants were confined to the study centre beginning Day -1 until the completion of assessments on Day 8.

For each cohort, plasma concentrations and relevant PK parameters of BIC and LEN were listed and summarized using descriptive statistics (as applicable). Two-sided 90% CIs were calculated for the ratios of geometric least-squares means (GLSMs) between test and reference treatments being compared.

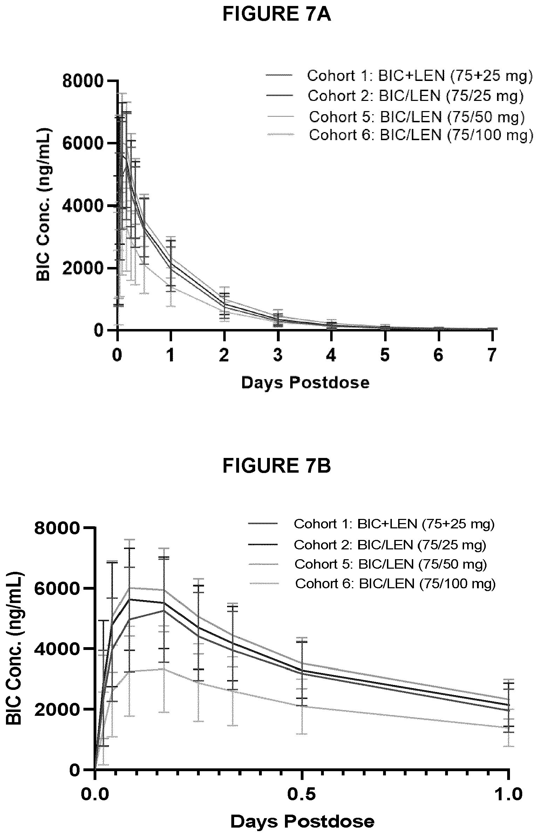

The PK properties of bictegravir from the evaluated tablets are shown in the table below and in Figures 7A and 7B:

| **Bictegravir clinical PK data** | | | | | | | |
|---|---|---|---|---|---|---|---|
| | **Cohort 1** | **Cohort 2** | **Cohort 2 vs Cohort 1 %GLS M (90%Cl )** | **Cohort 5** | **Cohort 5 vs Cohort 1 %GLS M (90%Cl )** | **Cohort 6** | **Cohort 6 vs Cohort 1 %GLS M (90%Cl )** |
| **PK paramete r*** | **C1 + C2 (BIC 75 mg + LEN 25 mg) (N=60)** | **M1 (BIC/LE N 75/25 mg) (N=61)** | | **M2 (BIC/LE N 75/50 mg) (N=60)** | | **M4 (BIC/LE N 75/100 mg) (N=60)** | |
| **Cₘₐₓ (ng/mL)** | 5790 (29.0) | 6260 (25.2) | 110 (100, 122) | 6580 (22.0) | 117 (106, 129) | 3850 (40.3) | 64.7 (58.7, 71.4) |
| **AUC_{0-Day8} (h*ng/m L)** | 129000 (34.5) | 142000 (31.4) | 113 (101, 127) | 159000 (27.4) | 127 (113, 143) | 92400 (44.4) | 69.8 (62.2, 78.3) |
| **AUC_{inf} (h*ng/m L)** | 129000 (34.3) | 142000 (31.5) | 113 (101, 127) | 159000 (27.6) | 127 (113, 143) | 92900 (44.5) | 69.9 (62.3, 78.4) |
| **%AUCₑₓₚ** | 0.887 (86.6) | 1.03 (242) | - | 0.775 (39.5) | - | 1.23 (59.8) | - |
| **Tₘₐₓ (h)** | 2.00 (2.00, 4.00) | 2.00 (1.50, 4.00) | - | 2.00 (1.25, 4.00) | - | 2.00 (1.00, 4.00) | - |
| **T_{1/2} (h)** | 18.3 (15.6, 21.3) | 19.7 (17.2, 21.6) | - | 21.0 (18.3, 24.7) | - | 19.6 (17.9, 22.4) | - |
| **CL/F (mL/h)** | 698 (68.3) | 579 (31.6) | - | 514 (33.6) | - | 1010 (57.0) | - |
| **Vz/F (mL)** | 18400 (61.3) | 16000 (30.3) | - | 15000 (29.8) | - | 28500 (47.2) | - |

| | | | | | | | |
|---|---|---|---|---|---|---|---|
| *Pharmacokinetic parameters are presented as mean (%CV) except Tₘₐₓ and T_{1/2}, which are presented as median (Quartile 1, Quartile 3), and shown to 3 significant digits. | | | | | | | |

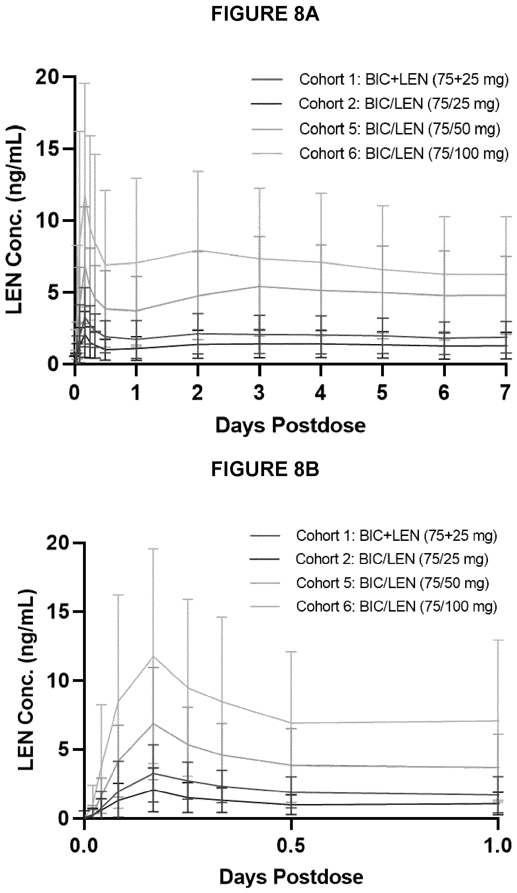

The PK properties of lenacapavir from the evaluated tablets are shown in the table below and in Figures 8A and 8B:

| **Lenacapavir clinical PK data** | | | | | | | |
|---|---|---|---|---|---|---|---|
| | **Cohort 1** | **Cohort 2** | **Cohort 2 vs Cohort 1 %GLSM (90%Cl)** | **Cohort 5** | **Cohort 5 vs Cohort 1 %GLSM (90%Cl)** | **Cohort 6** | **Cohort 6 vs Cohort 1 (%GLS M (90%Cl)** |
| **PK paramete r*** | **C1 + C2** (BIC 75 mg + LEN 25 mg) **(N=60)** | **M1** (BIC/LE N 75/25 mg) **(N=61)** | | **M2** (BIC/LE N 75/50 mg) **(N=60)** | | **M4** (BIC/LE N 75/100 mg) **(N=60)** | |
| **Cₘₐₓ (ng/mL)** | 3.66 (57.7) | 2.21 (73.2) | 51.3 (42.1, 62.5) | 7.39 (61.4) | 200 (164, 244) | 12.7 (67.9) | 328 (268, 400) |
| **AUC0-Day8 (h*ng/m L)** | 329 (62.0) | 217 (69.1) | 59.2 (49.1, 71.5) | 800 (60.4) | 241 (200, 291) | 1180 (67.3) | 339 (281, 410) |
| **Tₘₐₓ (h)** | 4.00 (4.00, 6.00) | 4.00 (4.00, 48.00) | | 4.00 (4.00, 4.00) | | 4.00 (4.00, 6.00) | |

| | | | | | | | |
|---|---|---|---|---|---|---|---|
| *Pharmacokinetic parameters are presented as mean (%CV) except Tₘₐₓ, which is presented as median (Quartile 1, Quartile 3), and shown to 3 significant digits. | | | | | | | |

The results demonstrate that the Cₘₐₓ, AUC_{inf} and AUC_{0-Day8} of bictegravir from the 75/100 mg BIC/LEN monolayer tablet (M4) was reduced relative to the combination of 75 mg BIC + 25 mg LEN single agent tablets (C1+C2).

Additionally, the results demonstrate that AUC_{0-Day8} of lenacapavir from the 75/25 mg BIC/LEN monolayer tablet (M1) was reduced relative to the co-administration of 75 mg BIC + 25 mg LEN (C1+C2). In contrast, the AUC_{0-Day8} of lenacapavir from the 75/50 mg BIC/LEN monolayer tablet (M2) was superior (about 2- to 2.4-fold higher) relative to administration of 25 mg LEN as a single agent (C2).

### Example 9 - Summary of the Pre-Clinical and Clinical Data

A summary of the pre-clinical and clinical data discussed in the examples above is provided in the following table:

| **Formula tion** | **Dose Strengt h** | **Compar ator** | **BIC** | | | **LEN** | | |
|---|---|---|---|---|---|---|---|---|
| | | | **Dissolut ion** (% dissolve d, relative) | **Dog PK (at HED)** (% exposu re, relativ e) | **Huma n PK** (AUC₀ -Day 8 % exposu re, relativ e; 90% CI) | **Dissolut ion** (% dissolve d, relative) | **Dog PK (at HED)** (% exposu re, relativ e) | **Huma n PK** (AUC₀ -Day 8 % exposu re, relativ e; 90% CI) |
| **Monolay er** | **M1 (BIC/L EN 75/25 mg)** | **C1+C2 (BIC 75 mg** + **LEN 25 mg)** | ∼120% | 125 ± 61 % | 113% (101, 126) | ∼70% | 60 ± 32 % | 59.2% (49.1, 71.5) |
| | **M2 (BIC/L EN 75/50 mg)** | **C1+C2 (BIC 75 mg + LEN 25 mg)** | Match | 95 ± 76 % | 127% (113, 143) | ∼170% | 320 ± 110 % | 241% (200, 291) |
| | **M2 (BIC/L EN 75/50 mg)** | **C1+C3 (BIC 75 mg + LEN 50 mg)** | Match | 82 ± 71 % | | Match | 160 ± 78 % | 121% (99.8, 146)^{a} |
| | **M3 (BIC/L EN 75/75 mg)** | **C1+C3 (BIC 75 mg + LEN 50 mg)** | ∼70% | 78 ± 57% | - | ∼150% | 250 ± 152% | - |
| | **M4 (BIC/L EN 75/100 mg)** | **C1+C2 (BIC 75 mg + LEN 25 mg)** | ∼50% | 70 ± 47% | 69.8% (62.2, 78.3) | - | 430 ± 139% | 339% (281, 410) |
| | **M4 (BIC/L EN 75/100 mg)** | **C1+C3 (BIC 75 mg + LEN 50 mg)** | ∼50% | 60 ± 46% | - | ∼180% | 215 ± 152% | 170% (140, 205)^{a} |
| **Bilayer** | **B1 (BIC/L EN 75/25 mg)** | **C1+C2 (BIC 75 mg + LEN 25 mg)** | ∼120% | 134 ± 85 % | - | Match | 250 ± 187 % | - |
| | **B2 (BIC/L EN 75/50 mg)** | **C1+C3 (BIC 75 mg + LEN 50 mg)** | ∼120% | 113 ± 60% | - | Match | 135 ± 81% | - |

| | | | | | | | | |
|---|---|---|---|---|---|---|---|---|
| ^{a}Dose-normalized for comparison, assumes linearity | | | | | | | | |

### Example 10 - Evaluation of filler effect on manufacturability of BIC layer formulation

The impact of the choice of filler in the 75 mg bictegravir layer of the bilayer BIC/LEN bilayer tablet formulation was evaluated. Bilayer tablets comprising 75/25 mg BIC/LEN and 75/50 mg BIC/LEN were manufactured in which the bictegravir layer composition was varied as summarized in the table below. The lenacapavir layer compositions were manufactured having the same composition as C2 (for the 75/25 mg BIC/LEN tablets) and C3 (for the 75/50 mg BIC/LEN tablets).

| **Component** | **K1 (2:1 Mannito l:MCC)** | **K2 (1:1 Mannitol :MCC)** | **K3 (1:2 Mannitol: MCC)** | **K4 (100% IG^{d} MCC)** | **K5 (100% IG^{d} MCC + 0.5% EG Mg St)** | **K6 (IG^{d} + EG^{d} MCC)** |
|---|---|---|---|---|---|---|
| ***Intragranular*** | ***Composition (%w*/*w)*** | | | | | |
| Bictegravir Sodium | 26.18 | 26.18 | 26.18 | 26.18 | 26.18 | 26.18 |
| Mannitol (Pearlitol 160C)^{a} | 42.88 | 32.16 | 21.44 | - | - | - |
| Microcrystalli ne Cellulose (Avicel PH101)^{a} | 21.44 | 32.16 | 42.88 | 64.32 | 64.00 | 54.32 |
| Croscarmellos e Sodium | 8.00 | 8.00 | 8.00 | 8.00 | 7.96 | 8.00 |
| (AC-DI-SOL)b | | | | | | |
| Magnesium Stearate (Hyqual Code 5712)^{c} | 0.50 | 0.50 | 0.50 | 0.50 | 0.50 | 0.50 |

| ***Extragranular*** | | | | | | |
|---|---|---|---|---|---|---|
| Microcrystalli ne Cellulose (Avicel PH101)^{a} | - | - | - | - | - | 10.00 |
| Magnesium Stearate (Hyqual Code 5712)^{c} | 1.00 | 1.00 | 1.00 | 1.00 | 1.50 | 1.00 |
| Total Layer Weight (mg) | 300 mg | 300 mg | 300 mg | 300 mg | 300 mg | 300 mg |

| | | | | | | |
|---|---|---|---|---|---|---|
| ^{a}Filler ^{b}Disintegrant ^{c}Lubricant ^{d}IG = Intragranular; EG = Extragranular ^{e}Opadry II Yellow 85F520107 contains 40.0% w/w Polyvinyl Alcohol (USP/Ph. Eur), 22.6% w/w Titanium Dioxide (USP/Ph. Eur), 20.2% w/w Polyethylene Glycol/Macrogol 3350 (USP/Ph. Eur), 14.8% w/w Talc (USP/Ph. Eur), 2.4% w/w Iron Oxide Yellow (NF). ^{f} Purified water is used to film-coat the BIC/LEN tablets and is removed during the coating process. | | | | | | |

First lenacapavir sodium and excipients in solution were spray dried and then secondary dried to form lenacapavir spray-dried dispersion. Lenacapavir spray-dried dispersion was blended with intragranular excipients, then milled/dispersed. The mixture was blended with a portion of the lubricant, roller compacted, and milled. The resulting granules were then blended with the remainder of the lubricant to yield the LEN final blend for compression.

Bictegravir sodium was blended with intragranular excipients, then milled/dispersed. The mixture was blended with a portion of the lubricant, roller compacted, and milled. The resulting granules were then blended with the remainder of the lubricant to yield the BIC final blend for compression.

BIC and LEN final blends were compressed into bilayer tablets to yield the BIC/LEN tablet cores.

### Manufacturing parameters

The resulting BIC layers were evaluated across a number of manufacturing parameters, the results of which are shown in the table below:

| **BIC Layer Formulation^{a}** | **Punch Sticking or Filming^{b}** | **Ejection Forces (N)^{c}** | **Delamination in Linear Region^{d}** | **Blend properties^{e}** |
|---|---|---|---|---|
| **K1 (2:1 Mannitol:MCC)** | Major Sticking | 500-1500 | Minimal | Acceptable |
| **K2 (1:1 Mannitol:MCC)** | Major Sticking | <800 | Minimal | Acceptable |
| **K3 (1:2 Mannitol:MCC)** | Major Filming | <500-600 | Minimal | Acceptable |
| **K4 (100% IG MCC)** | Minor Filming (stable) | <200-300 | Minimal | Acceptable |
| **K5 (100% IG MCC blend + 0.5% EG Mg St)** | Minor Filming (stable) | 350-400 | Not evaluated | Not evaluated |
| **K6 (IG + EG MCC)** | Moderate Filming | <200 | Minimal | Not evaluated |

| | | | | |
|---|---|---|---|---|
| ^{a}Target Roller Compaction (RC) Force: 12 kN/cm (10-14 kN/cm); LEN formulation same as LEN single agent (SA) formulation. ^{b}Sticking/filming evaluation was performed on the individual BIC blend prior to compression. The sticking/filming evaluation was done with at least 1300 hits on Korsch XL-100; 1.5% EG Mg St only 500 hits on Korsch XL-100; 2:1 and 1:1 Mannitol:MCC only run on Compaction Simulator (CS). ^{c}Ejection force evaluation was performed on the final BIC/LEN tablet (i.e. after combining the BIC and LEN layers). ^{d} Delamination evaluation was performed on the final BIC/LEN tablet (i.e. after combining the BIC and LEN layers). The delamination was evaluated on hardness testing; run on CS mimicking XL-400, n=3 tablets/force. Minimal is up to 1/3 tablets/force across target RC range. ^{e}Blend properties evaluation was performed on the individual BIC blend prior to compression. The blend properties evaluation included compressibility profiles, tensile strength, flow, permeability. | | | | |

The results show that the use of 100% Intragranular MCC and 100% Intraganular MCC + 0.5% Extragranular Mg St provided BIC layers (K4 and K5) that were stable to filming, required low ejection forces and had acceptable blend properties. In contrast, the use of mannitol:MCC filler mixes (K1, K2, and K3) led to major sticking or filming in the tested formulations.

### BIC Non-Sink Dissolution (FaSSIF)

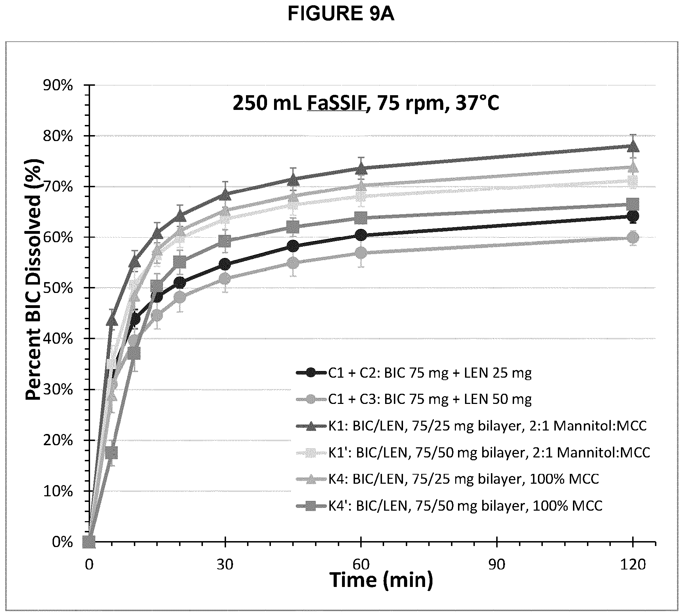

The dissolution of bictegravir from 75/25 mg BIC/LEN and 75/50 mg BIC/LEN bilayer tablets with different BIC layer formulations (K1/K1' and K4/K4') was determined as a function of time using the method of Example 5. The results were compared to the dissolution of bictegravir from the combination of comparative tablets of 75 mg BIC + 25 mg LEN (C1+C2) and 75mg BIC + 50 mg LEN (C1+C3). The results are shown in Figure 9A.

### LEN Non-Sink Dissolution (Cremophor)

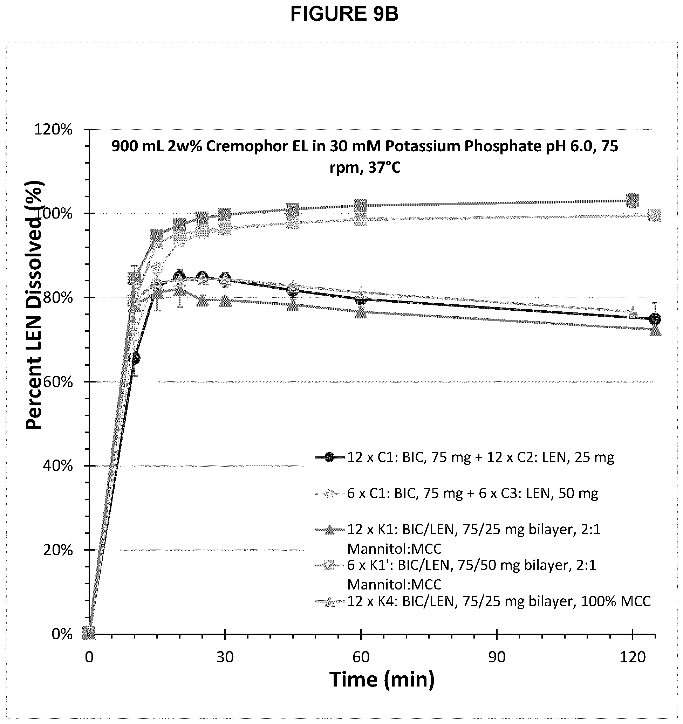

The dissolution of lenacapavir from 75/25 mg BIC/LEN and 75/50 mg BIC/LEN bilayer tablets with different BIC layer formulations (K1/K1' and K4/K4') was determined as a function of time using the method of Example 6. The results were compared to the dissolution of lenacapavir from the combination of comparative tablets of 75 mg BIC + 25 mg LEN (C1+C2) and 75mg BIC + 50 mg LEN (C1+C3). The results are shown in Figure 9B and show that the dissolution of lenacapavir from the 75/25 mg BIC/LEN bilayer tablets was equivalent to that of the comparative 75 mg BIC + 25 mg LEN coadministration, and the dissolution of lenacapavir from the 75/50 mg BIC/LEN bilayer tablets was equivalent to that of the comparative 75 mg BIC + 50 mg LEN coadministration.

### Pre-Clinical PK in Dogs

The PK properties of bictegravir and lenacapavir arising from 75/25 mg BIC/LEN and 75/50 mg BIC/LEN bilayer tablets with different BIC layer formulations (K1/K1' and K4/K4') was evaluated using the methods described in Example 7. The results are shown in the table below:

| **Bictegravir Pre-Clinical PK Parameters** | | | | |
|---|---|---|---|---|
| **PK parameter** | **K1 (75/25 mg BIC/LEN)** | **K1' (75/50 mg BIC/LEN)** | **K4 (75/25 mg BIC/LEN)** | **K4' (75/50 mg BIC/LEN)** |
| **AUCₗₐₛₜ (µM*hr)** | 179 ± 122 | 154 ± 58 | 172 ± 75 | 168 ± 27 |
| **AUC_{inf} (µM*hr)** | 179 ± 122 | 154 ± 58 | 172 ± 75 | 168 ± 27 |
| **Cₘₐₓ (µM)** | 29 ± 9 | 31 ± 10 | 28 ± 5 | 27 ± 5 |
| **Tₘₐₓ (hr)** | 1.5 ± 0.6 | 1.5 ± 0.6 | 1.7 ± 0.6 | 1.7 ± 0.5 |
| **T_{1/2} (hr)** | 14 ± 7 | 11 ± 10 | 26 ± 10 | 23 ± 5 |
| **F (%)** | 32 ± 20 | 27 ± 12 | 35 ± 18 | 33 ± 8 |

The PK properties of lenacapavir in dogs from the evaluated tablets are shown in the table below:

| **PK parameter** | **K1 (75/25 mg BIC/LEN)** | **K1' (75/50 mg BIC/LEN)** | **K4 (75/25 mg BIC/LEN)** | **K4' (75/50 mg BIC/LEN)** |
|---|---|---|---|---|
| **AUCₗₐₛₜ (µM*hr)** | 1.3 ± 0.7 | 1.7 ± 0.9 | 2.5 ± 1.8 | 2.7 ± 1.2 |
| **AUC_{inf} (µM*hr)** | 1.4 ± 0.7 | 1.7 ± 0.9 | 2.8 ± 2.3 | 2.9 ± 1.5 |
| **Cₘₐₓ (µM)** | 0.07 ± 0.04 | 0.09 ± 0.05 | 0.10 ± 0.06 | 0.12 ± 0.06 |
| **Tₘₐₓ (hr)** | 5 ± 2 | 4 ± 1 | 5 ± 1 | 4 ± 0 |
| **T_{1/2} (hr)** | 22 ± 9 | 22 ± 5 | 22 ± 4 | 29 ± 19 |
| **F (%)** | 3 ± 2 | 2 ± 1 | 7 ± 7 | 3 ± 1 |

### Example 11- Study to Compare Bictegravir/Lenacapavir Versus Current Therapy in People with HIV-1 Who Are Successfully Treated With a Complicated Regimen

This is a Phase 2/3 randomized, open-label, multicenter, active-controlled study to evaluate the safety and efficacy of bictegravir/lenacapavir versus stable baseline regimen in virologically suppressed people with HIV-1 on stable complex treatment regimens. The trial will include 5 treatment arms:
**Arm 1 (Experimental: Phase 2: Bictegravir (BIC) 75 mg + Lenacapavir (LEN) 25 mg):** Participants will switch from their stable baseline regimen (SBR) to a regimen of BIC 75 mg plus LEN 25 mg. Participants will receive a 2-day loading dose regimen of LEN 600 mg (on Days 1 and 2), in addition to the daily doses of BIC 75 mg plus LEN 25 mg starting on Day 1 up to the end of randomized treatment (ERT) visit, participants will be treated for at least 24 weeks during the Randomized Period.

Following Randomized Period, the participants will have an option to participate in an Extension Period to receive BIC/LEN fixed dose combination (FDC) at the selected dose.

**Arm 2 (Experimental: Phase 2: Bictegravir (BIC) 75 mg + Lenacapavir (LEN) 50 mg):** Participants will switch from their SBR to a regimen of BIC 75 mg plus LEN 50 mg. Participants will receive a 2-day loading dose regimen of LEN 600 mg (on Days 1 and 2), in addition to the daily doses of BIC 75 mg plus LEN 50 mg starting on Day 1 up to the ERT visit, participants will be treated for at least 24 weeks during the Randomized Period.

Following Randomized Period, the participants will have an option to participate in an Extension Period to receive BIC/LEN FDC at the selected dose.

**Arm 3 (Active Comparator: Phase 2: Stable Baseline Regimen (SBR)):** Participants will continue with their SBR per prescription for up to the ERT visit, participants will be treated for at least 24 weeks during the Randomized Period.

Following Randomized Period, the participants will have an option to participate in an Extension Period to receive BIC/LEN FDC at the selected dose.

SBR will include a combination of antiretroviral (ARV) regimen. ARV regimen may include the following, except for participants taking a single tablet regimen or taking a complete parenteral regimen (Cabenuva).

Nucleos(t)ide Reverse Transcriptase Inhibitors:
a. Abacavir
b. Emtricitabine
c. Lamivudine
d. Tenofovir alafenamide
e. Tenofovir disoproxil fumarate
f. Zidovudine

Non-Nucleosite Reverse Transcriptase Inhibitors:
a. Delavirdine
b. Efavirenz
c. Nevirapine
d. Rilpivirine
e. Doravirine

Integrase Inhibitors:
a. Bictegravir
b. Cabotegravir
c. Dolutegravir
d. Elvitegravir
e. Raltegravir

Protease Inhibitors:
a. Atazanavir
b. Darunavir
c. Fosamprenavir
d. Indinavir
e. Lopinavir
f. Nelfinavir
g. Saquinavir
h. Tipranavir

Chemokine Co-receptor 5 (CCR5) Antagonist:
a. Maraviroc

Fusion Inhibitors:
a. Enfuvirtide

gp 120 Attachment Inhibitor:
a. Fostemsavir

Anti-CD4 Monoclonal Antibodies:
a. Ibalizumab-uiyk

**Arm 4 (Experimental: Phase 3: BIC/LEN Fixed-dose Combination (FDC)):** Participants will switch to a regimen of BIC/LEN FDC (dose selection per Week 24 date from Phase 2). Participants will receive a 2-day loading dose regimen of LEN 600 mg (on Days 1 and 2), in addition to the daily doses of BIC/LEN FDC starting on Day 1 up to the ERT visit, participants will be treated for at least 48 weeks during the Randomized Period.

Following Randomized Period, the participants will have an option to participate in an Extension Period to receive BIC/LEN FDC at the selected dose.

**Arm 5 (Active Comparator: Phase 3: Stable Baseline Regimen (SBR)):** Participants will continue with their SBR per prescription for up to the ERT visit, participants will be treated for at least 48 weeks during the Randomized Period.

Following Randomized Period, the participants will have an option to participate in an Extension Period to receive BIC/LEN FDC at the selected dose.

SBR will include a combination of antiretroviral (ARV) regimen. ARV regimen may include the following, except for participants taking a single tablet regimen or taking a complete parenteral regimen (Cabenuva).

Nucleos(t)ide Reverse Transcriptase Inhibitors:
a. Abacavir
b. Emtricitabine
c. Lamivudine
d. Tenofovir alafenamide
e. Tenofovir disoproxil fumarate
f. Zidovudine

Non-Nucleosite Reverse Transcriptase Inhibitors:
a. Delavirdine
b. Efavirenz
c. Nevirapine
d. Rilpivirine
e. Doravirine

Integrase Inhibitors:
a. Bictegravir
b. Cabotegravir
c. Dolutegravir
d. Elvitegravir
e. Raltegravir

Protease Inhibitors:
a. Atazanavir
b. Darunavir
c. Fosamprenavir
d. Indinavir
e. Lopinavir
f. Nelfinavir
g. Saquinavir
h. Tipranavir

Chemokine Co-receptor 5 (CCR5) Antagonist:
a. Maraviroc

Fusion Inhibitors:
a. Enfuvirtide

gp 120 Attachment Inhibitor:
a. Fostemsavir

Anti-CD4 Monoclonal Antibodies:
a. Ibalizumab-uiyk

### Endpoints

Primary Outcome Measures will be:
Phase 2: Proportion of participants with HIV-1 RNA ≥ 50 copies/mL at week 24 as determined by the US FDA-defined snapshot algorithm [Time Frame: Week 24]
Phase 3: Proportion of participants with HIV-1 RNA ≥ 50 copies/mL at week 48 as determined by the US FDA-defined snapshot algorithm [Time Frame: Week 48]

Secondary Outcome Measures will be:
Phase 2: Proportion of participants with HIV-1 RNA < 50 copies/mL at week 24 as determined by the US FDA-defined snapshot algorithm [Time Frame: Week 24]
Phase 2: Change from baseline in CD4 cell count at week 24 [Time Frame: Baseline, Week 24]
Phase 2: Percentage of participants experiencing treatment-emergent adverse events (AEs) through week 24 [Time Frame: First dose date up to week 24]
Phase 2: Pharmacokinetic (PK) parameter: Cₘₐₓ of bictegravir (BIC) and lenacapavir (LEN) at steady state [Time Frame: Day 1 up to Week 24] (Cₘₐₓ is defined as the maximum observed concentration of drug).
Phase 2: PK parameter: AUCₜₐᵤ of BIC and LEN at steady state [Time Frame: Day 1 up to week 24] (AUCₜₐᵤ is defined as the area under the concentration versus time curve over the dosing interval)
Phase 2: PK parameter: Cₜₐᵤ of BIC and LEN at steady state [Time Frame: Day 1 up to week 24] (Cₜₐᵤ is defined as the observed drug concentration at the end of the dosing interval)
Phase 3: Proportion of participants with HIV-1 RNA < 50 copies/mL at week 48 as determined by the US FDA-defined snapshot algorithm [Time Frame: Week 48]
Phase 3: Change from baseline in CD4 cell count at week 48 [Time Frame: Baseline, Week 48]
Phase 3: Percentage of participants experiencing treatment-emergent adverse events (AEs) through week 48 [Time Frame: First dose date up to week 48]

### Eligibility Criteria

### Key inclusion criteria:

Documented plasma human immunodeficiency virus type-1 (HIV-1) ribonucleic acid (RNA) levels < 50 copies/mL during treatment with the baseline regimen for a minimum period of 6 months prior to the screening visit.

### Plasma HIV-1 RNA levels < 50 copies/mL at screening.

Currently receiving a complex antiretroviral (ARV) regimen due to previous viral resistance, or intolerance, or contraindication to existing single-tablet regimens (STR). The criteria to define a complex regimen in this study are as follows:
a. A regimen containing a boosted protease inhibitor or a nonnucleos(t)ide reverse transcriptase inhibitor (NRTI) plus at least 1 other third agent (ie, an agent from a class other than NRTIs) (eg, bictegravir/emtricitabine/tenofovir alafenamide (coformulated; Biktarvy^{®})(BVY) + darunavir/cobicistat, BVY + etravirine), or
b. A regimen of ≥ 2 pills/day, or a regimen requiring dosing more than once daily, or
c. A regimen containing parenteral agent(s) (excluding a complete long-acting injectable regimen, such as intramuscular cabotegravir plus rilpivirine) as well as oral agents.

No documented or suspected resistance to bictegravir (BIC).

Estimated glomerular filtration rate ≥ 15 mL/min according to the Cockcroft-Gault formula for creatinine clearance (CLcr) who are not on renal replacement therapy.

### Key exclusion criteria:

Prior use of, or exposure to, lenacapavir (LEN)
Active tuberculosis infection
Chronic hepatitis B virus (HBV) infection

As those skilled in the art will appreciate, numerous modifications and variations of the present disclosure are possible in light of these teachings, and all such are contemplated hereby.

For example, in addition to the embodiments described herein, the present disclosure contemplates and claims those disclosures resulting from the combination of features of the disclosure cited herein and those of the cited prior art references which complement the features of the present disclosure. Similarly, it will be appreciated that any described material, feature, or article may be used in combination with any other material, feature, or article, and such combinations are considered within the scope of this disclosure.

All publications, patents and patent applications are incorporated by reference in their entirety, as though individually incorporated by reference. The disclosure has been described with reference to various specific and embodiments and techniques. However, it should be understood that many variations and modifications may be made while remaining within the spirit and scope of the disclosure.

Further embodiments of the invention are provided below.
1. A tablet comprising bictegravir or a pharmaceutically acceptable salt thereof and lenacapavir or a pharmaceutically acceptable salt thereof, wherein the amount of bictegravir or pharmaceutically acceptable salt thereof corresponds to about 50-100 mg bictegravir free acid, and the amount of lenacapavir or pharmaceutically acceptable salt thereof corresponds to about 25-100 mg lenacapavir free acid.
2. The tablet according to embodiment 1, wherein the amount of bictegravir or pharmaceutically acceptable salt thereof corresponds to about 50-75 mg bictegravir free acid.
3. The tablet according to embodiment 1 or 2, wherein the amount of bictegravir or pharmaceutically acceptable salt thereof corresponds to about 75 mg bictegravir free acid.
4. The tablet according to any preceding embodiment, wherein the bictegravir or pharmaceutically acceptable salt thereof is bictegravir sodium.
5. The tablet according to any preceding embodiment, wherein the amount of lenacapavir or pharmaceutically acceptable salt thereof corresponds to about 25-75 mg lenacapavir free acid.
6. The tablet according to embodiment 5, wherein the amount of lenacapavir or pharmaceutically acceptable salt thereof corresponds to about 25-50 mg lenacapavir free acid.
7. The tablet according to any one of embodiment s 1-4, wherein the amount of lenacapavir or pharmaceutically acceptable salt thereof corresponds to about 100 mg lenacapavir free acid.
8. The tablet according to any one of embodiments 1-6, wherein the amount of lenacapavir or pharmaceutically acceptable salt thereof corresponds to about 50 mg lenacapavir free acid.
9. The tablet according to any one of embodiments 1-6, wherein the amount of lenacapavir or pharmaceutically acceptable salt thereof corresponds to about 25 mg lenacapavir free acid.
10. The tablet according to any preceding embodiment, wherein the lenacapavir or pharmaceutically acceptable salt thereof is lenacapavir sodium.
11. The tablet according to any preceding embodiment, wherein the bictegravir or pharmaceutically acceptable salt thereof is bictegravir sodium and the lenacapavir or pharmaceutically acceptable salt thereof is lenacapavir sodium.
12. The tablet according to any preceding embodiment, wherein the tablet is free from active pharmaceutical ingredients other than (a) bictegravir or a pharmaceutically acceptable salt thereof and (b) lenacapavir or a pharmaceutically acceptable salt thereof.
13. The tablet according to any preceding embodiment, wherein the tablet is a monolayer tablet.
14. The tablet according to any one of embodiments 1-12, wherein the tablet is a multilayer tablet.
15. The tablet according to any one of embodiments 1-12 and embodiment 14, wherein the tablet is a bilayer tablet.
16. The tablet according to embodiment 14 or 15, wherein the tablet comprises (a) a first layer comprising bictegravir or a pharmaceutically acceptable salt thereof, and (b) a second layer comprising lenacapavir or a pharmaceutically acceptable salt thereof.
17. The tablet according to embodiment 16, wherein (a) the first layer is substantially free of lenacapavir or a pharmaceutically acceptable salt thereof, or (b) the second layer is substantially free of bictegravir or a pharmaceutically acceptable salt thereof.
18. The tablet according to any one of embodiments 1-6 and 9-17, wherein the amount of bictegravir or pharmaceutically acceptable salt thereof corresponds to about 75 mg bictegravir free acid, and wherein the amount of lenacapavir or pharmaceutically acceptable salt thereof corresponds to about 25 mg lenacapavir free acid.
19. The tablet according to any one of embodiments 1-6, 8, and 10-17, wherein the amount of bictegravir or pharmaceutically acceptable salt thereof corresponds to about 75 mg bictegravir free acid, and wherein the amount of lenacapavir or pharmaceutically acceptable salt thereof corresponds to about 50 mg lenacapavir free acid.
20. The tablet according to any one of embodiments 1-7 and 10-17, wherein the amount of bictegravir or pharmaceutically acceptable salt thereof corresponds to about 75 mg bictegravir free acid, and wherein the amount of lenacapavir or pharmaceutically acceptable salt thereof corresponds to about 100 mg lenacapavir free acid.
21. The tablet according to any one of embodiments 1-6, 9-13, and 18, wherein the tablet is a monolayer tablet, wherein the amount of bictegravir or pharmaceutically acceptable salt thereof corresponds to about 75 mg bictegravir free acid, and wherein the amount of lenacapavir or pharmaceutically acceptable salt thereof corresponds to about 25 mg lenacapavir free acid.
22. The tablet according to any one of embodiments 1-6, 8, 10-13, and 19, wherein the tablet is a monolayer tablet, wherein the amount of bictegravir or pharmaceutically acceptable salt thereof corresponds to about 75 mg bictegravir free acid, and wherein the amount of lenacapavir or pharmaceutically acceptable salt thereof corresponds to about 50 mg lenacapavir free acid.
23. The tablet according to any one of embodiments 1-7, 10-13, and 20, wherein the tablet is a monolayer tablet, wherein the amount of bictegravir or pharmaceutically acceptable salt thereof corresponds to about 75 mg bictegravir free acid, and wherein the amount of lenacapavir or pharmaceutically acceptable salt thereof corresponds to about 100 mg lenacapavir free acid.
24. The tablet according to any one of embodiments 1-6, 9-12, and 14-18, wherein the tablet is a bilayer tablet comprising (a) a first layer comprising bictegravir or a pharmaceutically acceptable salt thereof, wherein the amount of bictegravir or pharmaceutically acceptable salt thereof corresponds to about 75 mg bictegravir free acid, and (b) a second layer comprising lenacapavir or a pharmaceutically acceptable salt thereof, wherein the amount of lenacapavir or pharmaceutically acceptable salt thereof corresponds to about 50 mg lenacapavir free acid.
25. The tablet according to any one of embodiments 1-6, 8, 10-12, 14-17, and 19, wherein the tablet is a bilayer tablet comprising (a) a first layer comprising bictegravir or a pharmaceutically acceptable salt thereof, wherein the amount of bictegravir or pharmaceutically acceptable salt thereof corresponds to about 75 mg bictegravir free acid, and (b) a second layer comprising lenacapavir or a pharmaceutically acceptable salt thereof, wherein the amount of lenacapavir or pharmaceutically acceptable salt thereof corresponds to about 25 mg lenacapavir free acid.
26. The tablet according to any one of embodiments 18-25, wherein the bictegravir or pharmaceutically acceptable salt thereof is bictegravir sodium and the lenacapavir or pharmaceutically acceptable salt thereof is lenacapavir sodium.
27. The tablet according to any preceding embodiment, wherein the tablet further comprises at least one excipient selected from: copovidone, poloxamer, mannitol, microcrystalline cellulose, croscarmellose sodium and magnesium stearate.
28. The tablet according to embodiment 27, wherein the tablet comprises copovidone, poloxamer, mannitol, microcrystalline cellulose, croscarmellose sodium and magnesium stearate.
29. The tablet according to embodiment 28, wherein the tablet comprises about 5-50 mg copovidone, about 0.5-10 mg poloxamer, about 50-200 mg mannitol, about 50-250 mg microcrystalline cellulose, about 25-50 mg croscarmellose sodium and about 1-10 mg magnesium stearate.
30. The tablet according to embodiment 29, wherein the tablet is a monolayer tablet, and wherein the tablet comprises about 5-30 mg copovidone, about 100-200 mg mannitol, about 50-100 mg microcrystalline cellulose, and about 5-10 mg magnesium stearate.
31. The tablet according to embodiment 29, wherein the tablet is a bilayer tablet, and wherein the tablet comprises about 5-15 mg copovidone, about 0.5-5 mg poloxamer, about 50-125 mg mannitol, about 200-250 mg microcrystalline cellulose, and about 5-10 mg magnesium stearate.
32. The tablet according to any one of embodiments 16, 17, 24, and 25, wherein the first layer comprises a filler selected from mannitol, microcrystalline cellulose, and a combination thereof.
33. The tablet according to embodiment 32, wherein the weight ratio of mannitol to microcrystalline cellulose in the first layer is about 2:1 to about1:2.
34. The tablet according to embodiment 32, wherein the weight ratio of mannitol to microcrystalline cellulose in the first layer is about 2:1.
35. The tablet according to embodiment 32, wherein the weight ratio of mannitol to microcrystalline cellulose in the first layer is about 1:1.
36. The tablet according to embodiment 32, wherein the weight ratio of mannitol to microcrystalline cellulose in the first layer is about 1:2.
37. The tablet according to embodiment 32, wherein the filler is microcrystalline cellulose and wherein the first layer is substantially free from mannitol.
38. The tablet according to embodiment 37, wherein the first layer comprises about 30-75% w/w microcrystalline cellulose.
39. The tablet according to embodiment 38, wherein the first layer comprises about 50-75% w/w microcrystalline cellulose.
40. The tablet according to embodiment 39, wherein the first layer comprises about 60-70% w/w microcrystalline cellulose.
41. The tablet according to any preceding embodiment, wherein the tablet is coated with a film coating.
42. The tablet according to embodiment 41, wherein the film coating comprises Opadry II.
43. The tablet according to any preceding embodiment, wherein the tablet comprises about 10-25 %w/w bictegravir or a pharmaceutically acceptable salt thereof and about 5-25 %w/w lenacapavir or a pharmaceutically acceptable salt thereof.
44. The tablet according to any to any one of embodiments 1-6, 8, 10-13, 18, 21, 25-30, and 41-43, wherein the tablet is a monolayer tablet, wherein the amount of bictegravir or pharmaceutically acceptable salt thereof corresponds to about 75 mg bictegravir free acid, and the amount of lenacapavir or pharmaceutically acceptable salt thereof corresponds to about 25 mg lenacapavir free acid, and wherein the tablet further comprises copovidone, poloxamer, mannitol, microcrystalline cellulose, croscarmellose sodium and magnesium stearate.
45. The tablet according to any one of embodiments 1-6, 9-13, 18, 21, 25-30, and 41-44, wherein the tablet is a monolayer tablet, wherein the amount of bictegravir or pharmaceutically acceptable salt thereof corresponds to about 75 mg bictegravir free acid, and the amount of lenacapavir or pharmaceutically acceptable salt thereof corresponds to about 25 mg lenacapavir free acid, and wherein the tablet comprises about 5-8 mg copovidone, about 1-3 mg poloxamer, about 175-200 mg mannitol, about 90-100 mg microcrystalline cellulose, about 30-40 mg croscarmellose sodium, and about 5-8 mg magnesium stearate.
46. The tablet according to embodiment 45, wherein the tablet weighs about 430-440 mg excluding the film coating.
47. The tablet according to any to any one of embodiments 1-6, 8, 10-13, 19, 22, 26-30, and 41-43, wherein the tablet is a monolayer tablet, wherein the amount of bictegravir or pharmaceutically acceptable salt thereof corresponds to about 75 mg bictegravir free acid, and the amount of lenacapavir or pharmaceutically acceptable salt thereof corresponds to about 50 mg lenacapavir free acid, and wherein the tablet further comprises copovidone, poloxamer, mannitol, microcrystalline cellulose, croscarmellose sodium and magnesium stearate.
48. The tablet according to any one of embodiments 1-6, 8, 10-13, 19, 22, 26-30, 41-43, and 47, wherein the tablet is a monolayer tablet, wherein the amount of bictegravir or pharmaceutically acceptable salt thereof corresponds to about 75 mg bictegravir free acid, and the amount of lenacapavir or pharmaceutically acceptable salt thereof corresponds to about 50 mg lenacapavir free acid, and wherein the tablet comprises about 10-15 mg copovidone, about 2-5 mg poloxamer, about 150-175 mg mannitol, about 75-90 mg microcrystalline cellulose, about 30-40 mg croscarmellose sodium, and about 5-8 mg magnesium stearate.
49. The tablet according to embodiment 48, wherein the tablet weighs about 430-440 mg.
50. The tablet according to any to any one of embodiments 1-6, 9-12, 14-18, 24, 26-29, 31, 32, 37, 38, and 41-43, wherein the tablet is a bilayer tablet comprising (a) a first layer comprising bictegravir or a pharmaceutically acceptable salt thereof, and (b) a second layer comprising lenacapavir or a pharmaceutically acceptable salt thereof,
   wherein the amount of bictegravir or pharmaceutically acceptable salt thereof corresponds to about 75 mg bictegravir free acid, and wherein the first layer further comprises microcrystalline cellulose, croscarmellose sodium and magnesium stearate, and
   wherein the amount of lenacapavir or pharmaceutically acceptable salt thereof corresponds to about 25 mg lenacapavir free acid, and wherein the second layer further comprises copovidone, poloxamer, mannitol, microcrystalline cellulose, croscarmellose sodium and magnesium stearate.
51. The tablet according to any one of embodiments 1-6, 9-12, 14-18, 24, 26-29, 31, 32, 37, 38, 41-43, and 50, wherein the tablet is a bilayer tablet comprising (a) a first layer comprising bictegravir or a pharmaceutically acceptable salt thereof, and (b) a second layer comprising lenacapavir or a pharmaceutically acceptable salt thereof,
   wherein the amount of bictegravir or pharmaceutically acceptable salt thereof corresponds to about 75 mg bictegravir free acid, and wherein the first layer further comprises about 185-200 mg microcrystalline cellulose, about 20-30 mg croscarmellose sodium, and about 3-5 mg magnesium stearate, and
   wherein the amount of lenacapavir or pharmaceutically acceptable salt thereof corresponds to about 25 mg lenacapavir free acid, and wherein the second layer further comprises about 5-8 mg copovidone, about 1-3 mg poloxamer, about 45-60 mg mannitol, about 20-30 mg microcrystalline cellulose, about 5-15 mg croscarmellose sodium, and about 1-3 mg magnesium stearate.
52. The tablet according to any to any one of embodiments 1-6, 9-12, 14-18, 25-29, 31, 32, 37, 38, and 41-43, wherein the tablet is a bilayer tablet comprising (a) a first layer comprising bictegravir or a pharmaceutically acceptable salt thereof, and (b) a second layer comprising lenacapavir or a pharmaceutically acceptable salt thereof,
   wherein the amount of bictegravir or pharmaceutically acceptable salt thereof corresponds to about 75 mg bictegravir free acid, and wherein the first layer further comprises microcrystalline cellulose, croscarmellose sodium and magnesium stearate, and
   wherein the amount of lenacapavir or pharmaceutically acceptable salt thereof corresponds to about 50 mg lenacapavir free acid, and wherein the second layer further comprises copovidone, poloxamer, mannitol, microcrystalline cellulose, croscarmellose sodium and magnesium stearate.
53. The tablet according to any one of embodiments 1-6, 8, 10-12, 14-17, 19, 25-29, 31, 32, 37, 38, 41-43, and 52, wherein the tablet is a bilayer tablet comprising (a) a first layer comprising bictegravir or a pharmaceutically acceptable salt thereof, and (b) a second layer comprising lenacapavir or a pharmaceutically acceptable salt thereof,
   wherein the amount of bictegravir or pharmaceutically acceptable salt thereof corresponds to about 75 mg bictegravir free acid, and wherein the first layer further comprises about 185-200 mg microcrystalline cellulose, about 20-30 mg croscarmellose sodium and about 3-5 mg magnesium stearate, and
   wherein the amount of lenacapavir or pharmaceutically acceptable salt thereof corresponds to about 50 mg lenacapavir free acid, and wherein the second layer further comprises about 10-15 mg copovidone, about 2-5 mg poloxamer, about 100-110 mg mannitol, about 45-60 mg microcrystalline cellulose, about 15-25 mg croscarmellose sodium and about 2-5 mg magnesium stearate.
54. The tablet according to embodiment 52 or 53, wherein the tablet weighs about 545-555 mg.
55. The tablet according to any one of embodiments 44-54, wherein the bictegravir or pharmaceutically acceptable salt thereof is bictegravir sodium.
56. The tablet according to any one of embodiments 44-55, wherein the lenacapavir or pharmaceutically acceptable salt thereof is lenacapavir sodium.
57. The tablet according to any preceding embodiment, wherein the lenacapavir or pharmaceutically acceptable salt thereof is present in a spray dried dispersion.
58. The tablet according to any preceding embodiment, wherein the tablet releases at least about 50% of the bictegravir or pharmaceutically acceptable salt thereof in about 30 minutes, measured using USP apparatus II, in 250 mL of fasted simulated intestinal fluid, pH 6.5, at 37 °C and paddle speed of 75 rpm.
59. The tablet according to embodiment 58, wherein the tablet releases at least about 60% of the bictegravir or pharmaceutically acceptable salt thereof in about 30 minutes, measured using USP apparatus II, in 250 mL of fasted simulated intestinal fluid, pH 6.5, at 37 °C and paddle speed of 75 rpm.
60. The tablet according to embodiment 59, wherein the tablet releases at least about 70% of the bictegravir or pharmaceutically acceptable salt thereof in about 30 minutes, measured using USP apparatus II, in 250 mL of fasted simulated intestinal fluid, pH 6.5, at 37 °C and paddle speed of 75 rpm.
61. The tablet according to any preceding embodiment, wherein the tablet releases at least about 50% of the lenacapavir or pharmaceutically acceptable salt thereof in about 15 minutes, measured using USP apparatus II, in 900 mL of 2% w/w Cremophor EL in 30 mM potassium phosphate, pH 6.0, at 37 °C and paddle speed of 75 rpm.
62. The tablet according to embodiment 60, wherein the tablet releases at least about 70% of the lenacapavir or pharmaceutically acceptable salt thereof in about 15 minutes, measured using USP apparatus II, in 900 mL of 2% w/w Cremophor EL in 30 mM potassium phosphate, pH 6.0, at 37 °C and paddle speed of 75 rpm.
63. The tablet according to embodiment 61, wherein the tablet releases at least about 80% of the lenacapavir or pharmaceutically acceptable salt thereof in about 15 minutes, measured using USP apparatus II, in 900 mL of 2% w/w Cremophor EL in 30 mM potassium phosphate, pH 6.0, at 37 °C and paddle speed of 75 rpm.
64. A method of producing the tablet according to any one of the preceding embodiments.
65. A tablet according to any one of embodiments 1-63 for use in the therapeutic or prophylactic treatment of an HIV infection in a subject.
66. A method of therapeutic treatment of an HIV infection comprising administering to a subject in need thereof a tablet according to any one of embodiments 1-63.
67. A method of preventing HIV infection comprising administering to a subject in need thereof a tablet according to any one of embodiments 1-63.
68. A tablet for use according to embodiment 65, or the method according to embodiment 66 or 67, wherein the prophylactic treatment is pre-exposure prophylaxis (PrEP) to reduce the risk of sexually acquired HIV in a subject.
69. A tablet for use according to embodiment 65, or the method according to embodiment 66 or 67, wherein the prophylactic treatment is post-exposure prophylaxis (PEP) to reduce the risk of sexually acquired HIV in a subject.
70. The tablet for use according to any one of embodiment 65, 68, or 69, or the method according to any one of embodiment 65-68, wherein the subject is virologically suppressed.
71. The method or tablet for use according to embodiment 70, wherein the subject is treatment experienced.
72. The method according to any one of embodiments 66-67, or the tablet for use according to any one of embodiments 65, or 68-71, wherein the amount of bictegravir or pharmaceutically acceptable salt thereof corresponds to about 75 mg bictegravir free acid, and wherein the amount of lenacapavir or pharmaceutically acceptable salt thereof corresponds to about 25 mg lenacapavir free acid.
73. The method according to any one of embodiments 66-67, or the tablet for use according to any one of embodiments 65, or 68-71, wherein the amount of bictegravir or pharmaceutically acceptable salt thereof corresponds to about 75 mg bictegravir free acid, and wherein the amount of lenacapavir or pharmaceutically acceptable salt thereof corresponds to about 50 mg lenacapavir free acid.
74. The method according to any one of embodiments 66-67, or the tablet for use according to any one of embodiments 65, or 68-71, wherein the amount of bictegravir or pharmaceutically acceptable salt thereof corresponds to about 75 mg bictegravir free acid, and wherein the amount of lenacapavir or pharmaceutically acceptable salt thereof corresponds to about 100 mg lenacapavir free acid.
75. The method according to any one of embodiments 66-67, or the tablet for use according to any one of embodiments 65, or 68-71, wherein the tablet is a monolayer tablet, wherein the amount of bictegravir or pharmaceutically acceptable salt thereof corresponds to about 75 mg bictegravir free acid, and wherein the amount of lenacapavir or pharmaceutically acceptable salt thereof corresponds to about 25 mg lenacapavir free acid.
76. The method according to any one of embodiments 66-67, or the tablet for use according to any one of embodiments 65, or 68-71, wherein the tablet is a monolayer tablet, wherein the amount of bictegravir or pharmaceutically acceptable salt thereof corresponds to about 75 mg bictegravir free acid, and wherein the amount of lenacapavir or pharmaceutically acceptable salt thereof corresponds to about 50 mg lenacapavir free acid.
77. The method according to any one of embodiments 66-67, or the tablet for use according to any one of embodiments 65, or 68-71, wherein the tablet is a monolayer tablet, wherein the amount of bictegravir or pharmaceutically acceptable salt thereof corresponds to about 75 mg bictegravir free acid, and wherein the amount of lenacapavir or pharmaceutically acceptable salt thereof corresponds to about 100 mg lenacapavir free acid.
78. The method according to any one of embodiments 66-67, or the tablet for use according to any one of embodiments 65, or 68-71, wherein the tablet is a bilayer tablet comprising (a) a first layer comprising bictegravir or a pharmaceutically acceptable salt thereof, wherein the amount of bictegravir or pharmaceutically acceptable salt thereof corresponds to about 75 mg bictegravir free acid, and (b) a second layer comprising lenacapavir or a pharmaceutically acceptable salt thereof, wherein the amount of lenacapavir or pharmaceutically acceptable salt thereof corresponds to about 25 mg lenacapavir free acid.
79. The method according to any one of embodiments 66-67, or the tablet for use according to any one of embodiments 65, or 68-71, wherein the tablet is a bilayer tablet comprising (a) a first layer comprising bictegravir or a pharmaceutically acceptable salt thereof, wherein the amount of bictegravir or pharmaceutically acceptable salt thereof corresponds to about 75 mg bictegravir free acid, and (b) a second layer comprising lenacapavir or a pharmaceutically acceptable salt thereof, wherein the amount of lenacapavir or pharmaceutically acceptable salt thereof corresponds to about 50 mg lenacapavir free acid.
80. The tablet for use according to embodiments 64, or 67-78, or the method according to any one of embodiments 66-67, or 65, 68, 71-79, wherein the tablet is administered once daily.
81. The method or tablet for use according to embodiment 80, wherein the tablet is administered once daily following a loading dose of lenacapavir or pharmaceutically acceptable salt thereof corresponding to about 1200 mg lenacapavir free acid.
82. The method or tablet for use according to embodiment 81, wherein the loading dose is administered over two days.
83. The method or tablet for use according to embodiment 82, wherein an amount of lenacapavir or pharmaceutically acceptable salt thereof corresponding to about 600 mg lenacapavir free acid is administered on the first day, and an amount of lenacapavir or pharmaceutically acceptable salt thereof corresponding to about 600 mg lenacapavir free acid is administered on the second day.

## Claims

1. A tablet comprising bictegravir or a pharmaceutically acceptable salt thereof and lenacapavir or a pharmaceutically acceptable salt thereof, wherein the tablet is a monolayer tablet, wherein the amount of bictegravir or pharmaceutically acceptable salt thereof corresponds to about 75 mg bictegravir free acid, and wherein the amount of lenacapavir or pharmaceutically acceptable salt thereof corresponds to about 50 mg lenacapavir free acid.

2. The tablet according to claim 1, wherein the tablet comprises about 10-25 %w/w bictegravir or a pharmaceutically acceptable salt thereof and about 5-25 %w/w lenacapavir or a pharmaceutically acceptable salt thereof.

3. The tablet according to claim 1 or claim 2, wherein the bictegravir or pharmaceutically acceptable salt thereof is bictegravir sodium.

4. The tablet according to any preceding claim, wherein the tablet comprises about 79 mg bictegravir sodium.

5. The tablet according to any preceding claim, wherein the lenacapavir or pharmaceutically acceptable salt thereof is lenacapavir sodium.

6. The tablet according to any preceding claim, wherein the tablet comprises about 51 mg lenacapavir sodium.

7. The tablet according to any preceding claim, wherein the tablet comprises copovidone, poloxamer, mannitol, microcrystalline cellulose, croscarmellose sodium and magnesium stearate.

8. The tablet according to claim 7, wherein the tablet comprises about 5-15 mg copovidone, about 0.5-10 mg poloxamer, about 50-200 mg mannitol, about 50-250 mg microcrystalline cellulose, about 25-50 mg croscarmellose sodium and about 1-10 mg magnesium stearate, optionally wherein the tablet comprises about 10-15 mg copovidone, about 2-5 mg poloxamer, about 150-175 mg mannitol, about 75-90 mg microcrystalline cellulose, about 30-40 mg croscarmellose sodium and about 5-8 mg magnesium stearate.

9. The tablet according to any one of claims 1-8, wherein the tablet is coated with a film coating, preferably wherein the film coating is a non-functional film coating.

10. The tablet according to claim 9, wherein the tablet is coated with a titanium dioxide free film coating.

11. The tablet according to claim 9 or claim 10, wherein the tablet weighs about 430-440 mg excluding the film coating.

12. A tablet according to any one of claims 1-11 for use in the therapeutic or prophylactic treatment of an HIV infection in a subject.

13. The tablet for use according to claim 12, wherein the subject is virologically suppressed.

14. The tablet for use according to claim 13, wherein the subject is treatment experienced.

15. The tablet for use according to any one of claims 12-14, wherein the tablet is administered once daily following a loading dose of lenacapavir or pharmaceutically acceptable salt thereof corresponding to about 1200 mg lenacapavir free acid.
